(19) Europäisches Patentamt / European Patent Office / Office européen des brevets

(11) **EP 3 313 441 B1**

(12) **EUROPEAN PATENT SPECIFICATION**

(45) Date of publication and mention of the grant of the patent:
**21.02.2024 Bulletin 2024/08**

(21) Application number: **16815351.8**

(22) Date of filing: **24.06.2016**

(51) International Patent Classification (IPC):
*C07K 16/28* (2006.01)     *A61K 39/39* (2006.01)
*A61K 39/395* (2006.01)     *C07K 16/30* (2006.01)
*A61P 37/04* (2006.01)     *A61P 35/00* (2006.01)

(52) Cooperative Patent Classification (CPC):
**C07K 16/2896; A61K 45/06; A61P 35/00;**
**A61P 37/04; C07K 16/2803; C07K 16/2827;**
A61K 2039/505; A61K 2039/507; A61K 2039/545;
C07K 2317/76

(86) International application number:
**PCT/US2016/039165**

(87) International publication number:
**WO 2016/210223 (29.12.2016 Gazette 2016/52)**

(54) **IMMUNE MODULATION AND TREATMENT OF SOLID TUMORS WITH ANTIBODIES THAT SPECIFICALLY BIND CD38**

IMMUNMODULATION UND BEHANDLUNG VON SOLIDEN TUMOREN MIT SPEZIFISCH AN CD38 BINDENDEN ANTIKÖRPERN

MODULATION IMMUNITAIRE ET TRAITEMENT DE TUMEURS SOLIDES AVEC DES ANTICORPS SE LIANT SPÉCIFIQUEMENT À CD38

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.06.2015  US 201562184018 P**
              **02.11.2015  US 201562249546 P**
              **04.11.2015  US 201562250566 P**
              **04.12.2015  US 201562263307 P**
              **04.05.2016  US 201662331489 P**

(43) Date of publication of application:
**02.05.2018  Bulletin 2018/18**

(73) Proprietor: **Janssen Biotech, Inc.**
**Horsham, PA 19044 (US)**

(72) Inventors:
• **AHMADI, Tahamtan**
  **Spring House, Pennsylvania 19477 (US)**

• **CASNEUF, Tineke**
  **1081 HV Amsterdam (NL)**
• **LOKHORST, Henk M.**
  **1081 HV Amsterdam (NL)**
• **MUTIS, Tuna**
  **1081 HV Amsterdam (NL)**
• **SASSER, Amy**
  **Doylestown, Pennsylvania 18901 (US)**

(74) Representative: **Carpmaels & Ransford LLP**
**One Southampton Row**
**London WC1B 5HA (GB)**

(56) References cited:
**WO-A1-2012/092612      WO-A1-2014/068114**
**WO-A2-2008/073160      WO-A2-2015/009726**
**US-A1- 2009 148 449      US-A1- 2014 099 254**
**US-A1- 2014 356 318      US-A1- 2015 125 447**
**US-A1- 2015 246 123**

- M. DE WEERS ET AL: "Daratumumab, a Novel Therapeutic Human CD38 Monoclonal Antibody, Induces Killing of Multiple Myeloma and Other Hematological Tumors", THE JOURNAL OF IMMUNOLOGY, vol. 186, no. 3, 27 December 2010 (2010-12-27), pages 1840-1848, XP055210884, ISSN: 0022-1767, DOI: 10.4049/jimmunol.1003032
- CHILLEMI A ET AL: "Anti-CD38 antibody therapy: windows of opportunity yielded by the functional characteristics of the target molecule", MOLECULAR MEDICINE, FEINSTEIN INSTITUTE FOR MEDICAL RESEARCH, WASHINGTON, DC; US, vol. 19, no. 1, 1 May 2013 (2013-05-01), pages 99-108, XP002735274, ISSN: 1076-1551, DOI: 10.2119/MOLMED.2013.00009 [retrieved on 2013-04-17]
- SUZANNE L. TOPALIAN ET AL: "Immune Checkpoint Blockade: A Common Denominator Approach to Cancer Therapy", CANCER CELL, vol. 27, no. 4, 1 April 2015 (2015-04-01), pages 450-461, XP055372181, US ISSN: 1535-6108, DOI: 10.1016/j.ccell.2015.03.001
- DANIEL T. PATTON ET AL: "The PI3K p110[delta] Regulates Expression of CD38 on Regulatory T Cells", PLOS ONE, vol. 6, no. 3, 1 March 2011 (2011-03-01), page e17359, XP055568720, DOI: 10.1371/journal.pone.0017359
- CLAUDIA MAURI ET AL: "The expanding family of regulatory B cells", INTERNATIONAL IMMUNOLOGY, vol. 27, no. 10, 12 June 2015 (2015-06-12) , pages 479-486, XP055568703, ISSN: 0953-8178, DOI: 10.1093/intimm/dxv038
- JAMES E TALMADGE ET AL: "History of myeloid-derived suppressor cells", NATURE REVIEWS CANCER, vol. 13, no. 10, 24 September 2013 (2013-09-24), pages 739-752, XP055568927, DOI: doi: 10.1038/nrc3581
- JAKUB KREJCIK ET AL: "Immunomodulatory Effects and Adaptive Immune Response to Daratumumab in Multiple Myeloma", BLOOD, vol. 126, 3 December 2015 (2015-12-03), page 3037, XP055568280,
- J. KREJCIK ET AL: "Daratumumab depletes CD38+ immune regulatory cells, promotes T-cell expansion, and skews T-cell repertoire in multiple myeloma", BLOOD, vol. 128, no. 3, 24 May 2016 (2016-05-24), pages 384-394, XP055460007, ISSN: 0006-4971, DOI: 10.1182/blood-2015-12-687749
- NAJJAR ET AL.: 'Abstract P227: Accumulation of MDSC Subsets in Renal Cell Carcinoma Correlates with Grade and Progression Free Survival, and is Associated with Intratumoral Expression of IL -1 b, IL -8 and CXCL5' JOURNAL FOR IMMUNOTHERAPY OF CANCER vol. 2, 06 November 2014, pages 110 - 112, XP021202495
- BACHIREDDY ET AL.: 'Haematological Malignancies: at the Forefront of Immunotherapeutic Innovation' NATURE REVIEWS CANCER vol. 15, 01 April 2015, pages 201 - 215, XP055341031
- YE ET AL.: 'Abstract P240: Treg Increases HepG2 Cell Growth by RANK-RANKL pathway' JOUMAL FOR IMMUNOTHERAPY OF CANCER vol. 2, 06 November 2014, pages 115 - 117, XP021202508

Remarks:

The complete document including Reference Table(s) and the Sequence Listing(s) can be downloaded from the EPO website

## Description

### FIELD OF THE INVENTION

The present disclosure relates to the medical use of immune modulation and treatment of solid tumors with antibodies that specifically bind CD38.

### BACKGROUND OF THE INVENTION

The immune system is tightly controlled by a network of costimulatory and co-inhibitory ligands and receptors. These molecules provide secondary signals for T cell activation and provide a balanced network of positive and negative signals to maximize immune responses against infection and tumors, while limiting immunity to self (Wang et al., (Epub Mar. 7, 2011) J Exp Med 208(3):577-92; Lepenies et al., (2008) Endocr Metab Immune Disord Drug Targets 8:279-288).

Immune checkpoint therapy to treat solid tumors, targeting co-inhibitory pathways in T cells to promote antitumor immune responses, has led to advances in clinical care of cancer patients with approval of anti-CTLA-4 and anti-PD-1 antibodies YERVOY® (ipilimumab), KEYTRUDA® (pembrolizumab) and OPDIVO® (nivolumab). While anti-PD-1/PD-L1 antibodies are demonstrating encouraging clinical responses in patients with multiple solid tumors, the response rates are still fairly low, about 15% - 20% in pretreated patients (Swaika et al., (2015) Mol Immunol doi:10.1016/j.molimm.2015.02.009).

While natural killer cells (NK), dendritic cells (DC) and effector T cells are capable of driving potent anti-tumor responses, tumor cells often induce an immunosuppressive microenvironment, favoring the development of immunosuppressive populations of immune cells, such as myeloid-derived suppressor cells (MDSC), regulatory T-cells (Treg) or regulatory B-cells (Breg), which contribute to tumor immune tolerance and the failure of immunotherapy regimens in cancer patients and experimental tumor models.

Thus, there remains a need to develop new cancer immunotherapies that induce adaptive immune response against tumors or target the immunosuppressive immune cells.

M de Weers et al. (2010) The Journal of Immunology vol. 186, no. 3, 27 December 2010, pages 1840-1848, discloses that daratumumab, a novel therapeutic CD38 monoclonal antibody, induces killing of multiple myeloma and other hematological tumors.

Chillemi et al. (2013) Molecular Medicine, vol. 19, no. 1, pages 99-108, discusses the characteristics of target molecule CD38 and anti-CD38 antibody therapy.

WO 2015/009726 A2 discloses medical uses of CD38 agonists.

WO 2012/092612 A1 discloses anti-CD38 antibodies and certain therapeutic and diagnostic uses thereof.

### SUMMARY OF THE INVENTION

The invention is defined by the claims. Specifically, the invention provides an antibody that specifically binds CD38 for use in treating a patient having a solid tumor, comprising the VH of SEQ ID NO: 4 and the VL of SEQ ID NO: 5, wherein the antibody is of IgG1 isotype.

Any references in the description to methods of treatment refer to the compounds, pharmaceutical compositions and medicaments of the present invention for use in a method for treatment of the human (or animal) body by therapy (or for diagnosis). The antibody of the present invention is for use in a method of treating a patient having a solid tumor comprising administering to the patient in need thereof a therapeutically effective amount of the antibody.

The antibody of the present disclosure may also be useful in a method for treating a patient having a regulatory T-cell (Treg) mediated disease comprising administering to the patient in need thereof a therapeutically effective amount of the antibody.

The antibody of the present disclosure may also be useful in a method for treating a patient having a myeloid-derived suppressor cell (MDSC) mediated disease comprising administering to the patient in need thereof a therapeutically effective amount of the antibody.

The antibody of the present disclosure may also be useful in a method for treating a patient having a regulatory B-cell (Breg) mediated disease comprising administering to the patient in need thereof a therapeutically effective amount of the antibody.

The antibody of the present disclosure may also be useful in a method of suppressing activity of a regulatory T-cell (Treg), comprising contacting the Treg with the antibody.

The antibody of the present disclosure may also be useful in a method of suppressing activity of a myeloid-derived suppressor cell (MDSC), comprising contacting the MDSC with the antibody.

The antibody of the present disclosure may also be useful in a method of suppressing activity of a regulatory B-cell (Breg), comprising contacting the Breg with the antibody.

The antibody of the present disclosure may also be useful in a method of enhancing an immune response in a patient, comprising administering to the patient the antibody.

The antibody for use of the present invention may also be useful in treating a patient having a solid tumor comprising reducing the number of Tregs cells in the patient.

The antibody for use of the present invention may also be useful in treating a patient having a solid tumor, comprising reducing the number of myeloid-derived suppressor cells (MDSC) in the patient.

The antibody of the present disclosure may also be useful in a method of of suppressing activity of an immune suppressor cell, comprising contacting the immune suppressing cell with the antibody.

The antibody of the present disclosure may also be useful in a method of treating a patient having a viral infection, comprising administering to the patient in need thereof the antibody.

**BRIEF DESCRIPTION OF THE DRAWINGS**

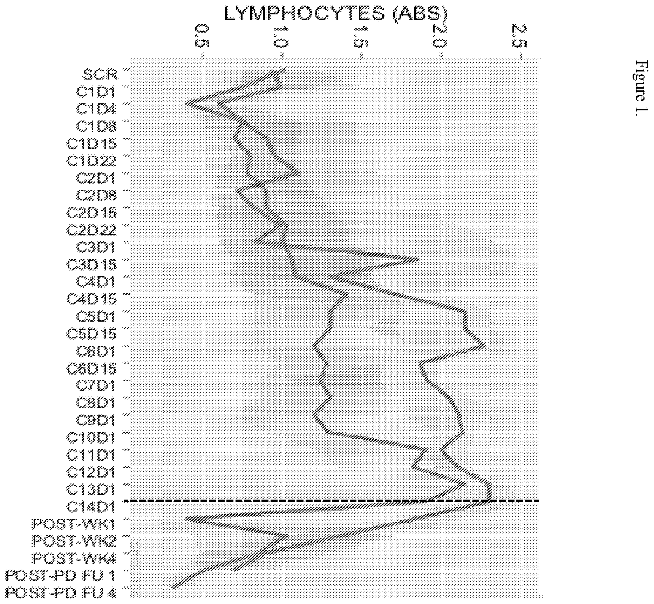

**Figure 1** shows that the median number of lymphocytes was increased in patients responding to DARZALEX™ (daratumumab) treatment at 8 mg/kg (upper line) or 16 mg/kg (lower line) doses over time, and that the lymphocyte numbers returned to baseline after end of treatment. Study: SIRIUS. X-axis indicates time expressed as treatment cycle and days of dosing within each treatment cycle (C1D1: cycle 1, day 1; C1D4; cycle 1, day 4, etc). SCR: baseline; EOT: end of treatment; WK: week; POST-WK: post-treatment at indicated weeks; post-PD FU: follow-up after progression. The highlighted areas in gray shades indicate the 25-27% Interquartile Range (IQR) for the data points for each visit for responders.

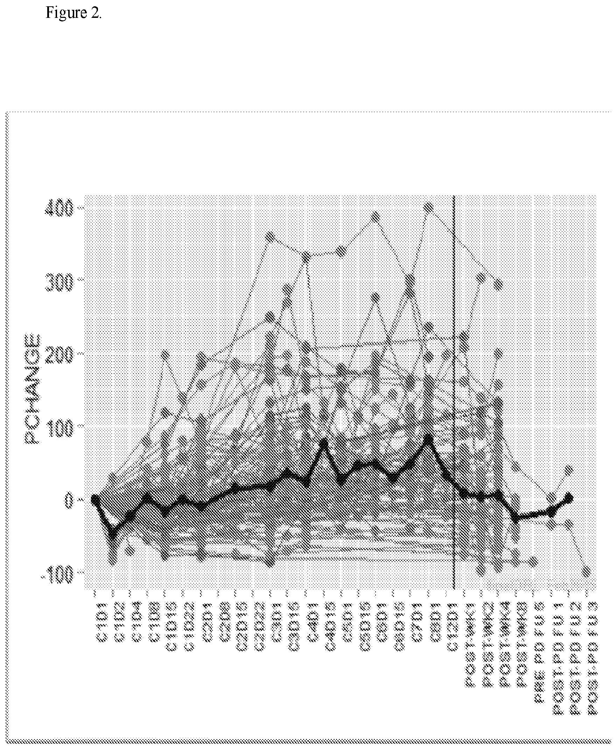

**Figure 2** shows the percent (%) change of absolute counts of CD3$^+$ T cells to baseline in peripheral blood in patients treated with DARZALEX™ (daratumumab) for each individual patient (light gray lines). Study: SIRIUS (MMY2002). The X-axis indicates time expressed as treatment cycle and days of dosing within each treatment cycle (C1D1: cycle 1, day 1; C1D4; cycle 1, day 4, etc). WK: week; POST-WK: post-treatment at indicated weeks; POST-PD FU: follow-up after progression. The black line shows the median % change for all patients.

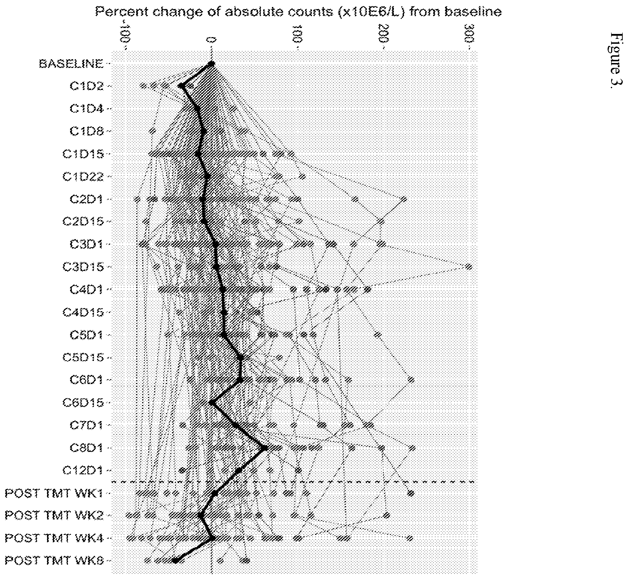

**Figure 3** shows the percent (%) change of absolute counts of CD4$^+$ T cells to baseline in peripheral blood in patients treated with DARZALEX™ (daratumumab) for each individual patient (light gray lines). Study: SIRIUS. The X-axis indicates time expressed as treatment cycle and days of dosing within each treatment cycle (C1D1: cycle 1, day 1; C1D4; cycle 1, day 4, etc). WK: week; POST-TMT: post-treatment. The black line shows the median % change for all patients.

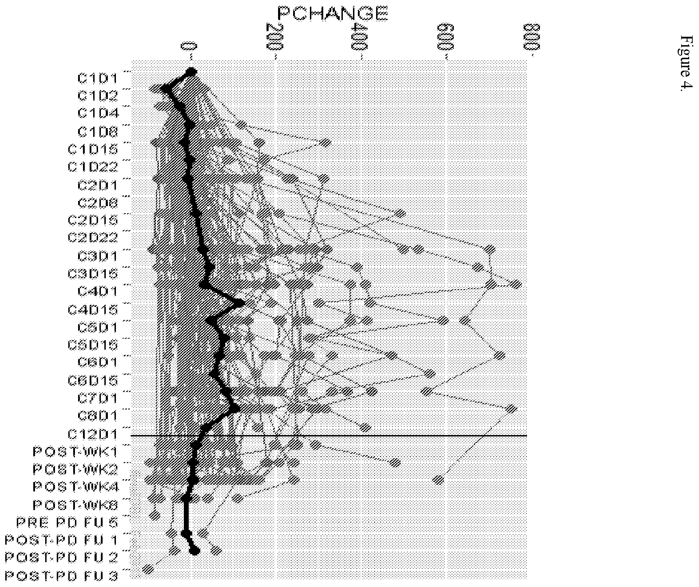

**Figure 4** shows the percent (%) change of absolute counts of CD8$^+$ T cells to baseline in peripheral blood in patients treated with DARZALEX™ (daratumumab) for each individual patient (light gray lines). Study: SIRIUS. The X-axis indicates time expressed as treatment cycle and days of dosing within each treatment cycle (C1D1: cycle 1, day 1; C1D4; cycle 1, day 4, etc). WK: week; Pre-PD FU: follow-up before progression; Post-PD FU: follow-up after progression. The black line shows the median % change for all patients.

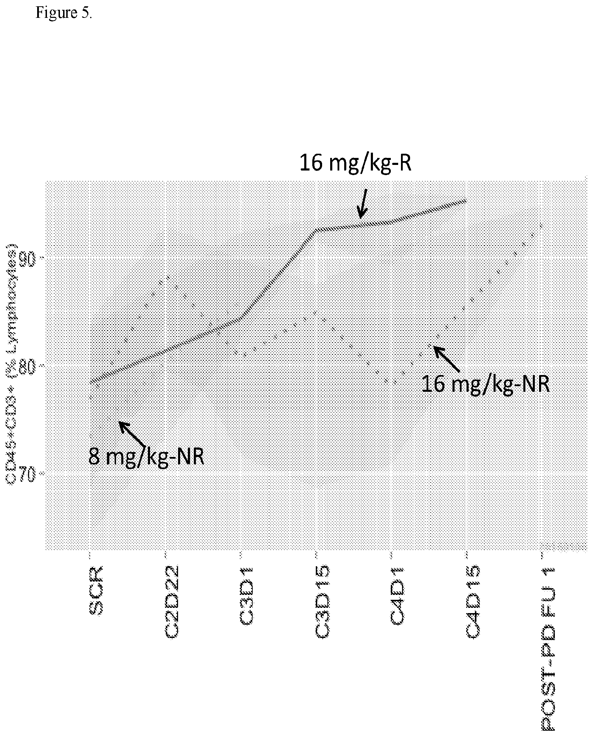

**Figure 5** shows that the number of CD45$^+$CD3$^+$ cells (measured as percentage of lymphocytes) in bone marrow aspirates was increased during DARZALEX™ (daratumumab) treatment over time at doses 8 mg/kg or 16 mg/kg. The graph includes both responders and non-responders as indicated. Study: SIRIUS. The X-axis indicates time expressed as treatment cycle and days of dosing within each treatment cycle (C2D22: cycle 2, day 22; etc). SCR: baseline; Post-PD FU1: follow-up after progression. The highlighted areas in gray shade indicate the 25-27% Interquartile Range (IQR) for the data points for each visit for the non-responders dosed at 8 mg/kg, the responders dosed at 16 mg/kg, or the non-responders dosed at 16 mg/kg, respectively. NR: no-responder; R: responder.

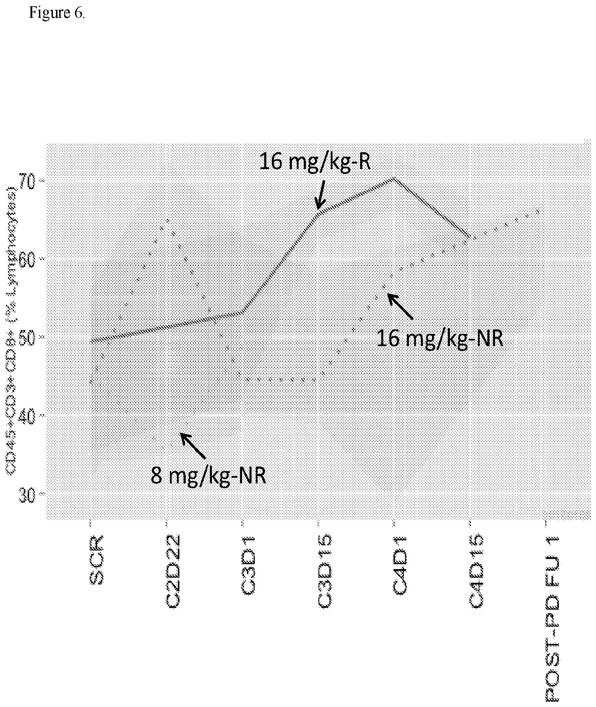

**Figure 6** shows that the number of CD45$^+$ CD3$^+$ CD8$^+$ cells (measured as percentage of lymphocytes) in bone marrow aspirates was increased during DARZALEX™ (daratumumab) treatment over time at doses 8 mg/kg or 16 mg/kg. The graph includes both responders and non-responders as indicated. Study: SIRIUS. The X-axis indicates time expressed as treatment cycle and days of dosing within each treatment cycle (C2D22: cycle 2, day 22; etc). SCR: baseline; Post-PD FU1: follow-up after progression. The highlighted areas in gray shade indicate the 25-27% Interquartile Range (IQR) for the data points for each visit for the non-responders dosed at 8 mg/kg, the responders dosed at 16 mg/kg, or the non-responders dosed at 16 mg/kg, respectively. NR: no-responder; R: responder.

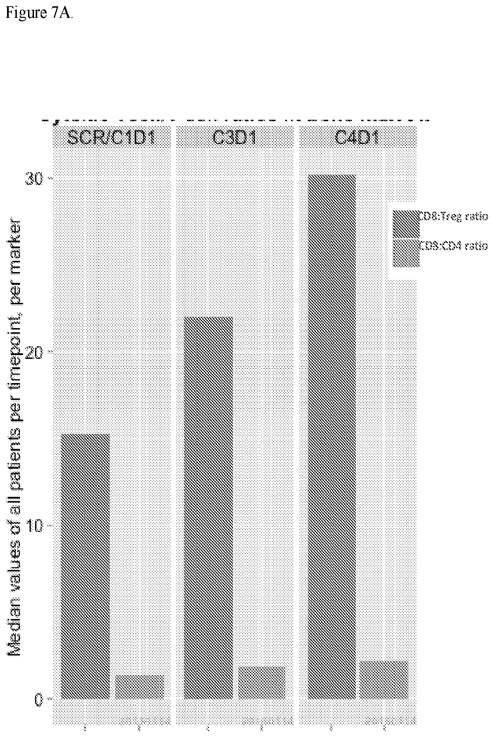

**Figure 7A** shows that the ratio of CD8$^+$/ Treg and CD8$^+$/CD4$^+$ cells in peripheral blood expressed as median values of all treated patients increased over time during DARZALEX™ (daratumumab) treatment. Time points: C1D1: cycle 1 day; C3D1: cycle 3 day 1; C4D1: cycle 4 day 1. Study: SIRIUS. SRC: baseline.

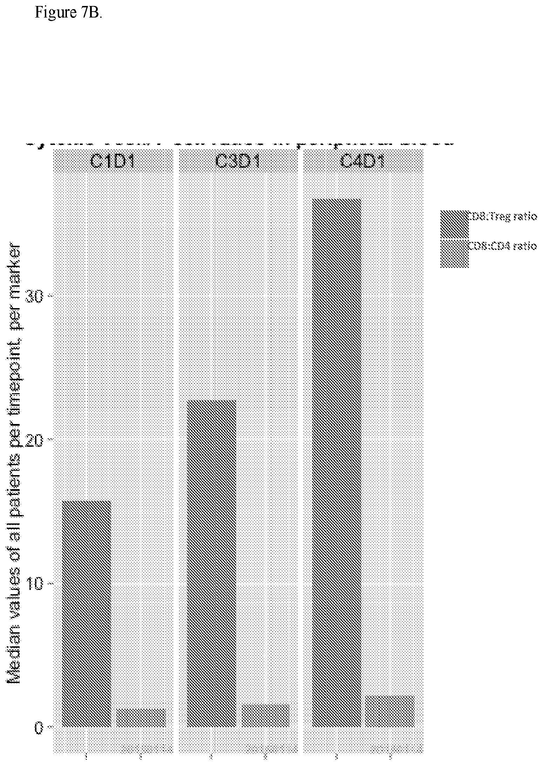

**Figure 7B** shows that the ratio of CD8$^+$/Treg cells in bone marrow aspirates expressed as median values of all treated patients increased over time during DARZALEX™ (daratumumab) treatment. Time points: C1D1: cycle 1 day; C3D1: cycle 3 day 1; C4D1: cycle 4 day 1. Study: SIRIUS.

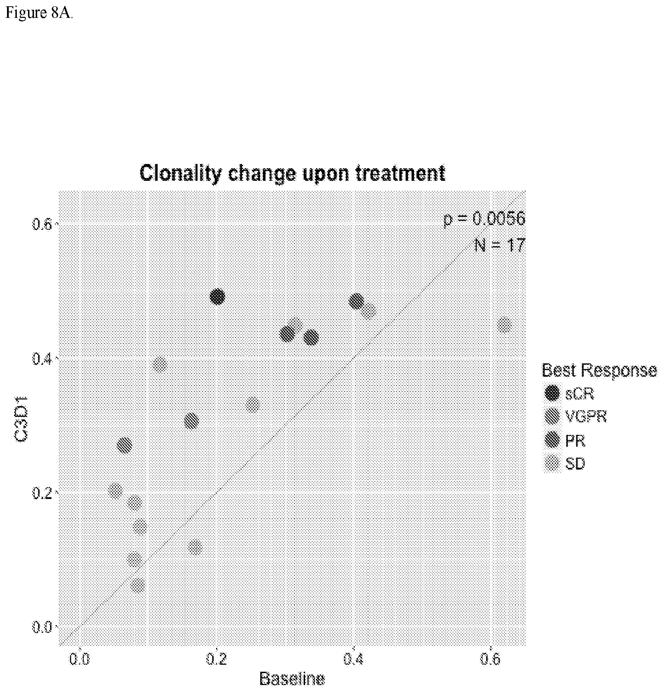

**Figure 8A** shows that responders had increased CD8$^+$ T-cell clonality when compared to non-responders, as measured using % change in abundance (CIA) of particular clonal cells. Study: GEN501 17 patient subset.

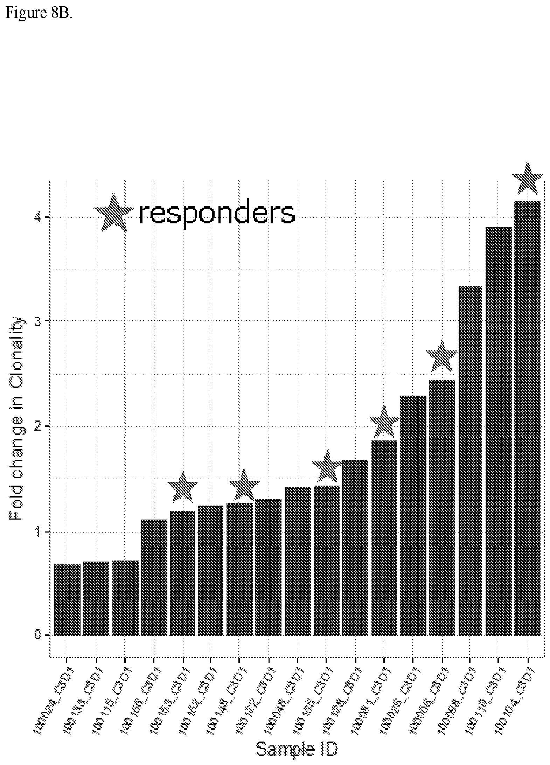

**Figure 8B** shows the fold change in CD8[+] T-cell clonality in individual patients pre- vs. post DARZALEX™ (daratumumab) treatment. Responders are indicated with the star. Clonality was measured as fold change in abundance (CIA) of particular clonal cells. Study: GEN501 17 patient subset.

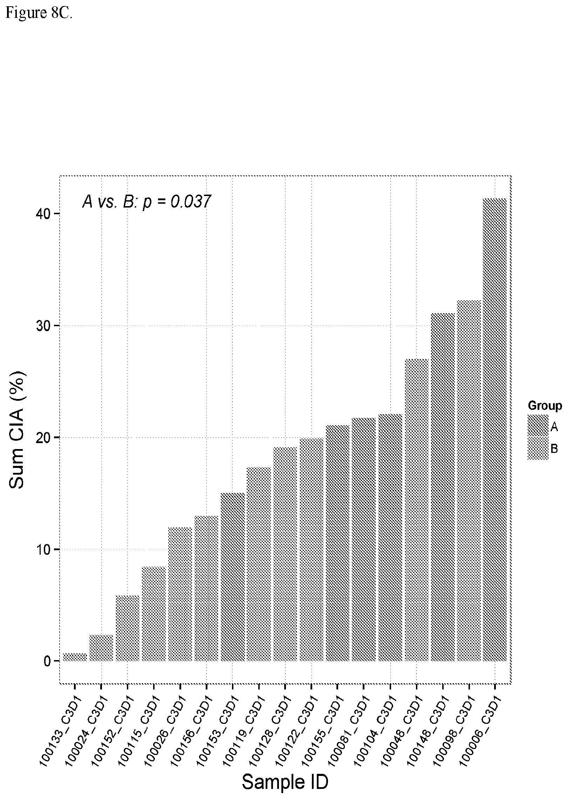

**Figure 8C** shows that responders (Group A) had greater total expansion in the TCR repertoire, measured using CIA (change in abundance) when compared to non-responders (Group B). P=0.037. Study: GEN501 17 patient subset.

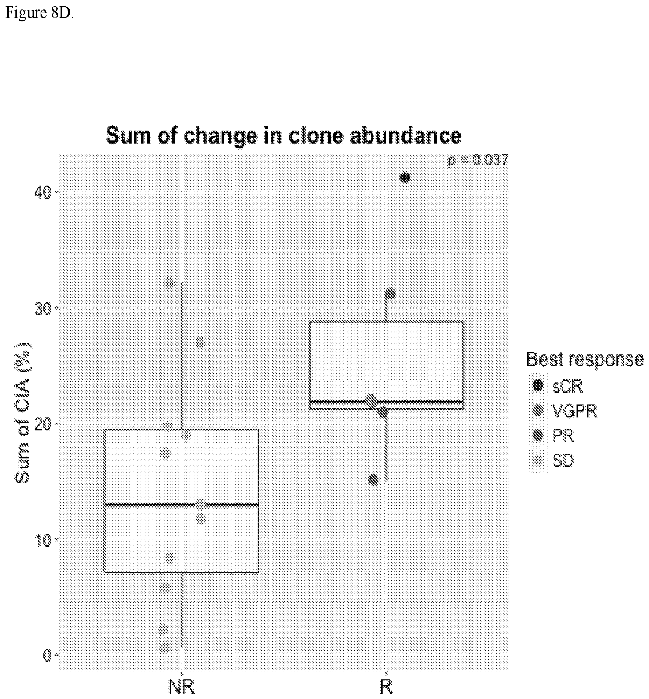

**Figure 8D** shows the sum of absolute change in abundance (CIA) in responders and non-responders for each expanded T cell clone. P=0.035 between responders (Group A) and non-responders (Group B). Study: GEN501 17 patient subset.

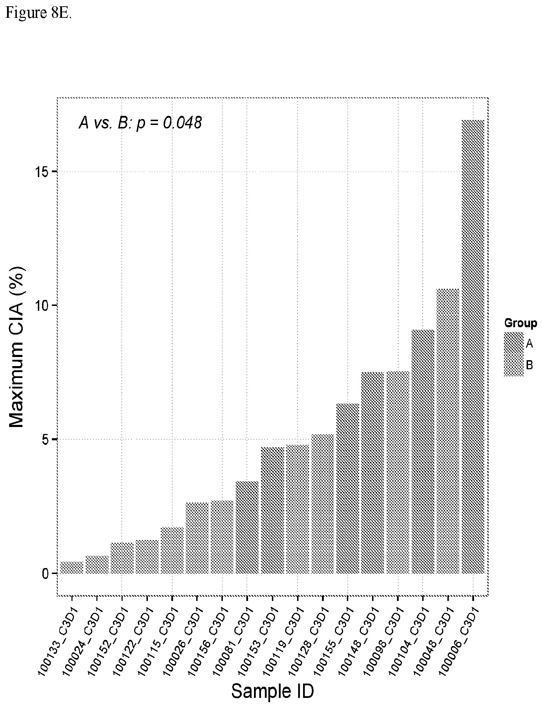

**Figure 8E** shows the maximum CIA of a single T-cell clone in responders (Group A) and non-responders (Group B). Study: GEN501 17 patient subset.

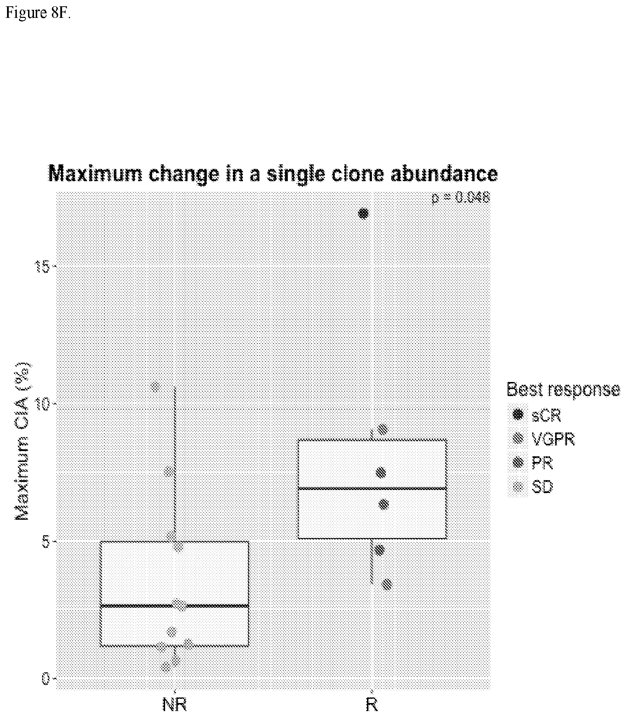

**Figure 8F** shows that responders (Group A) had greater maximum expansion of a single clone, measured using maximum % CIA, when compared to non-responders (Group B). P=0.0477. Study: GEN501 17 patient subset.

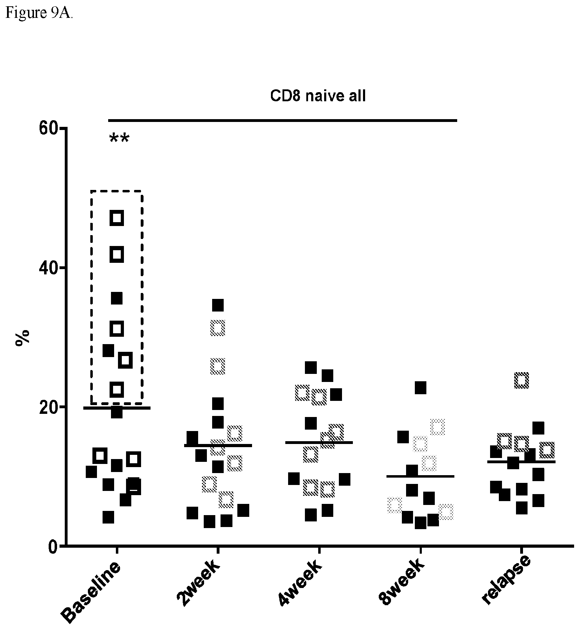

**Figure 9A** shows the percentage (%) of CD8[+] naive cells in peripheral blood in non-responders (NR, black squares) and in patients having at least minimal response (MR, white squares) to DARZALEX™ (daratumumab) at baseline, or at 2 weeks, 4 weeks or 8 weeks of treatment, or after relapse. Study: GEN501 17 patient subset. $**p=1.82 \times 10^{-4}$.

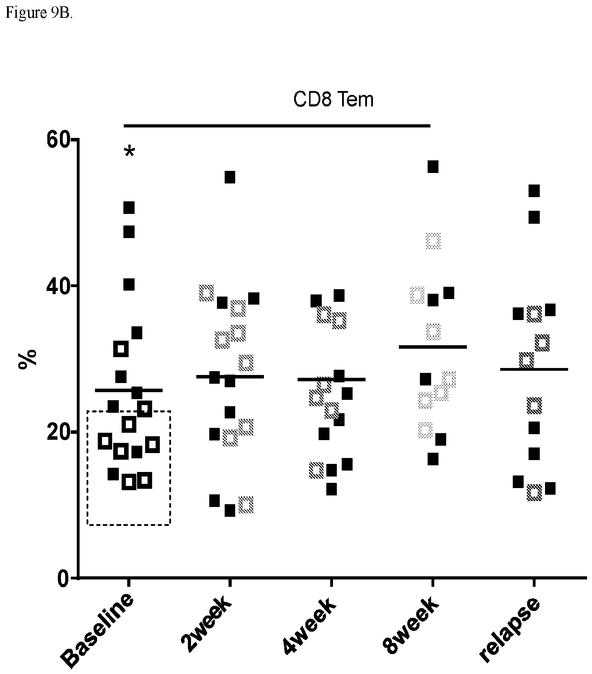

**Figure 9B** shows the percentage of CD8[+] central memory cells (Tem) in peripheral blood in non-responders (NR, black squares) and in patients having at least minimal response (MR, white squares) to DARZALEX™ (daratumumab) at baseline, or at 2 weeks, 4 weeks or 8 weeks of treatment, or after relapse. Study: GEN501 17 patient subset. $*p=4.88 \times 10^{-2}$.

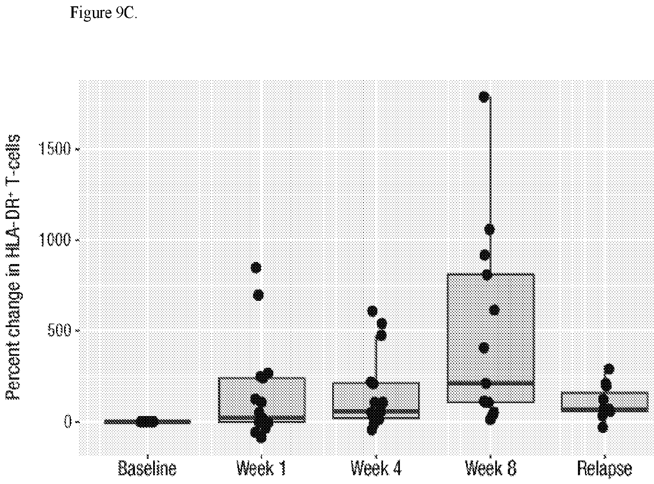

**Figure 9C** shows the percentage increase of HLA Class I restricted CD8[+] T cells in peripheral blood at baseline, or at week 1, 4 or 8 of treatment, or after relapse. Study: GEN501 17 patient subset.

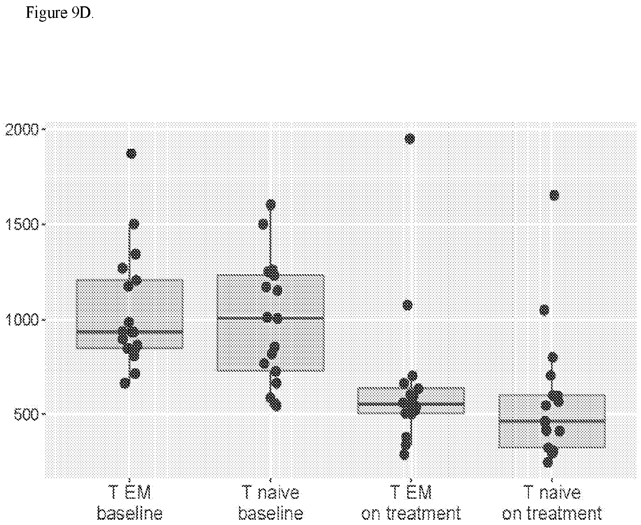

**Figure 9D** shows that CD38 is expressed at low levels in CD8[+] naïve T cells and in CD8[+] central memory cells (Tem) in peripheral blood at bassline or on treatment. Study: GEN501 17 patient subset. MFI: Mean fluorescent intensity.

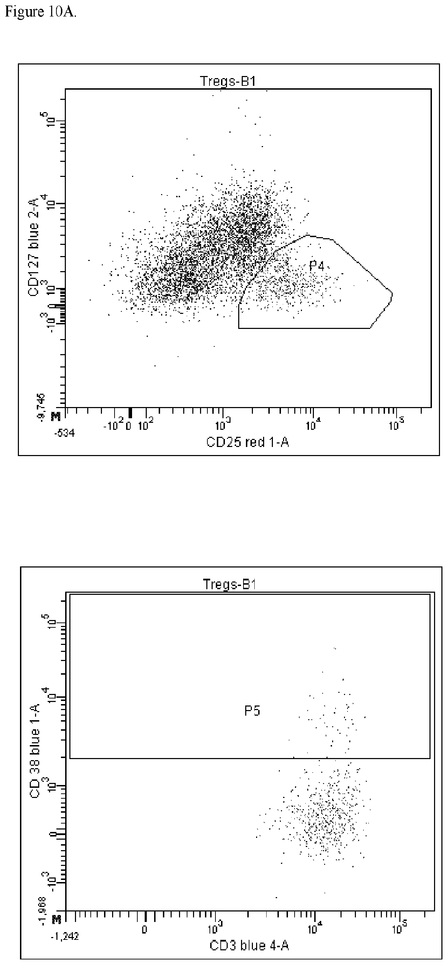

**Figure 10A** shows a histogram of FACS analyses showing frequency of Tregs (CD3[+] CD3[+]CD4[+]CD25[+]CD127[dim] (top histogram, P4 cell population) and the frequency of CD38[+] Tregs within the Treg population (bottom histogram, P5 cell population) in multiple myeloma patients at baseline. Study: GEN501 17 patient subset.

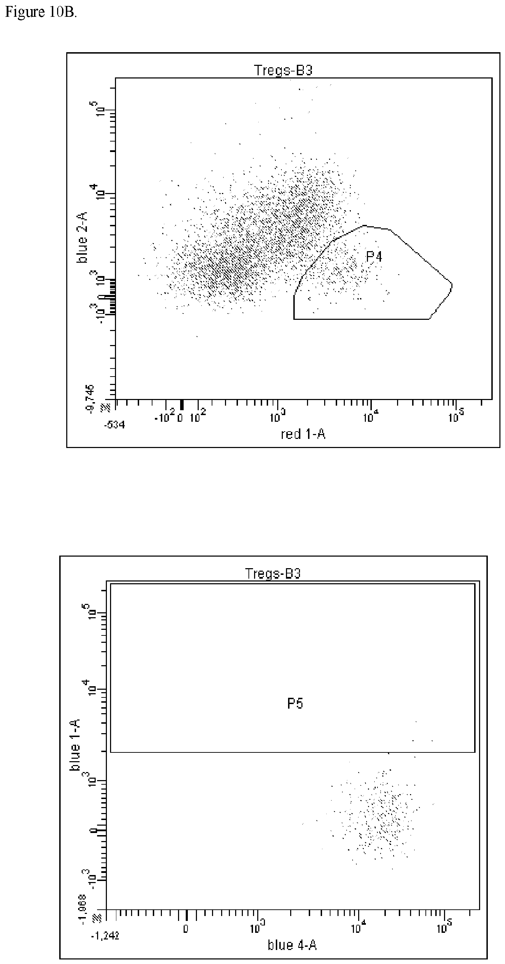

**Figure 10B** shows a histogram of FACS analyses showing frequency of Tregs (CD3[+] CD3[+]CD4[+]CD25[+]CD127[dim] (top histogram, P4 cell population) and the frequency of CD38[+] Tregs within the Treg population (bottom histogram, P5 cell population) in multiple myeloma patients after DARZALEX™ (daratumumab) treatment. DARZALEX™ (daratumumab) treatment depleted CD38[+] Tregs. Study: GEN501 17 patient subset.

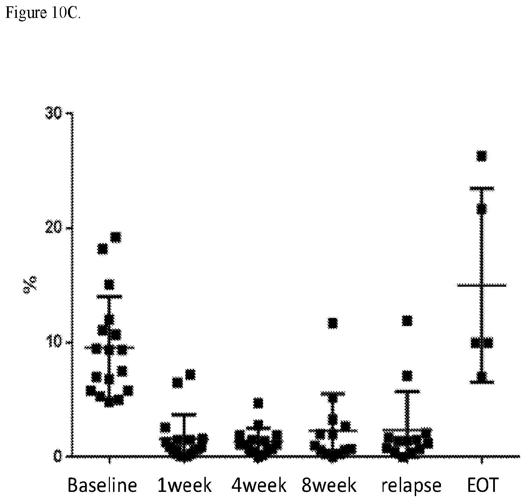

**Figure 10C** shows that frequency of the CD38[high] CD3[+]CD4[+]CD25[+]CD127[dim] Tregs in patients treated with DARZALEX™ (daratumumab) at baseline, or at 1 week, 4 week, 8weeks, after relapse or at end of treatment at 6 months (EOT). CD38[high] Treg frequency was reduced with DARZALEX™ (daratumumab) treatment and returned to baseline at EOT. Y-axis: % of CD38[lugh]CD3[+]CD4[+]CD25[+]CD127[dim] Tregs from CD3[+] T-cells. Study: GEN501 17 patient subset.

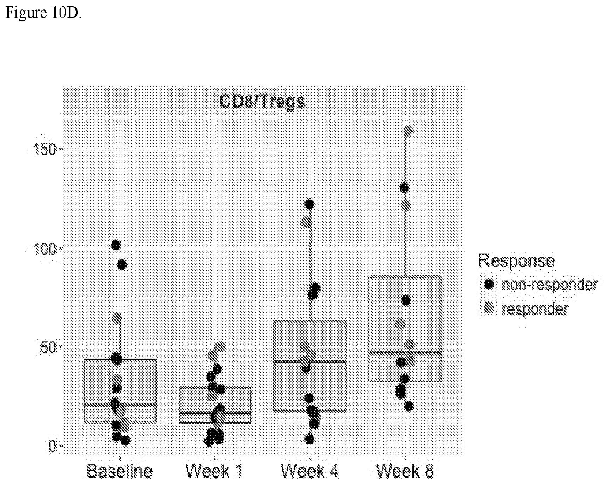

**Figure 10D** shows the CD8[+]/Treg cell ratio in responders and non-responders at baseline, at 1 week, 4 weeks and 8 weeks of treatment. The CD8[+]/Treg cell ratio was significantly higher in responders vs. non-responders (p=0.00955) at Week 8 of treatment. Study: GEN501 17 patient subset.

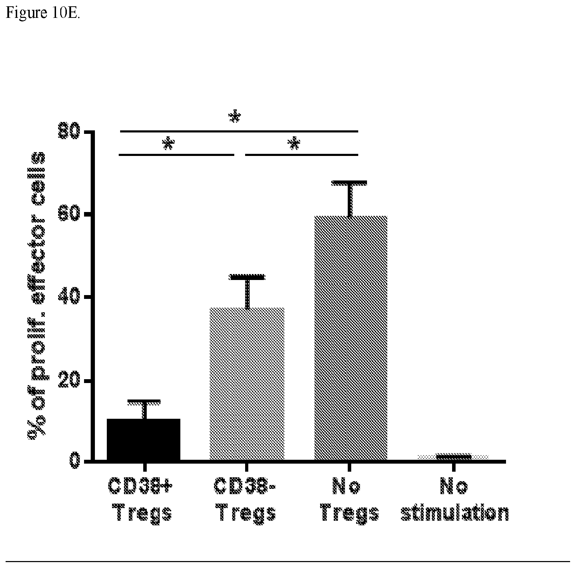

**Figure 10E** shows that effector cell proliferation is inhibited more efficiently in the presence of CD38[+] Tregs when compared to the CD38[-] Tregs or negative controls. Error bars represent standard error. Asterisks denote significant changes. Samples were obtained from multiple healthy donors. Cell proliferation was assessed through the dilution of carboxyfluorescein succinimidyl ester (CFSE).

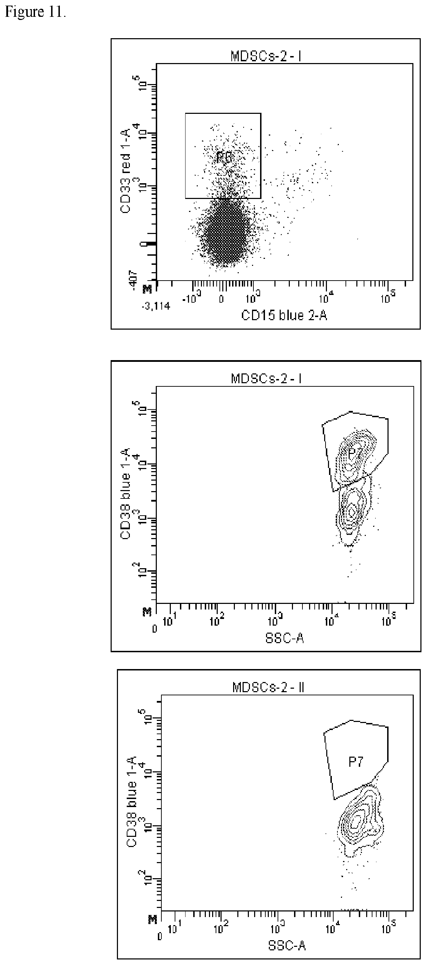

**Figure 11** shows that Myeloid-derived suppressor cells (MDSC) are present in multiple myeloma patients (top graph, boxed cells) and that about half of the cells expressed CD38 (middle graph, boxed cells). The CD38high MDSC population was depleted in patients treated with one infusion of DARZALEX™ (daratumumab) (bottom graph, boxed cells). Study: GEN501 17 patient subset.

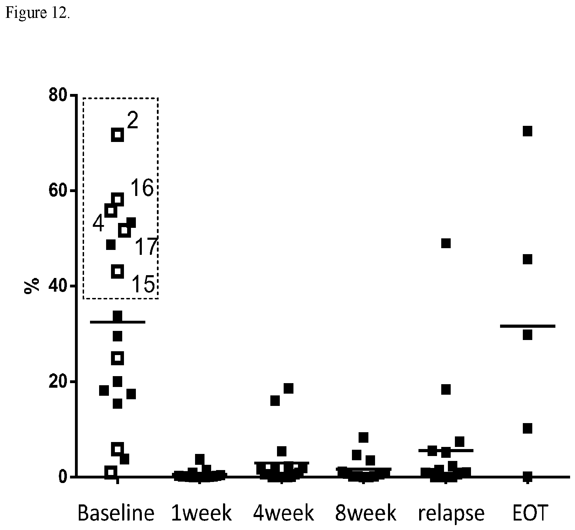

**Figure 12** shows that the number of CD38high MDSCs (CD11b[+]HLADR[-]CD14[-]CD33[+]CD15[+]) was reduced in patients after 1 week, 4 week or 8 week treatment with DARZALEX™ (daratumumab) when compared to the baseline, and returned to close to baseline after end of treatment (EOT). Relapsed patients still demonstrated reduced CD38high MDSCs. Black squares: non-responders; white squares: patients with at least Minimal Response to DARZALEX™ (daratumumab) treatment. The vertical lines indicate the median values in each group. Patients 2, 4, 15, 16 and 17 demonstrated high initial CD38high MDSCs population. Study: GEN501 17 patient subset.

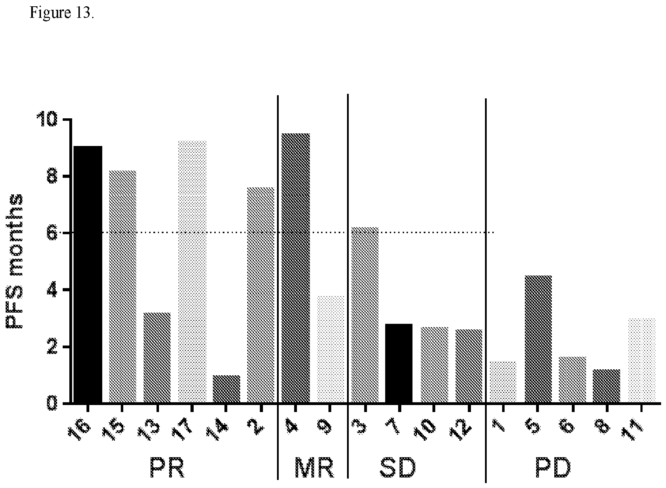

**Figure 13** shows that the patients with highest CD38high MDSCs (patients 2, 4, 15, 16 and 17) had the highest Progression-Free Survival (PFS). These patients had either partial Response (PR) or Minimal Response (MR) to DARZALEX™ (daratumumab) treatment. SD : Stable Disease; PD : Progressive Disease. X-axis shows the PFS for each individual numbered patient.

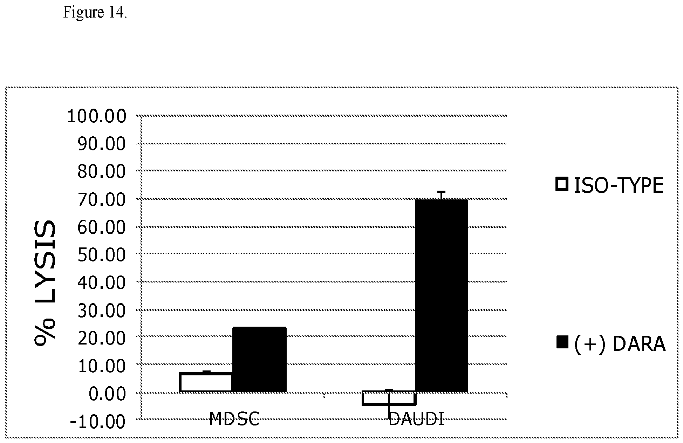

**Figure 14** shows that MDSC are sensitive to DARZALEX™ (daratumumab)-induced ADCC. Daudi cells were used as positive control for target cells in the assays. % cell lysis was measured.

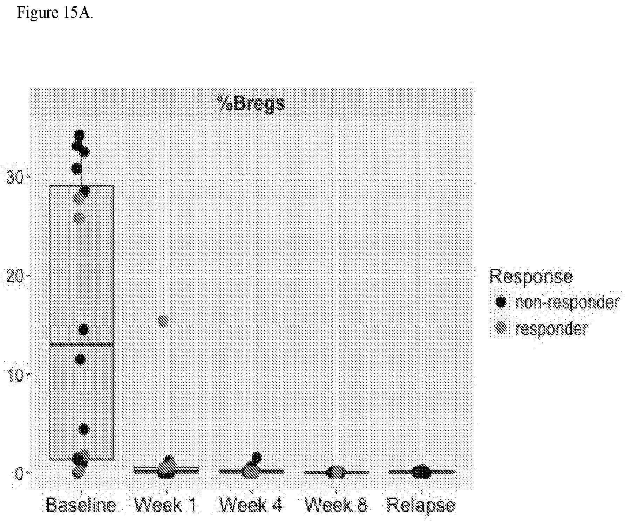

**Figure 15A** shows that CD38+ Bregs were depleted in patients treated with DARZALEX™ (daratumumab) at Week1, Week 4 and Week 8 of treatment.

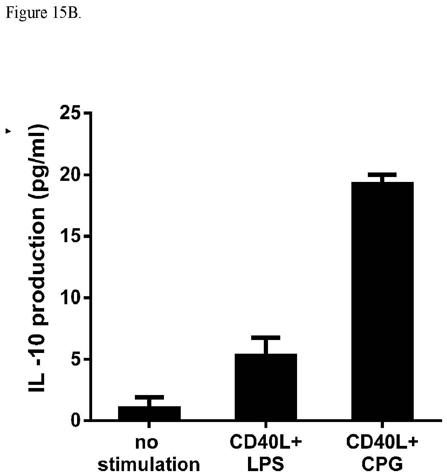

**Figure 15B** shows that CD38+ Bregs secrete IL-10 upon stimulation.

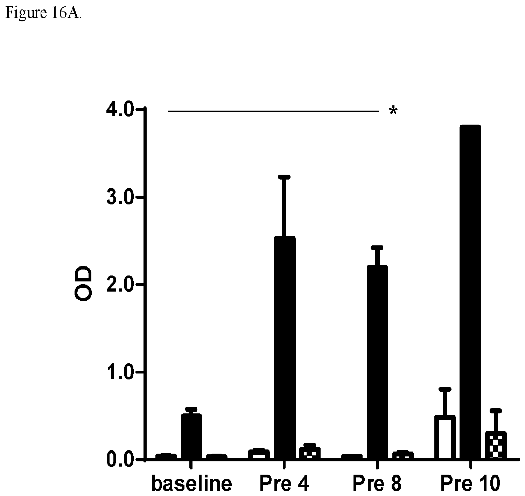

**Figure 16A** shows the anti-viral response measured through CMV, EBV and influenza virus specific (CEF) IFN-y production in PBMCs from DARZALEX™ (daratumumab) treated patient with VGPR at baseline and at indicated times during treatment. OD: optical density. White bar: negative control; black bar: CEF added; dashed bar: allogeneic PBMCs only. Asterix indicates a statistically significant change. Pre 4, 8, 10 = Week 4, 8 or 10 of treatment.

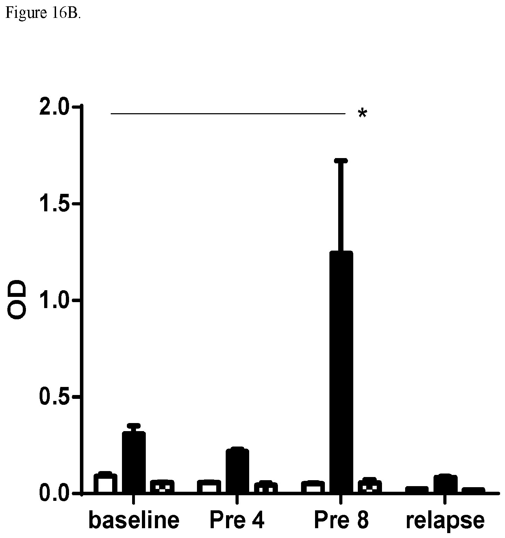

**Figure 16B** shows the anti-viral response measured through CMV, EBV and influenza virus specific (CEF) IFN-y production in PBMCs from DARZALEX™ (daratumumab) treated patient with CR at baseline and at indicated times during treatment. OD: optical density. White bar: negative control; black bar: CEF added; dashed bar: allogeneic PBMCs only. Asterix indicates a statistically significant change. Pre 4, 8, 10 = Week 4, 8 or 10 of treatment.

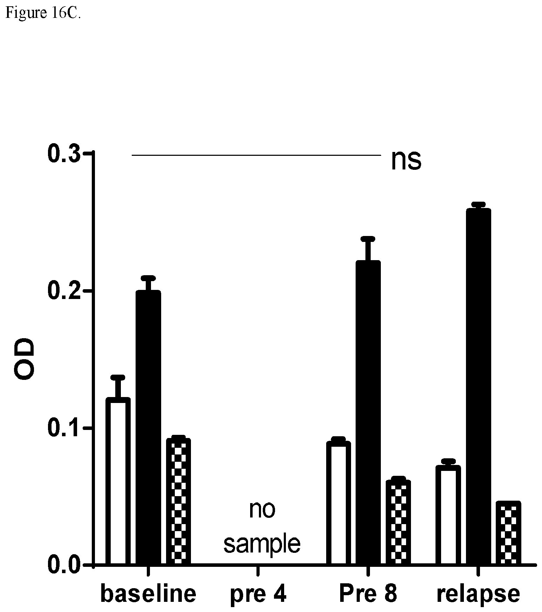

**Figure 16C** shows the anti-viral response measured through CMV, EBV and influenza virus specific (CEF) IFN-y production in PBMCs from DARZALEX™ (daratumumab) treated patient with PD at baseline and at indicated times during treatment. OD: optical density. White bar: negative control; black bar: CEF added; dashed bar: allogeneic PBMCs only. Ns: not significant. Pre 4, 8 = Week 4 or 8 of treatment.

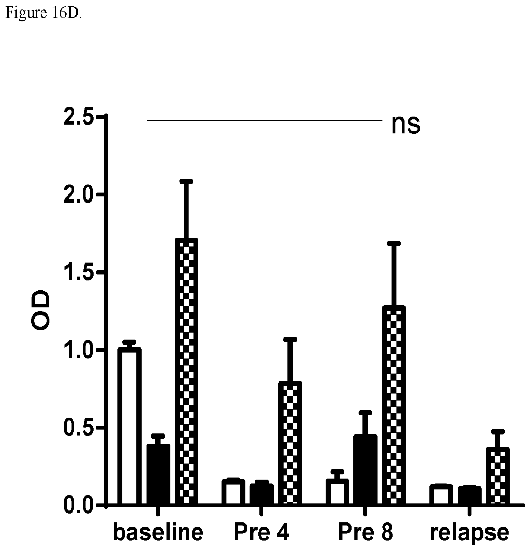

**Figure 16D** shows the anti-viral response measured through CMV, EBV and influenza virus specific (CEF) IFN-y production in PBMCs from DARZALEX™ (daratumumab) treated patient with MR at baseline and at indicated times during treatment. OD: optical density. White bar: negative control; black bar: CEF added; dashed bar: allogeneic PBMCs only. Ns: not significant. Pre 4, 8 = Week 4 or 8 of treatment.

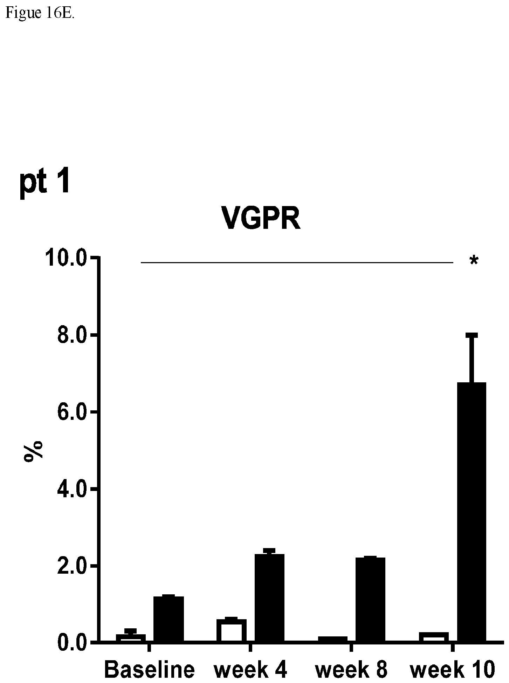

**Figure 16E** shows the percentage (%) of proliferating virus-reactive T cells in PBMCs from DARZALEX™ (daratumumab) treated patient with VGPR at baseline and at indicated times during treatment. White bar: negative control; black bar: CEF added. Asterix indicates a statistically significant change. Pre 4, 8, 10 = Week 4, 8 or 10 of treatment.

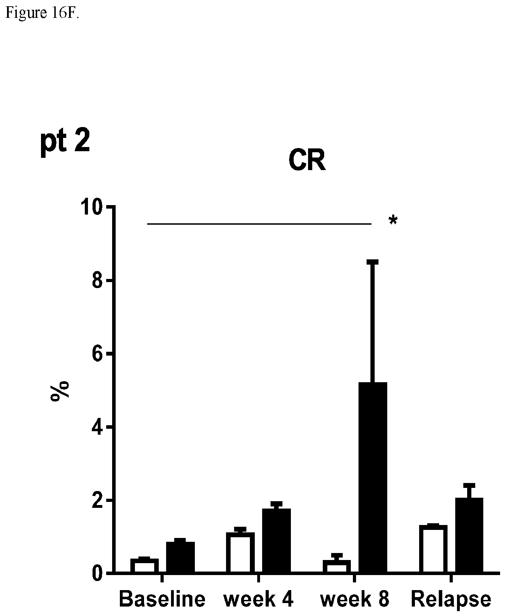

**Figure 16F** shows the percentage (%) of proliferating virus-reactive T cells in PBMCs from DARZALEX™ (daratumumab) treated patient with CR at baseline and at indicated times during treatment. White bar: negative control; black bar: CEF added. Asterix indicates a statistically significant change. Pre 4, 8, 10 = Week 4, 8 or 10 of treatment.

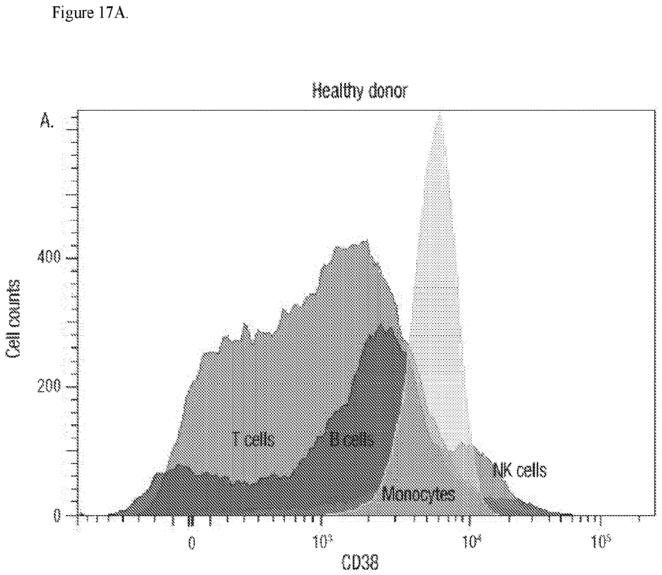

**Figure 17A** shows a histogram of FACS analyses showing CD38 expression levels on natural killer cells (NK), monocytes, B cells and T cells from a healthy donor.

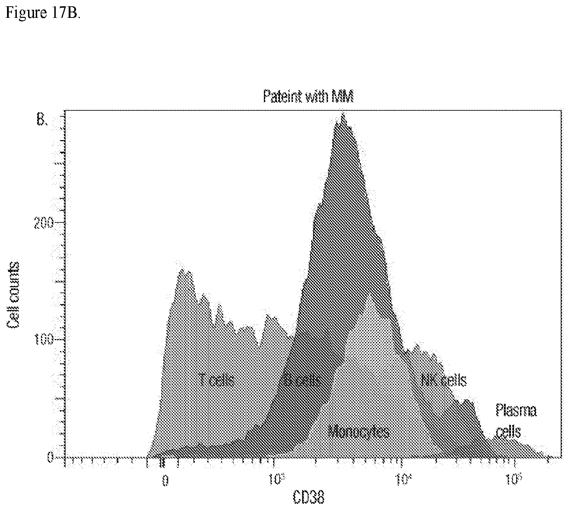

**Figure 17B** shows a histogram of FACS analyses showing CD38 expression levels on plasma cells, natural killer cells (NK), monocytes, B cells and T cells from a multiple myeloma patient.

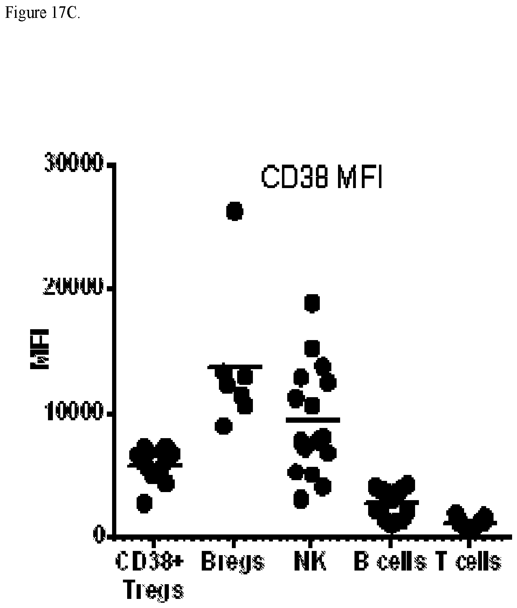

**Figure 17C** shows a comparison of the mean fluorescent intensity (MFI) of CD38 in CD38+ Tregs, Bregs, NK, B cells and T cells from relapsed and refractory multiple myeloma patients. CD38 was expressed at lower level in B cells and T cells when compared to the CD38+Tregs, Bregs and NK cells.

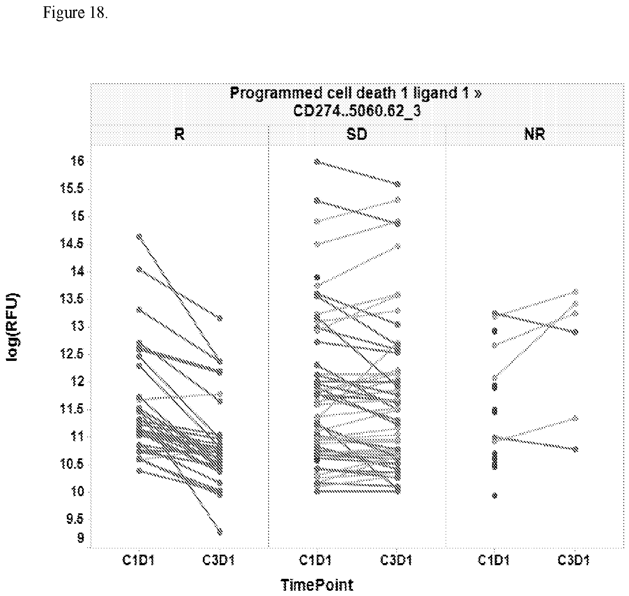

**Figure 18** shows that PD-L1 protein is downregulated in PBMC samples from responders (R) and upregulated in non-responders (NR) overtime. SD: stable disease. C1D1: cycle 1 day 1; C3D 1, cycle 3, day 1. Y axis shows the log2 protein concentration values.

## DETAILED DESCRIPTION

As used in this specification and the appended claims, the singular forms "a," "an," and "the" include plural referents unless the content clearly dictates otherwise. Thus, for example, reference to "a cell" includes a combination of two or more cells, and the like.

"CD38" refers to the human CD38 protein (synonyms: ADP-ribosyl cyclase 1, cADPr hydrolase 1, cyclic ADP-ribose hydrolase 1). Human CD38 has an amino acid sequence shown in GenBank accession number NP_001766 and in SEQ ID NO: 1. It is well known that CD38 is a single pass type II membrane protein with amino acid residues 1-21 representing the cytosolic domain, amino acid residues 22-42 representing the transmembrane domain, and residues 43-300 representing the extracellular domain of CD38.

SEQ ID NO: 1

MANCEFSPVSGDKPCCRLSRRAQLCLGVSILVLILVVVLAVVVPRWRQQWSGPGT
TKRFPETVLARCVKYTEIHPEMRHVDCQSVWDAFKGAFISKHPCNITEEDYQPLM
KLGTQTVPCNKILLWSRIKDLAHQFTQVQRDMFTLEDTLLGYLADDLTWCGEFN
TSKINYQSCPDWRKDCSNNPVSVFWKTVSRRFAEAACDVVHVMLNGSRSKIFDK
NSTFGSVEVHNLQPEKVQTLEAWVIHGGREDSRDLCQDPTIKELESIISKRNIQFSC
KNIYRPDKFLQCVKNPEDSSCTSEI

"Antibodies" as used herein is meant in a broad sense and includes immunoglobulin molecules including monoclonal antibodies including murine, human, humanized and chimeric monoclonal antibodies, antibody fragments, bispecific or multispecific antibodies, dimeric, tetrameric or multimeric antibodies, single chain antibodies, domain antibodies and any other modified configuration of the immunoglobulin molecule that comprises an antigen binding site of the required specificity.

Immunoglobulins may be assigned to five major classes, namely IgA, IgD, IgE, IgG and IgM, depending on the heavy chain constant domain amino acid sequence. IgA and IgG are further sub-classified as the isotypes IgA1, IgA2, IgG1, IgG2, IgG3 and IgG4. Antibody light chains of any vertebrate species can be assigned to one of two clearly distinct types, namely kappa ($\kappa$) and lambda ($\lambda$), based on the amino acid sequences of their constant domains.

"Antibody fragments" refers to a portion of an immunoglobulin molecule that retains the heavy chain and/or the light chain antigen binding site, such as heavy chain complementarity determining regions (HCDR) 1, 2 and 3, light chain complementarity determining regions (LCDR) 1, 2 and 3, a heavy chain variable region (VH), or a light chain variable region (VL). Antibody fragments include a Fab fragment, a monovalent fragment consisting of the VL, VH, CL and CH1 domains; a F(ab)$_2$ fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a domain antibody (dAb) fragment (Ward et al., Nature 341:544- 6, 1989), which consists of a VH domain. VH and VL domains may be engineered and linked together via a synthetic linker to form various types of single chain antibody designs in which the VH/VL domains pair intramolecularly, or intermolecularly in those cases when the VH and VL domains are expressed by separate single chain antibody constructs, to form a monovalent antigen binding site, such as single chain Fv (scFv) or diabody; described for example in Intl. Pat. Publ. Nos. WO1998/44001, WO1988/01649, WO1994/13804, and WO1992/01047. These antibody fragments are obtained using well known techniques known to those of skill in the art, and the fragments are screened for utility in the same manner as are full length antibodies.

"Isolated antibody" refers to an antibody or antibody fragment that is substantially free of other antibodies having different antigenic specificities (e.g., an isolated antibody specifically binding CD38 is substantially free of antibodies that specifically bind antigens other than human CD38). An isolated antibody that specifically binds CD38, however, may have cross-reactivity to other antigens, such as orthologues of human CD38, such as *Macaca fascicularis* (cynomolgus) CD38. In case of a bispecific antibody, the bispecific antibody specifically binds two antigens of interest, and is substantially free of antibodies that specifically bind antigens other that the two antigens of interest. Moreover, an isolated antibody may be substantially free of other cellular material and/or chemicals. "Isolated antibody" encompasses antibodies that are isolated to a higher purity, such as antibodies that are 80%, 81%, 82%, 83%, 84%, 85%, 86%, 87%, 88%, 89%, 90%, 91%, 92%, 93%, 94%, 95%, 96%, 97%, 98%, 99% or 100% pure.

"Specific binding" or "specifically binds" or "binds" refers to an antibody binding to an antigen or an epitope within the antigen with greater affinity than for other antigens. Typically, the antibody binds to the antigen or the epitope within the antigen with an equilibrium dissociation constant ($K_D$) of about $1 \times 10^{-8}$ M or less, for example about $1 \times 10^{-9}$ M or less, about $1 \times 10^{-10}$ M or less, about $1 \times 10^{-11}$ M or less, or about $1 \times 10^{-12}$ M or less, typically with the $K_D$ that is at least one hundred fold less than its $K_D$ for binding to a nonspecific antigen (e.g., BSA, casein). The dissociation constant may be measured using standard procedures. Antibodies that specifically bind to the antigen or the epitope within the antigen may, however, have cross-reactivity to other related antigens, for example to the same antigen from other species (homologs), such as human or monkey, for example *Macaca fascicularis* (cynomolgus, cyno), *Pan troglodytes* (chimpanzee, chimp) or *Callithrix jacchus* (common marmoset, marmoset). While a monospecific antibody specifically binds one antigen or one epitope, a bispecific antibody specifically binds two distinct antigens or two distinct epitopes.

An antibody variable region consists of a "framework" region interrupted by three "antigen binding sites". The antigen binding sites are defined using various terms: Complementarity Determining Regions (CDRs), three in the VH (HCDR1, HCDR2, HCDR3) and three in the VL (LCDR1, LCDR2, LCDR3) are based on sequence variability (Wu and Kabat (1970) J Exp Med 132:211-50; Kabat et al Sequences of Proteins of Immunological Interest, 5th Ed. Public Health Service, National Institutes of Health, Bethesda, Md., 1991); "Hypervariable regions", "HVR", or "HV", three in the VH (H1, H2, H3) and three in the VL (L1, L2, L3) refer to the regions of antibody variable domains which are hypervariable in structure as defined by Chothia and Lesk (Chothia and Lesk (1987) Mol Biol 196:901-17). Other terms include "IMGT-CDRs" (Lefranc et al., (2003) Dev Comparat Immunol 27:55-77) and "Specificity Determining Residue Usage" (SDRU) (Almagro (2004) Mol Recognit 17:132-43). The International ImMunoGeneTics (IMGT) database (http://www_imgt_org) provides a standardized numbering and definition of antigen-binding sites. The correspondence between CDRs, HVs and IMGT delineations is described in Lefranc et al., (2003) Dev Comparat -Immunol 27:55-77.

"Chothia residues" as used herein are the antibody VL and VH residues numbered according to Al-Lazikani (Al-Lazikani et al., (1997) J Mol Biol 273:927-48).

"Framework" or "framework sequences" are the remaining sequences of a variable region other than those defined to be antigen binding sites. Because the antigen binding sites can be defined by various terms as described above, the exact amino acid sequence of a framework depends on how the antigen-binding site was defined.

"Humanized antibody" refers to an antibody in which the antigen binding sites are derived from non-human species and the variable region frameworks are derived from human immunoglobulin sequences. Humanized antibodies may include substitutions in the framework regions so that the framework may not be an exact copy of expressed human immunoglobulin or germline gene sequences.

"Human antibody" refers to an antibody having heavy and light chain variable regions in which both the framework and the antigen binding sites are derived from sequences of human origin. If the antibody contains a constant region, the constant region also is derived from sequences of human origin.

A human antibody comprises heavy or light chain variable regions that are "derived from" sequences of human origin wherein the variable regions of the antibody are obtained from a system that uses human germline immunoglobulin or rearranged immunoglobulin genes. Such systems include human immunoglobulin gene libraries displayed on phage, and transgenic non-human animals such as mice carrying human immunoglobulin loci. A "human antibody" may contain amino acid differences when compared to the human germline immunoglobulin or rearranged immunoglobulin genes due to for example naturally occurring somatic mutations or intentional introduction of substitutions in the framework or antigen binding site, or both. Typically, "human antibody" is at least about 80%, 81%, 82%, 83%, 84%, 85%, 86%, 87%, 88%, 89%, 90%, 91%, 92%, 93%, 94%, 95%, 96%, 97%, 98%, 99% or 100% identical in amino acid sequence to an amino acid sequence encoded by human germline immunoglobulin or rearranged immunoglobulin genes. In some cases, "human antibody" may contain consensus framework sequences derived from human framework sequence analyses, for example as described in Knappik et al., (2000) J Mol Biol 296:57-86, or synthetic HCDR3 incorporated into human immunoglobulin gene libraries displayed on phage, for example as described in Shi et al., (2010) J Mol Biol 397:385-96, and Intl. Pat. Publ. No. WO2009/085462.

Human antibodies derived from human immunoglobulin sequences may be generated using systems such as phage display incorporating synthetic CDRs and/or synthetic frameworks, or can be subjected to in vitro mutagenesis to improve antibody properties, resulting in antibodies that do not naturally exist within the human antibody germline repertoire in vivo.

Antibodies in which antigen binding sites are derived from a non-human species are not included in the definition of human antibody.

"Recombinant antibody" includes all antibodies that are prepared, expressed, created or isolated by recombinant means, such as antibodies isolated from an animal, for example a mouse or a rat that is transgenic or transchromosomal for human immunoglobulin genes or a hybridoma prepared therefrom (described further below), antibodies isolated from a host cell transformed to express the antibody, antibodies isolated from a recombinant, combinatorial antibody library, and antibodies prepared, expressed, created or isolated by any other means that involve splicing of human immunoglobulin gene sequences to other DNA sequences, or antibodies that are generated in vitro using Fab arm exchange such as bispecific antibodies.

"Monoclonal antibody" refers to a preparation of antibody molecules of single molecular composition. A monoclonal antibody composition displays a single binding specificity and affinity for a particular epitope, or in a case of a bispecific monoclonal antibody, a dual binding specificity to two distinct epitopes. "Monoclonal antibody" therefore refers to an antibody population with single amino acid composition in each heavy and each light chain, except for possible well known alterations such as removal of C-terminal lysine from the antibody heavy chain. Monoclonal antibodies may have heterogeneous glycosylation within the antibody population. Monoclonal antibody may be monospecific or multispecific, or monovalent, bivalent or multivalent. A bispecific antibody is included in the term monoclonal antibody.

"Epitope" means a portion of an antigen to which an antibody specifically binds. Epitopes usually consist of chemically active (such as polar, non-polar or hydrophobic) surface groupings of moieties such as amino acids or polysaccharide side chains and may have specific three-dimensional structural characteristics, as well as specific charge characteristics. An epitope may be composed of contiguous and/or noncontiguous amino acids that form a conformational spatial unit. For a noncontiguous epitope, amino acids from differing portions of the linear sequence of the antigen come in close proximity in 3-dimensional space through the folding of the protein molecule.

"Variant" refers to a polypeptide or a polynucleotide that differs from a reference polypeptide or a reference polynucleotide by one or more modifications for example, substitutions, insertions or deletions.

"In combination with" means that two or more therapeutics are administered to a subject together in a mixture, concurrently as single agents or sequentially as single agents in any order. In general, each agent will be administered at a dose and/or on a time schedule determined for that agent.

"Treat" or "treatment" refers to therapeutic treatment wherein the object is to slow down (lessen) an undesired physiological change or disease, such as the development or spread of tumor or tumor cells, or to provide a beneficial or desired clinical outcome during treatment. Beneficial or desired clinical outcomes include alleviation of symptoms, diminishment of extent of disease, stabilized (i.e., not worsening) state of disease, delay or slowing of disease progression, lack of metastasis, amelioration or palliation of the disease state, and remission (whether partial or total), whether detectable or undetectable. "Treatment" may also mean prolonging survival as compared to expected survival if a subject was not receiving treatment. Those in need of treatment include those subjects already with the undesired physiological change or disease as well as those subjects prone to have the physiological change or disease.

"Therapeutically effective amount" refers to an amount effective, at dosages and for periods of time necessary, to achieve a desired therapeutic result. A therapeutically effective amount may vary according to factors such as the disease state, age, sex, and weight of the individual, and the ability of a therapeutic or a combination of therapeutics to elicit a desired response in the individual. Exemplary indicators of an effective therapeutic or combination of therapeutics include, for example, improved well-being of the patient, reduction in a tumor burden, arrested or slowed growth of a tumor, and/or absence of metastasis of cancer cells to other locations in the body.

"Inhibits growth" (e.g. referring to tumor cells) refers to a measurable decrease or delay in the tumor cell growth or tumor tissue *in vitro* or *in vivo* when contacted with a therapeutic or a combination of therapeutics or drugs, when compared to the decrease or delay in the growth of the same tumor cells or tumor tissue in the absence of the therapeutic or the combination of therapeutic drugs. Inhibition of growth of a tumor cell or tumor tissue *in vitro* or *in vivo* may be at least about 10%, 20%, 30%, 40%, 50%, 60%, 70%, 80%, 90%, 99%, or 100%.

"Regulatory T cells" or "Tregs" or "Treg" refers to T lymphocytes that regulates the activity of other T cell(s) and/or other immune cells, usually by suppressing their activity. The Tregs may be $CD3^+CD4^+CD25^+CD127^{dim}$ T cells. It is appreciated that the Tregs may not be fully restricted to this phenotype, and may express Foxp3.

"Effector T cells" or "Teffs" or "Teff" refers to T lymphocytes that carry out a function of an immune response, such as killing tumor cells and/or activating an antitumor immune-response which can result in clearance of the tumor cells from the body. The Teffs may be $CD3^+$ with $CD4^+$ or $CD8^+$. The Teffs may secrete, contain or express markers such as IFN-y, granzyme B and ICOS. It is appreciated that the Teffs may not be fully restricted to these phenotypes.

"Function of Tregs" or "Treg function" refers to a suppressive function of the Tregs that relates to regulation of host immune responses and/or prevention of autoimmunity. Function of Tregs may be suppression of an anti-tumor response elicited by $CD8^+$ T cells, natural killer (NK) cells, MØ cells, B cells, or dendritic cells (DCs), or suppression of proliferation of effector T cells.

"Inhibit function of Tregs" or "inhibit Treg function" refers to decreasing the level of function of Tregs *in vitro* or *in vivo* in an animal or human subject, which may be determined by conventional techniques known in the art. The level of the function of Tregs may be decreased by, for example, at least about 1%, 5%, 10%, 20%, 30%, 40%, 50%, 60%, 70%, 80%, 90%, 95%, 99% or 100%. "Inhibit function of Tregs" include reducing the number of Tregs, for example by killing the Tregs via antibody effector functions such as antibody-dependent cellular cytotoxicity (ADCC).

"Myeloid-derived suppressor cells" or "MDSCs" or "MDSC" refers to a specialized population of cells that are of the hematopoietic lineage and express the macrophage/monocyte marker CD1 1b and the granulocyte marker Gr-1/Ly-6G. Phenotype of the MDSCs may be for example $CD11b^+HLA-DR^-CD14^-CD33^+CD15^+$. The MDSCs express low or undetectable expression of the mature antigen presenting cell markers MHC Class II and F480. The MDSCs are immature cells of the myeloid lineage and may further differentiate into several cell types, including macrophages, neutrophils, dendritic cells, monocytes or granulocytes. The MDSCs may be found naturally in normal adult bone marrow of human and animals or in sites of normal hematopoiesis, such as the spleen.

"Inhibit function of MDSCs" or "inhibit MDSC function" refers to decreasing the level of function of MDSCs *in vitro* or *in vivo* in an animal or human subject, which may be determined by conventional techniques known in the art. The level of the function of MDSC may be decreased by, for example, at least about 1%, 5%, 10%, 20%, 30%, 40%, 50%, 60%, 70%, 80%, 90%, 95%, 99% or 100%. "Inhibit function of MDSC" include reducing the number of MDSC, for example by killing the MDSC via antibody effector functions, such as ADCC. The MDSCs may suppress T cell responses such as proliferation, clonal expansion or cytokine production by various mechanisms such as production of reactive oxygen species, peroxynitrites, increased arginase metabolism due to high levels of arginase, and increased nitrous oxide synthase. The MDSCs may response to IFN-γ and several cytokines such as IL-4 and IL-13. IFN-γ may activate MDSCs which induces the activity of nitric-oxide synthase 2 (NOS2). Alternately, Th2 cytokines such as interleukin-4 (IL-4) and IL-13 may activate MDSCs which may lead to the induction of arginase-1 (ARG1) activity. The metabolism of L-arginine by either NOS2 or ARG1 may lead to the inhibition of T-cell proliferation, and the activity of both enzymes together can result in T-cell apoptosis through the production of reactive nitrogen-oxide species.

"Treg related disease" refers to a disease or disorder linked to T regulatory cells (Tregs). Treg related disease may be caused by Treg function, for example, suppression of an anti-tumor response or suppression of effector T cell proliferation. The Treg mediated disease may be cancer. "Treg related disease" and "Treg mediated disease" are used exchangeably herein.

"Enhance response of effector T cells" or "enhance T cell responses" refers to enhancement or stimulation of effector T cells *in vitro* or *in vivo* in an animal or human subject to have a sustained or amplified biological function, or renew or reactivate exhausted or inactive T-cells. Exemplary T-cell responses are proliferation, secretion of $\gamma$-interferon from CD 8$^+$ T-cells, antigen responsiveness, or clonal expansion. The manner of measuring this enhancement is known to one of ordinary skill in the art.

"MDSC related disease" refers to a disease or disorder linked to myeloid-derived suppressor cells (MDSCs). MDSC related disease may be caused by a MDSC function, for example, suppression of an anti-tumor response or effector T cell proliferation. The MDSC mediated disease may be cancer. "MDSC related disease" and "MDSC mediated disease" are used exchangeably herein.

"Regulatory B cell" or "Breg" or "Bregs" refers to B lymphocytes that suppress immune responses. The Bregs may be CD19$^+$CD24$^+$CD38$^+$ cells, and may suppress immune responses by inhibiting T cell proliferation mediated by IL-10 secreted by the Bregs. It is appreciated that other Breg subsets exists, and are described in for example Ding et al., (2015) Human Immunology 76: 615-621.

"Breg related disease" refers to a disease or disorder linked to regulatory B cells. Breg related disease may be caused by for example Breg mediated suppression of an antitumor response or effector T cell proliferation. The Breg mediated disease may be cancer. "Breg related disease" and "Breg mediated disease" are used exchangeably herein.

"Patient" includes any human or nonhuman animal. "Nonhuman animal" includes all vertebrates, e.g., mammals and non-mammals, such as nonhuman primates, sheep, dogs, cats, horses, cows chickens, amphibians, reptiles, etc. "Patient" and "subject" are used interchangeably herein.

The invention provides an antibody that specifically binds CD38 for use in treating a patient having a solid tumor, comprising the VH of SEQ ID NO: 4 and the VL of SEQ ID NO: 5, wherein the antibody is of IgG1 isotype.

The invention relates to the medical use of said antibody in a method for treating a patient having a solid tumor with an antibody that specifically binds CD38 regardless whether the tumor cells express CD38 or not. The disclosure also relates to methods for treating a patient having regulatory T cell (Treg), myeloid-derived suppressor cell (MDSC) or regulatory B cell (Breg) mediated disease. The disclosure also relates to methods for modulating Treg, MDSC or Breg activity to treat solid tumors that are CD38 positive and/or associated with high levels of these immune suppressive cells.

The invention is based, at least in part, on the discovery that the anti-CD38 antibody DARZALEX™ (daratumumab) has an immunomodulatory activity in patients, reducing the number of immune suppressive Tregs, MDSCs and Bregs, increasing the number of CD8$^+$ T cells and the ratio of CD8$^+$ to Tregs, promoting CD8$^+$ central memory cell formation and increasing T cell clonality.

DARZALEX™ (daratumumab) and other anti-CD38 antibodies are being evaluated in the clinic for their efficacy to treat heme malignancies and plasma cell disorders, including multiple myeloma, by the ability of the antibody to eliminate CD38-positive cells by antibody effector functions, such as ADCC, CDC, ACDP and apoptosis, but their immunomodulatory activity in promoting adaptive immune responses has not been recognized. Other immune modulatory antibodies (anti-PD1, anti-CTLA4) function through targeting components of the immune system that suppress anti-tumor responses. For example, anti-PD1 antibodies have been demonstrated to increase T-cell proliferation, stimulate antigen-specific memory responses, and partially relieve Treg-mediated suppression of effector T cells *in vitro* (for example, see U.S. Pat. No. 8,779,105). Two anti-PD-1 antibodies are currently approved for treatment of melanoma, OPDIVO® (nivolumab) and KEYTRUDA® (pembrolizumab) and these antibodies are in clinical development for various solid tumors, such as lung non-small cell carcinoma, prostate, head and neck, gastrointestinal, stomach, prostate, fallopian tube, ovarian, pancreatic, breast and brain cancer, renal, bladder, urethral, oesophageal and colorectal cancer. Anti-CTLA-4 antibody YERVOY® (ipilimumab) has been approved for treatment of melanoma. YERVOY® (ipilimumab) and another anti-CTLA-4 antibody, tremelimumab are also being developed for prostate, non-small cell lung cancer, ovarian, gastrointestinal, stomach, colorectal, renal, oesophageal, and genitourinary cancer.

Without wishing to be bound by any particular theory, based on the immunomodulatory effects observed with DARZALEX™ (daratumumab) described herein, DARZALEX™ (daratumumab) and other anti-CD38 antibodies may be efficacious in treatment of solid tumors. Due to the general activation of immune response observed in patients treated with DARZALEX™ (daratumumab), patients having CD38-negative solid tumors may respond to anti-CD38 antibody therapies as well.

The invention provides an antibody that specifically binds CD38 for use in treating a patient having a solid tumor, comprising the VH of SEQ ID NO: 4 and the VL of SEQ ID NO: 5, wherein the antibody is of IgG1 isotype. The invention relates to the medical use of said antibody in a method of treating a patient having a solid tumor, comprising administering to the patient in need thereof a therapeutically effective amount of an antibody that specifically binds CD38 for a time sufficient to treat the solid tumor.

The disclosure also relates to a method of treating a patient having a regulatory T cell (Treg) mediated disease, comprising administering to the patient in need thereof a therapeutically effective amount of an antibody that specifically binds CD38 for a time sufficient to treat the Treg mediated disease.

The disclosure also relates to a method of treating a patient having a myeloid-derived suppressor cell (MDSC) mediated disease, comprising administering to the patient in need thereof a therapeutically effective amount of an antibody that specifically binds CD38 for a time sufficient to treat the MDSC mediated disease.

The disclosure also relates to a method of treating a patient having a regulatory B cell (Breg) mediated disease, comprising administering to the patient in need thereof a therapeutically effective amount of an antibody that specifically binds CD38 for a time sufficient to treat the Breg mediated disease.

The disclosure also relates to a method of suppressing activity of a regulatory T cell (Treg), comprising contacting the regulatory T cell with an antibody that specifically binds CD38.

The disclosure also relates to a method of suppressing activity of a myeloid-derived suppressor cell (MDSC), comprising contacting the MDSC with an antibody that specifically binds CD38.

The disclosure also relates to a method of suppressing activity of a regulatory B cell (Breg), comprising contacting the Breg with an antibody that specifically binds CD38.

The disclosure also relates to a method of treating a patient having a solid tumor, comprising reducing the number of regulatory T cells (Treg) in the patient by administering to the patient an antibody that specifically binds CD38.

The disclosure also relates to a method of treating a patient having a solid tumor, comprising reducing the number of myeloid-derived suppressor cells (MDSC) in the patient by administering to the patient an antibody that specifically binds CD38.

The disclosure also relates to a method of treating a patient having a solid tumor, comprising reducing the number of regulatory B cells (Breg) in the patient by administering to the patient an antibody that specifically binds CD38.

The disclosure also relates to a method of enhancing an immune response in a patient, comprising administering to the patient in need thereof an antibody that specifically binds CD38 for a time sufficient to enhance the immune response.

In some instances, the patient has a viral infection.

The disclosure also relates to a method of treating a viral infection in a patient, comprising administering to the patient in need therefor an antibody that specifically binds CD38 for a time sufficient to treat the viral infection.

In some embodiments, the immune response is an effector T cell (Teff) response.

In some embodiments, the Teff response is mediated by $CD4^+$ T cells or $CD8^+$ T cells.

In some embodiments, the Teff response is mediated by $CD4^+$ T cells.

In some embodiments, the Teff response is mediated by $CD8^+$ T cells.

In some embodiments, the Teff response is an increase in the number of $CD8^+$ T cells, increased $CD8^+$ T cell proliferation, increased T cell clonal expansion, increased $CD8^+$ memory cell formation, increased antigen-dependent antibody production, or increased cytokine, chemokine or interleukin production.

Proliferation of T cells may be assessed for example by measuring the rate of DNA synthesis using tritiated thymidine or measuring production of interferon-$\gamma$ (IFN-$\gamma$) *in vitro,* or measuring absolute number or percentage of T cells in a population of cells from patient samples using known methods.

Clonal expansion may be assessed by for example sequencing TCR from a pool of T cells using know methods.

Memory cell formation may be assessed by measuring the ratio of naïve T cells ($CD45R0^-$/$CD62L^+$) to memory T cells ($CD45RO^+$/$CD62L^{high}$) using for example FACS.

Cytokine, chemokine or interleukin production, such as production of interferon-$\gamma$ (IFN-$\gamma$), tumor necrosis factor-alpha (TNF-$\alpha$), IL-1, IL-2, IL-3, IL-4, IL-6, IL-8, IL-10, IL-12, IL-13, IL-16, IL-18 and IL-23, MIP-1$\alpha$, MIP-1$\beta$, RANTES, CCL4 may be assessed using standard methods such as ELISA or ELLISPOT assay.

Antigen-specific antibody production may be assessed from samples derived from patient using standard methods, such as ELISA or radioimmunoassay (RIA).

The meaning of "increase" or "increasing" various Teff responses is readily understood. The increase may be increase of at least about 5%, at least about 10%, 25%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 110%, 120%, 130%, 140%, 150%, 200%, 250%, 300%, 350%, 400% or more in a test sample or in a subject when compared to control, e.g., for example in a patient treated with an anti-CD38 antibody when compared to the same patient before treatment, or in a patient or group of patients that are responsive to anti-CD38 antibody treatment when compared to a patient or a group of patients that are non-responsive to the same treatment. Typically, the increase is statistically significant.

Similarly, the meaning of "reduce" or "reducing" or "decreasing" or "decrease" the number of Tregs, MDSCs and/or Bregs is readily understood. The decrease may be at least about 10%, 25%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%,110%, 120%, 130%, 140%, 150%, 200%, 250%, 300%, 350%, 400% or more in a test sample or in a subject when compared to control, e.g., for example in a patient treated with an anti-CD38 antibody when compared to the same patient before treatment, or in a patient or group of patients that are responsive to anti-CD38 antibody treatment when compared to a patient or a group of patients that are non-responsive to the same treatment. Typically, the decrease is statistically significant.

In some embodiments, the antibody that specifically binds CD38 inhibits function of immune suppressor cells.

In some embodiments, the immune suppressor cells are regulatory T cells (Tregs), myeloid-derived suppressor cells (MDSC) or regulatory B cells (Bregs).

In some embodiments, the Tregs are CD3$^+$CD4$^+$CD25$^+$CD127$^{dim}$ T cells.

In some embodiments, the CD3$^+$CD4$^+$CD25$^+$CD127$^{dim}$ cells express Foxp3.

In some embodiments, the CD3$^+$CD4$^+$CD25$^+$CD127$^{dim}$ T cells express CD38.

Treg function, such as their ability to suppress Teff cells, may be assessed using known methods, such as assessing the ability of Tregs to suppress Teff proliferation in mixed lymphocyte reaction (MLR).

Treg function may be inhibited by for example reducing the relative number of Tregs when compared to Teffs (e.g. increasing the ratio of CD8$^+$/Treg cells) by direct killing of Tregs or a sub-population of Tregs, such as CD38$^+$ Tregs.

In some embodiments, the Treg function is inhibited by killing the Treg cells.

In some embodiments, the Treg killing is mediated by antibody-induced antibody-dependent cell cytotoxicity (ADCC), antibody-dependent cell phagocytosis (ADCP), complement-dependent cytotoxicity (CDC) or apoptosis induced by an antibody specifically binding CD38.

In some embodiment, the Treg killing is mediated by ADCC.

In some embodiments, the CD38$^+$ Tregs are killed.

In some embodiments, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58% or 60% of Tregs are killed.

As CD38 is expressed only in a portion of Tregs and MDSCs, it is expected that treatment of patients with solid tumors will not result in systemic depletion of Tregs and MDSCs, therefore likely providing an improved safety profile.

In some embodiments, the MDSCs are CD11b$^+$HLA-DR-CD14$^-$CD33$^+$CD15$^+$ cells.

In some embodiments, the CD11b$^+$HLA-DR$^-$CD14$^-$CD33$^+$CD15$^+$ MDSCs express CD38.

MDSC function may be inhibited for example by reducing the number of MDSCs by direct killing of the cells.

In some embodiments, the MDSC function is inhibited by killing the CD38$^+$ MDSC.

In some embodiments, the MDSC killing is mediated by antibody-induced antibody-dependent cell cytotoxicity (ADCC), antibody-dependent cell phagocytosis (ADCP), complement-dependent cytotoxicity (CDC) or apoptosis induced by the antibody that specifically binds CD38.

In some embodiments, the MDSC killing is mediated by ADCC.

In some embodiments, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58% or 60% of the MDSCs are killed.

In some embodiments, the Bregs are CD19$^+$CD24$^+$CD38$^+$ cells.

The Breg function may be inhibited for example by reducing the number of Bregs by direct killing of the Bregs.

In some embodiments, the Breg function is inhibited by killing the CD38$^+$Bregs.

In some embodiments, the Breg killing is mediated by antibody-induced antibody-dependent cell cytotoxicity (ADCC), antibody-dependent cell phagocytosis (ADCP), complement-dependent cytotoxicity (CDC) or apoptosis induced by the antibody that specifically binds CD38.

In some embodiments, the Breg killing is mediated by ADCC.

In some embodiments, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58% or 60% of the Bregs are killed.

Tregs play a critical role in the maintenance of peripheral self-tolerance. Naturally occurring CD4$^+$CD25$^{hi}$ Tregs are produced in the thymus and express Foxp3, a transcriptional factor required for establishment and maintenance of Treg lineage identity and suppressor function. Tregs can accumulate at a disease site (e.g. within tumor), where they suppress the effector function of tumor antigen specific T cells, resulting in insufficient anti-tumor responses. Increased densities of tumor-infiltrating Foxp3$^+$ Tregs have been associated with poor prognosis in various solid tumors, including pancreatic, ovarian, and hepatocellular carcinoma. Depletion of Tregs results in enhanced antitumor immunity and tumor rejection in murine models but may also result in the development of autoimmune diseases.

Myeloid-derived suppressor cells (MDSC) are a heterogeneous population of early myeloid progenitors, immature granulocytes, macrophages, and dendritic cells at different stages of differentiation. They accumulate in large numbers in cancer patients and they have potent immunosuppressive functions, suppressing both the cytotoxic activities of natural killer cells (NK) and natural killer T cells (NKT), and the adaptive immune response mediated by CD8[+] T cells. While the mechanism of NK cell inhibition is currently not well-understood, multiple pathways are responsible for MDSC-mediated T cell suppression including production of arginase 1/ARG1 and upregulation of nitric oxide synthase 2 (NOS2). ARG1 and NOS2 metabolize L-arginine and either together or separately blocks the translation of the T cell CD3zeta chain, inhibits T cell proliferation, and promotes T cell apoptosis. Additionally, MDSCs secrete immunosuppressive cytokines and induce regulatory T cell development.

MDSC are induced by pro-inflammatory cytokines and are found in increased numbers in infectious and inflammatory pathological conditions. They accumulate in the blood, bone marrow, and secondary lymphoid organs of tumor-bearing mice and their presence in the tumor microenvironment has been suggested to have a causative role in promoting tumor-associated immune suppression.

MDSC have been described in patients with colon carcinoma, melanoma, hepatocellular carcinoma, head and neck squamous cell carcinoma, non-small cell lung carcinoma, renal cell carcinoma, pancreatic adenocarcinoma and breast carcinoma (Mandruzzato et al., (2009) J Immunol 182: 6562-6568; Liu et al., (2009) J Cancer Res Clin Oncol 136: 35-45; Ko et al., (2009) Clin Cancer Res 15: 2148-2157; Morse et al., (2009) Expert Opin Biol Ther 9: 331-339; Diaz-Montero et al., (2009) Cancer Immunol Immunother 58: 49-59; Corzo et al., (2009) J Immunol 182: 5693-5701). In cancer patients, Diaz et al (Diaz-Montero et al., (2009) Cancer Immunol Immunother 58: 49-59) propose that accumulation of MDSC correlates with more advanced disease and poor prognosis.

Tumor-infiltrating Bregs have been identified in solid tumors, and the Bregs may promote tumor growth and metastasis by various mechanisms such as suppressing the antitumor activity of CD8[+] T cells and NK cells, as described in for example Ding et al., (2015) Human Immunology 76:615-62.

"Antibody-dependent cellular cytotoxicity", "antibody-dependent cell-mediated cytotoxicity" or "ADCC" is a mechanism for inducing cell death that depends upon the interaction of antibody-coated target cells with effector cells possessing lytic activity, such as natural killer cells, monocytes, macrophages and neutrophils via Fc gamma receptors (FcγR) expressed on effector cells. For example, NK cells express FcγRIIIa, whereas monocytes express FcγRI, FcγRII and FcvRIIIa. Death of the antibody-coated target cell, such as CD38-expressing cells, occurs as a result of effector cell activity through the secretion of membrane pore-forming proteins and proteases. To assess ADCC activity of an antibody that specifically binds CD38, the antibody may be added to CD38-expressing cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Exemplary effector cells for such assays include peripheral blood mononuclear cells (PBMC) and NK cells. Exemplary target cells include Tregs or MDSCs expressing CD38. In an exemplary assay, target cells are labeled with 20 $\mu$Ci of $^{51}$Cr for 2 hours and washed extensively. Cell concentration of the target cells may be adjusted to $1 \times 10^6$ cells/ml, and anti-CD38 antibodies at various concentrations are added. Assays are started by adding target cells at an effector:target cell ratio of 40:1. After incubation for 3 hr at 37°C assays are stopped by centrifugation and $^{51}$Cr release from lysed cells are measured in a scintillation counter. Percentage of cellular cytotoxicity may be calculated as % maximal lysis which may be induced by adding 3% perchloric acid to target cells.

"Antibody-dependent cellular phagocytosis" ("ADCP") refers to a mechanism of elimination of antibody-coated target cells by internalization by phagocytic cells, such as macrophages or dendritic cells. ADCP may be evaluated by using Tregs or MDSCs expressing CD38 as target cells engineered to express GFP or other labeled molecule. Effector:target cell ratio may be for example 4: 1. Effector cells may be incubated with target cells for 4 hours with or without anti-CD38 antibody. After incubation, cells may be detached using accutase. Macrophages may be identified with anti-CD11b and anti-CD 14 antibodies coupled to a fluorescent label, and percent phagocytosis may be determined based on % GFP fluorescent in the CD11[+]CD14[+] macrophages using standard methods.

"Complement-dependent cytotoxicity", or "CDC", refers to a mechanism for inducing cell death in which an Fc effector domain of a target-bound antibody binds and activates complement component C1q which in turn activates the complement cascade leading to target cell death. Activation of complement may also result in deposition of complement components on the target cell surface that facilitate ADCC by binding complement receptors (e.g., CR3) on leukocytes.

The ability of monoclonal antibodies to induce ADCC may be enhanced by engineering their oligosaccharide component. Human IgG1 or IgG3 are N-glycosylated at Asn297 with the majority of the glycans in the well-known biantennary G0, G0F, G1, G1F, G2 or G2F forms. Antibodies produced by non-engineered CHO cells typically have a glycan fucose content of about at least 85%. The removal of the core fucose from the biantennary complex-type oligosaccharides attached to the Fc regions enhances the ADCC of antibodies via improved FcγRIIIa binding without altering antigen binding or CDC activity. Such mAbs may be achieved using different methods reported to lead to the successful expression of relatively high defucosylated antibodies bearing the biantennary complex-type of Fc oligosaccharides such as control of culture osmolality (Konno et al., (2012) Cytotechnology 64:249-65), application of a variant CHO line Lec13 as the host cell line (Shields et al., (2002) J Biol Chem 277:26733-26740), application of a variant CHO line EB66 as the host cell line (Olivier et al., (2010) MAbs 2(4), Epub ahead of print; PMID:20562582), application of a rat hybridoma cell line YB2/0 as the host cell line (Shinkawa et al., (2003) J Biol Chem 278:3466-3473), introduction of small interfering RNA specifically against the α 1,6-fucosyltrasferase (*FUT8*) gene (Mori et al., (2004) Biotechnol Bioeng 88:901-908), or coexpression of β-1,4-*N*-acetylglucosarninyltransferase III and Golgi α-mannosidase II or a potent alpha-mannosidase I inhibitor, kifunensine (Ferrara et al., (2006) J Biol Chem 281:5032-5036; Ferrara et al., (2006) Biotechnol Bioeng 93:851-861; Xhou et al., (2008) Biotechnol Bioeng 99:652-65). ADCC elicited by anti-CD38 antibodies used in the invention, and in some embodiments of each and every one of the numbered embodiments listed below, may also be enhanced by certain substitutions in the antibody Fc. Exemplary substitutions are for example substitutions at amino acid positions 256, 290, 298, 312, 356, 330, 333, 334, 360, 378 or 430 (residue numbering according to the EU index) as described in U.S. Pat. No. 6,737,056.

In some embodiments, the antibody that specifically binds CD38 comprises a substitution in the antibody Fc.

In some embodiments, the antibody that specifically binds CD38 comprises a substitution in the antibody Fc at amino acid positions 256, 290, 298, 312, 356, 330, 333, 334, 360, 378 or 430 (residue numbering according to the EU index).

In some embodiments, the antibody that specifically binds CD38 has a biantennary glycan structure with fucose content of about between 0% to about 15%, for example 15%, 14%, 13%, 12%, 11% 10%, 9%, 8%, 7%, 6%, 5%, 4%, 3%, 2%, 1% or 0%.

In some embodiments, the antibody that specifically binds CD38 has a biantennary glycan structure with fucose content of about 50%, 40%, 45%, 40%, 35%, 30%, 25%, 20%, 15%, 14%, 13%, 12%, 11% 10%, 9%, 8%, 7%, 6%, 5%, 4%, 3%, 2%, 1% or 0%

Substitutions in the Fc and reduced fucose content may enhance the ADCC activity of the antibody that specifically binds CD38.

"Fucose content" means the amount of the fucose monosaccharide within the sugar chain at Asn297. The relative amount of fucose is the percentage of fucosecontaining structures related to all glycostructures. These may be characterized and quantified by multiple methods, for example: 1) using MALDI-TOF ofN-glycosidase F treated sample (e.g. complex, hybrid and oligo- and high-mannose structures) as described in Intl. Pat. Publ. No. WO2008/077546; 2) by enzymatic release of the Asn297 glycans with subsequent derivatization and detection/ quantitation by HPLC (UPLC) with fluorescence detection and/or HPLC-MS (UPLC-MS); 3) intact protein analysis of the native or reduced mAb, with or without treatment of the Asn297 glycans with Endo S or other enzyme that cleaves between the first and the second GlcNAc monosaccharides, leaving the fucose attached to the first GlcNAc; 4) digestion of the mAb to constituent peptides by enzymatic digestion (e.g., trypsin or endopeptidase Lys-C), and subsequent separation, detection and quantitation by HPLC-MS (UPLC-MS) or 5) separation of the mAb oligosaccharides from the mAb protein by specific enzymatic deglycosylation with PNGase F at Asn 297. The oligosaccharides released may be labeled with a fluorophore, separated and identified by various complementary techniques which allow: fine characterization of the glycan structures by matrix-assisted laser desorption ionization (MALDI) mass spectrometry by comparison of the experimental masses with the theoretical masses, determination of the degree of sialylation by ion exchange HPLC (GlycoSep C), separation and quantification of the oligosacharride forms according to hydrophilicity criteria by normal-phase HPLC (GlycoSep N), and separation and quantification of the oligosaccharides by high performance capillary electrophoresis-laser induced fluorescence (HPCE-LIF).

"Low fucose" or "low fucose content" as used herein refers to antibodies with fucose content of about 0% - 15%.

"Normal fucose" or 'normal fucose content" as used herein refers to antibodies with fucose content of about over 50%, typically about over 60%, 70%, 80% or over 85%.

In some embodiments, the antibody that specifically binds CD38 may induce killing of Tregs, MDSCs and/or Bregs by apoptosis. Methods for evaluating apoptosis are well known, and include for example annexin IV staining using standard methods. The anti-CD38 antibodies used in the invention may induce apoptosis in about 20%, 25%, 30%, 35%, 40%, 45%, 50%, 55%, 60%, 65%, 70%, 75%, 80%, 85%, 90%, 95% or 100% of cells.

In some embodiments, the Teffs or the immune suppressor cells reside in bone marrow or in peripheral blood.

In some embodiments, the Teffs or the immune suppressor cells reside in bone marrow.

In some embodiments, the Teffs or the immune suppressor cells reside in peripheral blood.

In some embodiments, the antibody that specifically binds CD38 increases the ratio of CD8[+] T cells to Tregs.

In some embodiments, the antibody that specifically binds CD38 increases the ratio of CD8[+] central memory cells to CD8[+] naïve cells. CD8[+]central memory cells can be identified as CD45RO[+]/CD62L[+high] cells. CD8[+] naive cells can be identified as CD45RO/CD62L[+] cells.

A non-agonistic antibody that specifically binds CD38 refers to an antibody which upon binding to CD38 does not induce significant proliferation of a sample of peripheral blood mononuclear cells *in vitro* when compared to the proliferation induced by an isotype control antibody or medium alone.

In some embodiments, the non-agonistic antibody that specifically binds CD38 induces proliferation of peripheral blood mononuclear cells (PBMCs) in a statistically insignificant manner. PBMC proliferation may be assessed by isolating PBMCs from healthy donors and culturing the cells at $1 \times 10^5$ cells/well in flat bottom 96-well plates in the presence or absence of a test antibody in 200 µl RPMI After four day incubation at 37° C., 30 µl $^3$H-thymidine (16.7 µCi/ml) may be added, and culture may be continued overnight. $^3$H-thymidine incorporation may be assessed using a Packard Cobra gamma counter (Packard Instruments, Meriden, DT, USA), according to the manufacturer's instructions. Data may be calculated as the mean cpm (±SEM) of PBMCs obtained from several donors. Statistical significance or insignificance between samples cultured in the presence or absence of the test antibody is calculated using standard methods.

An exemplary anti-CD38 antibody that may be used in the invention is DARZALEX™ (daratumumab). DARZALEX™ (daratumumab) comprises a heavy chain variable region (VH) and a light chain variable region (VL) amino acid sequences shown in SEQ ID NO: 4 and 5, respectively, a heavy chain complementarity determining region 1 (HCDR1), a HCDR2 and a HCDR3 of SEQ ID NOs: 6, 7 and 8, respectively, and a light chain complementarity determining region 1 (LCDR1), a LCDR2 and a LCDR3 of SEQ ID NOs: 9, 10 and 11, respectively, and is of IgG1/κ subtype and described in U.S. Pat. No. 7,829,693. DARZALEX™ (daratumumab) heavy chain amino acid sequence is shown in SEQ ID NO: 12 and light chain amino acid sequence shown in SEQ ID NO: 13.

SEQ ID NO: 1

MANCEFSPVSGDKPCCRLSRRAQLCLGVSILVLILVVVLAVVVPRWRQQWSGPGT
TKRFPETVLARCVKYTEIHPEMRHVDCQSVWDAFKGAFISKHPCNITEEDYQPLM
KLGTQTVPCNKILLWSRIKDLAHQFTQVQRDMFTLEDTLLGYLADDLTWCGEFN
TSKINYQSCPDWRKDCSNNPVSVFWKTVSRRFAEAACDVVHVMLNGSRSKIFDK
NSTFGSVEVHNLQPEKVQTLEAWVIHGGREDSRDLCQDPTIKELESIISKRNIQFSC
KNIYRPDKFLQCVKNPEDSSCTSEI

SEQ ID NO: 2
SKRNIQFSCKNIYR

SEQ ID NO: 3
EKVQTLEAWVIHGG

SEQ ID NO: 4

EVQLLESGGGLVQPGGSLRLSCAVSGFTFNSFAMSWVRQAPGKGLEWVSA
ISGSGGGTYYADSVKGRFTISRDNSKNTLYLQMNSLRAEDTAVYFCAKDK
ILWFGEPVFDYWGQGTLVTVSS

SEQ ID NO: 5

EIVLTQSPATLSLSPGERATLSCRASQSVSSYLAWYQQKPGQAPRLLIYD
ASNRATGIPARFSGSGSGTDFTLTISSLEPEDFAVYYCQQRSNWPPTFGQ
GTKVEIK

SEQ ID NO: 6
SFAMS

SEQ ID NO: 7
AISGSGGGTYYADSVKG

SEQ ID NO: 8
DKILWFGEPVFDY

SEQ ID NO: 9
RASQSVSSYLA

SEQ ID NO: 10
DASNRAT

SEQ ID NO: 11
QQRSNWPPTF

SEQ ID NO: 12

EVQLLESGGGLVQPGGSLRLSCAVSGFTFNSFAMSWVRQAPGKGLEWVSAISGSG
GGTYYADSVKGRFTISRDNSKNTLYLQMNSLRAEDTAVYFCAKDKILWFGEPVF
DYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKRV
EPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVS
NKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHN
HYTQKSLSLSPGK

SEQ ID NO: 13

EIVLTQSPATLSLSPGERATLSCRASQSVSSYLAWYQQKPGQAPRLLIYDASNRAT
GIPARFSGSGSGTDFTLTISSLEPEDFAVYYCQQRSNWPPTFGQGTKVEIKRTVAAP
SVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

Antibodies may be evaluated for their competition with a reference antibody such as DARZALEX™ (daratumumab) having the VH of SEQ ID NO: 4 and the VL of SEQ ID NO: 5 for binding to CD38 using well known *in vitro* methods. In an exemplary method, CHO cells recombinantly expressing CD38 may be incubated with an unlabeled reference antibody for 15 min at 4°C, followed by incubation with an excess of fluorescently labeled test antibody for 45 min at 4°C. After washing in PBS/BSA, fluorescence may be measured by flow cytometry using standard methods. In another exemplary method, extracellular portion of human CD38 may be coated on the surface of an ELISA plate. Excess of the unlabeled reference antibody may be added for about 15 minutes and subsequently biotinylated test antibodies may be added. After washes in PBS/Tween, binding of the test biotinylated antibody may be detected using horseradish peroxidase (HRP)-conjugated streptavidin and the signal detected using standard methods. It is readily apparent that in the competition assays, the reference antibody may be labelled and the test antibody unlabeled. The test antibody competes with the reference antibody when the reference antibody inhibits binding of the test antibody, or the test antibody inhibits binding of the reference antibody to CD38 by at least 80%, for example 81%, 82%, 83%, 84%, 85%, 86%, 87%, 88%, 89%, 90%, 91%, 92%, 93%, 94%, 95%, 96%, 97%, 98%, 99% or 100%. The epitope of the test antibody may further be defined for example by peptide mapping or hydrogen/deuterium protection assays using known methods, or by crystal structure determination.

Antibodies binding to the region SKRNIQFSCKNIYR (SEQ ID NO: 2) and the region EKVQTLEAWVIHGG (SEQ ID NO: 3) of human CD38 (SEQ ID NO: 1) may be generated for example by immunizing mice with peptides having the amino acid sequences shown in SEQ ID NOs: 2 and 3 using standard methods and those described herein, and characterizing the obtained antibodies for binding to the peptides using for example ELISA or mutagenesis studies.

An anti-CD38 antibody disclosed herein binds to the region SKRNIQFSCKNIYR (SEQ ID NO: 2) and the region EKVQTLEAWVIHGG (SEQ ID NO: 3) of human CD38 (SEQ ID NO: 1). The epitope of the antibody used in the invention includes some or all of the residues having the sequences shown in SEQ ID NO: 2 or SEQ ID NO: 3. In some embodiments, the antibody epitope comprises at least one amino acid in the region SKRNIQFSCKNIYR (SEQ ID NO: 2) and at least one amino acid in the region EKVQTLEAWVIHGG (SEQ ID NO: 3) of human CD38 (SEQ ID NO: 1). In some embodiments, the antibody epitope comprises at least two amino acids in the region SKRNIQFSCKNIYR (SEQ ID NO: 2) and at least two amino acids in the region EKVQTLEAWVIHGG (SEQ ID NO: 3) of human CD38 (SEQ ID NO: 1). In some embodiments, the antibody epitope comprises at least three amino acids in the region SKRNIQFSCKNIYR (SEQ ID NO: 2) and at least three amino acids in the region EKVQTLEAWVIHGG (SEQ ID NO: 3) of human CD38 (SEQ ID NO: 1).

The antibody that specifically binds CD38 comprises the HCDR1, the HCDR2 and the HCDR3 amino acid sequences of SEQ ID NOs: 6, 7 and 8, respectively.

The antibody that specifically binds CD38 comprises the LCDR1, the LCDR2 and the LCDR3 amino acid sequences of SEQ ID NOs: 9, 10 and 11, respectively.

The antibody that specifically binds CD38 comprises the HCDR1, the HCDR2, the HCDR3, the LCDR1, the LCDR2 and the LCDR3 amino acid sequences of SEQ ID NOs: 6, 7, 8, 9, 10 and 11, respectively.

The antibody that specifically binds CD38 comprises the VH of SEQ ID NO: 4 and the VL of SEQ ID NO: 5.

In some embodiments, the antibody that specifically binds CD38 comprises the heavy chain of SEQ ID NO: 12 and the light chain of SEQ ID NO: 13.

Other exemplary anti-CD38 antibodies that may be used in accordance with the disclosure are:

mAb003 comprising the VH and the VL sequences of SEQ ID NOs: 14 and 15, respectively and described in U.S. Pat. No. 7,829,693. The VH and the VL of mAb003 may be expressed as IgG1/κ.

SEQ ID NO: 14

QVQLVQSGAEVKKPGSSVKVSCKASGGTFSSYAFSWVRQAPGQGLEWMGRVIPF

LGIANSAQKFQGRVTITADKSTSTAY

MDLSSLRSEDTAVYYCARDDIAALGPFDYWGQGTLVTVSSAS

SEQ ID NO: 15

DIQMTQSPSSLSASVGDRVTITCRASQGISSWLAWYQQKPEKAPKSLIYAASSLQS

GVPSRFSGSGSGTDFTLTISSLQP

EDFATYYCQQYNSYPRTFGQGTKVEIK;

mAb024 comprising the VH and the VL sequences of SEQ ID NOs: 16 and 17, respectively, described in U.S. Pat. No. 7,829,693. The VH and the VL of mAb024 may be expressed as IgG1/κ.

SEQ ID NO: 16

EVQLVQSGAEVKKPGESLKISCKGSGYSFSNYWIGWVRQMPGKGLEWMGIIYPH

DSDARYSPSFQGQVTFSADKSISTAY

LQWSSLKASDTAMYYCARHVGWGSRYWYFDLWGRGTLVTVSS

SEQ ID NO: 17

EIVLTQSPATLSLSPGERATLSCRASQSVSSYLAWYQQKPGQAPRLLIYDASNRAT

GIPARFSGSGSGTDFTLTISSLEP

EDFAVYYCQQRSNWPPTFGQGTKVEIK;

MOR-202 (MOR-03087) comprising the VH and the VL sequences of SEQ ID NOs: 18 and 19, respectively, described in US. Pat. No. 8,088,896. The VH and the VL of MOR-202 may be expressed as IgG1/κ.

SEQ ID NO: 18

QVQLVESGGGLVQPGGSLRLSCAASGFTFSSYYMNWVRQAPGKGLEWVSGISGD
PSNTYYADSVKGRFTISRDNSKNTLY
LQMNSLRAEDTAVYYCARDLPLVYTGFAYWGQGTLVTVSS

SEQ ID NO: 19

DIELTQPPSVSVAPGQTARISCSGDNLRHYYVYWYQQKPGQAPVLVIYGDSKRPS
GIPERFSGSNSGNTATLTISGTQAEDEADYYCQTYTGGASLVFGGGTKLTVLGQ;

Isatuximab; comprising the VH and the VL sequences of SEQ ID NOs: 20 and 21, respectively, described in U.S. Pat. No. 8,153,765. The VH and the VL of Isatuximab may be expressed as IgG1/κ.

SEQ ID NO 20:

QVQLVQSGAEVAKPGTSVKLSCKASGYTFTDYWMQWVKQRPGQGLEWIGT
IYPGDGDTGYAQKFQGKATLTADKSSKTVYMHLSSLASEDSAVYYCARGD
YYGSNSLDYWGQGTSVTVSS

SEQ ID NO: 21:

DIVMTQSHLSMSTSLGDPVSITCKASQDVSTVVAWYQQKPGQSPRRLIYS
ASYRYIGVPDRFTGSGAGTDFTFTISSVQAEDLAVYYCQQHYSPPYTFGG
GTKLEIK

Other exemplary anti-CD38 antibodies that may be used in the methods of the disclosure include those described in Int. Pat. Publ. No. WO05/103083, Intl. Pat. Publ. No. WO06/125640, Intl. Pat. Publ. No. WO07/042309, Intl. Pat. Publ. No. WO08/047242 or Intl. Pat. Publ. No. WO 14/178820.

The invention provides an antibody that specifically binds CD38 for use in treating a patient having a solid tumor, comprising the VH of SEQ ID NO: 4 and the VL of SEQ ID NO: 5, wherein the antibody is of IgG1 isotype.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 14 and the VL of SEQ ID NO: 15 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 16 and the VL of SEQ ID NO: 17 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 18 and the VL of SEQ ID NO: 19 or a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 20 and the VL of SEQ ID NO: 21 or a time sufficient to treat the solid tumor.

In some embodiments, the solid tumor is a melanoma.

In some embodiments, the solid tumor is a lung cancer.

In some embodiments, the solid tumor is a squamous non-small cell lung cancer (NSCLC).

In some embodiments, the solid tumor is a non-squamous NSCLC.

In some embodiments, the solid tumor is a lung adenocarcinoma.

In some embodiments, the solid tumor is a renal cell carcinoma (RCC) (e.g., a kidney clear cell carcinoma or a kidney papillary cell carcinoma), or a metastatic lesion thereof.

In some embodiments, the solid tumor is a mesothelioma.

In some embodiments, the solid tumor is a nasopharyngeal carcinoma (NPC).

In some embodiments, the solid tumor is a colorectal cancer.

In some embodiments, the solid tumor is a prostate cancer or castration-resistant prostate cancer.

In some embodiments, the solid tumor is a stomach cancer.

In some embodiments, the solid tumor is an ovarian cancer.

In some embodiments, the solid tumor is a gastric cancer.

In some embodiments, the solid tumor is a liver cancer.

In some embodiments, the solid tumor is pancreatic cancer.

In some embodiments, the solid tumor is a thyroid cancer.

In some embodiments, the solid tumor is a squamous cell carcinoma of the head and neck.

In some embodiments, the solid tumor is a carcinomas of the esophagus or gastrointestinal tract.

In some embodiments, the solid tumor is a breast cancer.

In some embodiments, the solid tumor is a fallopian tube cancer.

In some embodiments, the solid tumor is a brain cancer.

In some embodiments, the solid tumor is an urethral cancer.

In some embodiments, the solid tumor is a genitourinary cancer.

In some embodiments, the solid tumor is an endometriosis.

In some embodiments, the solid tumor is a cervical cancer.

In some embodiments, the solid tumor is a metastatic lesion of the cancer.

In some embodiments, the solid tumor lacks detectable CD38 expression.

The solid tumor lacks detectable CD38 expression when CD38 expression in the solid tumor tissue or on cells isolated from the solid tumor is statistically insignificant when compared to a control, e.g. expression detected with anti-CD38 antibody vs expression detected with an isotype control antibody using well known methods.

Anti-CD38 antibodies used in the methods of the disclosure may also be selected *de novo* from, e.g., a phage display library, where the phage is engineered to express human immunoglobulins or portions thereof such as Fabs, single chain antibodies (scFv), or unpaired or paired antibody variable regions (Knappik et al., (2000) J Mol Biol 296:57-86; Krebs et al., (2001) J Immunol Meth 254:67-84; Vaughan et al., (1996) Nature Biotechnology 14:309-314; Sheets et al., (1998) PITAS (USA) 95:6157-6162; Hoogenboom and Winter, (1991) J Mol Biol 227:381; Marks et al., (1991) J Mol Biol 222:581). CD38 binding variable domains may be isolated from e.g., phage display libraries expressing antibody heavy and light chain variable regions as fusion proteins with bacteriophage pIX coat protein as described in Shi et al., (2010) J Mol Biol 397:385-96, and Intl. Pat. Publ. No. WO09/085462. The antibody libraries may be screened for binding to human CD38 extracellular domain, the obtained positive clones further characterized, Fabs isolated from the clone lysates, and subsequentely cloned as full length antibodies. Such phage display methods for isolating human antibodies are established in the art. See for example: U.S. Pat. No. 5,223,409, U.S. Pat. No. 5,403,484, U.S. Pat. No. 5,571,698, U.S. Pat. No. 5,427,908, U.S. Pat. No. 5,580,717, U.S. Pat. No. 5,969,108, U.S. Pat. No. 6,172,197, U.S. Pat. No. 5,885,793, U.S. Pat. No. 6,521,404, U.S. Pat. No. 6,544,731, U.S. Pat. No. 6,555,313, U.S. Pat. No. 6,582,915, and U.S. Pat. No. 6,593,081.

The anti-CD38 antibody is of IgG1.

The Fc portion of the antibody may mediate antibody effector functions such as antibody-dependent cell-mediated cytotoxicity (ADCC), antibody-dependent cellular phagocytosis (ADCP) or complement dependent cytotoxicity (CDC). Such function may be mediated by binding of an Fc effector domain(s) to an Fc receptor on an immune cell with phagocytic or lytic activity or by binding of an Fc effector domain(s) to components of the complement system. Typically, the effect(s) mediated by the Fc-binding cells or complement components result in inhibition and/or depletion of target cells, for example CD38-expressing cells. Human IgG isotypes IgG1, IgG2, IgG3 and IgG4 exhibit differential capacity for effector functions. ADCC may be mediated by IgG1 and IgG3, ADCP may be mediated by IgG1, IgG2, IgG3 and IgG4, and CDC may be mediated by IgG1 and IgG3.

Antibodies that are substantially identical to the antibody comprising the VH of SEQ ID NO: 4 and the VL of SEQ ID NO: 5 may be used in the invention. The term "substantially identical" as used herein means that the two antibody VH or VL amino acid sequences being compared are identical or have "insubstantial differences". Insubstantial differences are substitutions of 1, 2, 3, 4, 5, 6, 7, 8, 9, 10, 11, 12, 13, 14, or 15 amino acids in an antibody heavy chain or light chain that do not adversely affect antibody properties. Percent identity may be determined for example by pairwise alignment using the default settings of the AlignX module of Vector NTI v.9.0.0 (Invitrogen, Carlsbad, CA). The protein sequences herein may be used as a query sequence to perform a search against public or patent databases to, for example, identify related sequences. Exemplary programs used to perform such searches are the XBLAST or BLASTP programs (http_/www_ncbi_nlm/nih_gov), or the GenomeQuest™ (GenomeQuest, Westborough, MA) suite using the default settings. Exemplary substitutions that may be made to the antibodies that specifically bind CD38 are for example conservative substitutions with an amino acid having similar charge, hydrophobic, or stereochemical characteristics. Conservative substitutions may also be made to improve antibody properties, for example stability or affinity, or to improve antibody effector functions. 1, 2, 3, 4, 5, 6, 7, 8, 9, 10, 11, 12, 13, 14, or 15 amino acid substitutions may be made for example to the heavy or the light chain of the anti-CD38 antibody. Furthermore, any native residue in the VH or the VL may also be substituted with alanine, as has been previously described for alanine scanning mutagenesis (MacLennan et al., Acta Physiol Scand Suppl 643:55-67, 1998; Sasaki et al., Adv Biophys 35:1-24, 1998). Desired amino acid substitutions may be determined by those skilled in the art at the time such substitutions are desired. Amino acid substitutions may be done for example by PCR mutagenesis (U.S. Pat. No. 4,683,195). Libraries of variants may be generated using well known methods, for example using random (NNK) or non-random codons, for example DVK codons, which encode 11 amino acids (Ala, Cys, Asp, Glu, Gly, Lys, Asn, Arg, Ser, Tyr, Trp) and screening the libraries for variants with desired properties. The generated variants may be tested for their binding to CD38, their ability to induce ADCC, ADCP or apoptosis, or modulate CD38 enzymatic activity *in vitro* using methods described herein.

In some embodiments, the antibody that specifically binds CD38 may bind human CD38 with a range of affinities ($K_D$). In one embodiment according to the invention, and in some embodiments of each and every one of the numbered embodiments listed below, the antibody that specifically binds CD38 binds to CD38 with high affinity, for example, with a $K_D$ equal to or less than about $10^{-7}$ M, such as but not limited to, 1-9.9 (or any range or value therein, such as 1, 2, 3, 4, 5, 6, 7, 8, or 9) $\times 10^{-8}$ M, $10^{-9}$ M, $10^{-10}$ M, $10^{-11}$ M, $10^{-12}$ M, $10^{-11}$ M, $10^{-11}$ M, $10^{-15}$ M or any range or value therein, as determined by surface plasmon resonance or the Kinexa method, as practiced by those of skill in the art. One exemplary affinity is equal to or less than $1 \times 10^{-8}$ M. Another exemplary affinity is equal to or less than $1 \times 10^{-9}$ M.

In some embodiments, the antibody that specifically binds CD38 is a bispecific antibody. The VL and/or the VH regions of the existing anti-CD38 antibodies or the VL and VH regions identified *de novo* as described herein may be engineered into bispecific full length antibodies. Such bispecific antibodies may be made by modulating the CH3 interactions between the monospecific antibody heavy chains to form bispecific antibodies using technologies such as those described in U.S. Pat. No. 7,695,936; Intl. Pat. Publ. No. WO04/111233; U.S. Pat. Publ. No. US2010/0015133; U.S. Pat. Publ. No. US2007/0287170; Intl. Pat. Publ. No. WO2008/119353; U.S. Pat. Publ. No. US2009/0182127; U.S. Pat. Publ. No. US2010/0286374; U.S. Pat. Publ. No. US2011/0123532; Intl. Pat. Publ. No. WO2011/131746; Int. Pat. Publ. No. WO2011/143545; or U.S. Pat. Publ. No. US2012/0149876. Additional bispecific structures into which the VL and/or the VH regions of the antibodies of the invention may be incorporated are for example Dual Variable Domain Immunoglobulins (Inlt. Pat. Publ. No. WO2009/134776), or structures that include various dimerization domains to connect the two antibody arms with different specificity, such as leucine zipper or collagen dimerization domains (Int. Pat. Publ. No. WO2012/022811, U.S. Pat. No. 5,932,448; U.S. Pat. No. 6,833,441).

For example, bispecific antibodies may be generated *in vitro* in a cell-free environment by introducing asymmetrical mutations in the CH3 regions of two monospecific homodimeric antibodies and forming the bispecific heterodimeric antibody from two parental monospecific homodimeric antibodies in reducing conditions to allow disulfide bond isomerization according to methods described in Intl.Pat. Publ. No. WO2011/131746. In the methods, the first monospecific bivalent antibody (e.g., anti-CD38 antibody) and the second monospecific bivalent antibody are engineered to have certain substitutions at the CH3 domain that promote heterodimer stability; the antibodies are incubated together under reducing conditions sufficient to allow the cysteines in the hinge region to undergo disulfide bond isomerization; thereby generating the bispecific antibody by Fab arm exchange. The incubation conditions may optimally be restored to non-reducing. Exemplary reducing agents that may be used are 2- mercaptoethylamine (2-MEA), dithiothreitol (DTT), dithioerythritol (DTE), glutathione, tris(2-carboxyethyl)phosphine (TCEP), L-cysteine and beta-mercaptoethanol, preferably a reducing agent selected from the group consisting of: 2- mercaptoethylamine, dithiothreitol and tris(2-carboxyethyl)phosphine. For example, incubation for at least 90 min at a temperature of at least 20°C in the presence of at least 25 mM 2-MEA or in the presence of at least 0.5 mM dithiothreitol at a pH of from 5-8, for example at pH of 7.0 or at pH of 7.4 may be used.

Exemplary CH3 mutations that may be used in a first heavy chain and in a second heavy chain of the bispecific antibody are K409R and/or F405L.

The antibody of the invention may be used to treat an animal patient belonging to any classification. Examples of such animals include mammals such as humans, rodents, dogs, cats and farm animals.

**Administration/ Pharmaceutical Compositions**

The antibodies that specifically bind CD38 may be provided in suitable pharmaceutical compositions comprising the antibody that specifically bind CD38 and a pharmaceutically acceptable carrier. The carrier may be diluent, adjuvant, excipient, or vehicle with which the antibodies that specifically bind CD38 are administered. Such vehicles may be liquids, such as water and oils, including those of petroleum, animal, vegetable or synthetic origin, such as peanut oil, soybean oil, mineral oil, sesame oil and the like. For example, 0.4% saline and 0.3% glycine may be used. These solutions are sterile and generally free of particulate matter. They may be sterilized by conventional, well-known sterilization techniques (*e.g.*, filtration). The compositions may contain pharmaceutically acceptable auxiliary substances as required to approximate physiological conditions such as pH adjusting and buffering agents, stabilizing, thickening, lubricating and coloring agents, etc. The concentration of the antibodies that specifically bind CD38 in such pharmaceutical formulation may vary widely, *i.e.,* from less than about 0.5%, usually to at least about 1% to as much as 15 or 20%, 25%, 30%, 35%, 40%, 45% or 50% by weight and will be selected primarily based on required dose, fluid volumes, viscosities, etc., according to the particular mode of administration selected. Suitable vehicles and formulations, inclusive of other human proteins, e.g., human serum albumin, are described, for example, in e.g. Remington: The Science and Practice of Pharmacy, 21st Edition, Troy, D.B. ed., Lipincott Williams and Wilkins, Philadelphia, PA 2006, Part 5, Pharmaceutical Manufacturing pp 691-1092, see especially pp. 958-989.

The mode of administration of the antibodies that specifically bind CD38 may be any suitable route such as parenteral administration, e.g., intradermal, intramuscular, intraperitoneal, intravenous or subcutaneous, pulmonary, transmucosal (oral, intranasal, intravaginal, rectal) or other means appreciated by the skilled artisan, as well known in the art. The antibodies that specifically bind CD38 may be administered intratumorally, to a lymph node draining site for local delivery into the tumor using known methods.

The antibodies that specifically bind CD38 may be administered to a patient by any suitable route, for example parentally by intravenous *(i.v.)* infusion or bolus injection, intramuscularly or subcutaneously or intraperitoneally. *i.v.* infusion may be given over for example 15, 30, 60, 90, 120, 180, or 240 minutes, or from 1, 2, 3, 4, 5, 6, 7, 8, 9, 10, 11 or 12 hours.

The dose given to a patient is sufficient to alleviate or at least partially arrest the disease being treated ("therapeutically effective amount") and may be sometimes 0.005 mg to about 100 mg/kg, e.g. about 0.05 mg to about 30 mg/kg or about 5 mg to about 25 mg/kg, or about 4 mg/kg, about 8 mg/kg, about 16 mg/kg or about 24 mg/kg, or for example about 1, 2, 3, 4, 5, 6, 7, 8, 9 or 10 mg/kg, but may even higher, for example about 15, 16, 17, 18, 19, 20, 21, 22, 23, 24, 25, 30, 40, 50, 60, 70, 80, 90 or 100 mg/kg.

A fixed unit dose may also be given, for example, 50, 100, 200, 500 or 1000 mg, or the dose may be based on the patient's surface area, e.g., 500, 400, 300, 250, 200, or 100 mg/m$^2$. Usually between 1 and 8 doses, (e.g., 1, 2, 3, 4, 5, 6, 7 or 8) may be administered, but 9, 10, 11, 12, 13, 14, 15, 16, 17, 18, 19, 20 or more doses may be given.

The administration of the antibodies that specifically bind CD38 may be repeated after one day, two days, three days, four days, five days, six days, one week, two weeks, three weeks, one month, five weeks, six weeks, seven weeks, two months, three months, four months, five months, six months or longer. Repeated courses of treatment are also possible, as is chronic administration. The repeated administration may be at the same dose or at a different dose. For example, the antibodies that specifically bind CD38 may be administered at 8 mg/kg or at 16 mg/kg at weekly interval for 8 weeks, followed by administration at 8 mg/kg or at 16 mg/kg every two weeks for an additional 16 weeks, followed by administration at 8 mg/kg or at 16 mg/kg every four weeks by intravenous infusion.

The antibodies that specifically bind CD38 may be by maintenance therapy, such as, e.g. once a week for a period of 6 months or more.

For example, the antibodies that specifically bind CD38 may be provided as a daily dosage in an amount of about 0.1-100 mg/kg, such as 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of week 1, 2, 3, 4, 5, 6, 7, 8, 9, 10, 11, 12, 13, 14, 15, 16, 17, 18, 19 or 20 after initiation of treatment, or any combination thereof, using single or divided doses of every 24, 12, 8, 6, 4, or 2 hours, or any combination thereof.

The antibodies that specifically bind CD38 may also be administered prophylactically in order to reduce the risk of developing cancer, delay the onset of the occurrence of an event in cancer progression, and/or reduce the risk of recurrence when a cancer is in remission. This may be especially useful in patients wherein it is difficult to locate a tumor that is known to be present due to other biological factors.

The antibodies that specifically bind CD38 may be lyophilized for storage and reconstituted in a suitable carrier prior to use. This technique has been shown to be effective with conventional protein preparations and well known lyophilization and reconstitution techniques can be employed.

The antibodies that specifically bind CD38 may be administered in combination with a second therapeutic agent.

The antibodies that specifically bind CD38 may be administered together with any one or more of the chemotherapeutic drugs or other anti-cancer therapeutics known to those of skill in the art. Chemotherapeutic agents are chemical compounds useful in the treatment of cancer and include growth inhibitory agents or other cytotoxic agents and include alkylating agents, anti-metabolites, anti-microtubule inhibitors, topoisomerase inhibitors, receptor tyrosine kinase inhibitors, angiogenesis inhibitors and the like. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-FU; folic acid analogues such as denopterin, methotrexate, pteropterin, trimetrexate; purine analogues such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; members oftaxoid or taxane family, such as paclitaxel (TAXOL®docetaxel (TAXOTERE®) and analogues thereof; chlorambucil; gemcitabine; 6-thioguanine; mercaptopurine; methotrexate; platinum analogues such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; esperamicins; capecitabine; inhibitors of receptor tyrosine kinases and/or angiogenesis, including NEXAVAR® (sorafenib), SUTENT® (sunitinib), VOTRIENT™ (pazopanib), PALLADIA™ (toceranib), ZACTIMA™ (vandetanib), RECENTIN® (cediranib), regorafenib (BAY 73-4506), axitinib (AG013736), lestaurtinib (CEP-701), TARCEVA® (erlotinib), IRESSA™ (gefitinib), Gilotrif® (afatinib), TYKERB® (lapatinib), neratinib, and the like, and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY 117018, onapristone, and FARESTON® (toremifene); and anti-androgens such as flutamide, nilutamide, bicalutamide, leuprolide, and goserelin; and pharmaceutically acceptable salts, acids or derivatives of any of the above. Other conventional cytotoxic chemical compounds as those disclosed in Wiemann et al., 1985, in Medical Oncology (Calabresi et aL, eds.), Chapter 10, McMillan Publishing, are also applicable.

Exemplary agents that may be used in combination with the antibody that specifically binds CD38 include tyrosine kinase inhibitors and targeted anti-cancer therapies such as IRESSA™ (gefitinib) and Tarceva® (erlotinib) and other antagonists of HER2, HER3, HER4 or VEGF. Exemplary HER2 antagonists include CP-724-714, HERCEPTIN™ (trastuzumab), OMNITARG™ (pertuzumab), TAK-165, TYKERB® (lapatinib) (EGFR and HER2 inhibitor), and GW-282974. Exemplary HER3 antagonists include anti-Her3 antibodies (see e.g., U.S. Pat. Publ. No. 2004/0197332). Exemplary HER4 antagonists include anti-HER4 siRNAs (see e.g., Maatta et al., Mol Biol Cell 17: 67-79, 2006,. An exemplary VEGF antagonist is (Avastin™ (Bevacizumab).

Exemplary agents that may be used in combination with the antibody that specifically binds CD38 include standard of care drugs for solid tumors, or an immune checkpoint inhibitor.

The second therapeutic agent may be an immune checkpoint inhibitor.

In some embodiments, the immune checkpoint inhibitor is an anti-PD-1 antibody, an anti-PD-L1 antibody, an anti-PD-L2 antibody, an anti-LAG3 antibody, an anti-TIM3 antibody, or an anti-CTLA-4 antibody.

In some embodiments, the immune checkpoint inhibitor is an antagonistic anti-PD-1 antibody, an antagonistic anti-PD-L1 antibody, an antagonistic anti-PD-L2 antibody, an antagonistic anti-LAG3 antibody, or an antagonistic anti-TIM3 antibody.

In some embodiments, the immune checkpoint inhibitor is an anti-PD-1 antibody.

In some embodiments, the immune checkpoint inhibitor is an anti-PD-L1 antibody.

In some embodiments, the immune checkpoint inhibitor is an anti-PD-L2 antibody.

In some embodiments, the immune checkpoint inhibitor is an anti-LAG3 antibody.

In some embodiments, the immune checkpoint inhibitor is an anti-TIM3 antibody.

In some embodiments, the immune checkpoint inhibitor is an anti-CTLA-4 antibody.

Any antagonistic anti-PD-1 antibodies may be used. Exemplary anti-PD-1 antibodies that may be used are OPVIDO® (nivolumab) and KEYTRUDA® (pembrolizumab). OPVIDO® (nivolumab) is described in for example US. Pat. No. 8,008,449 (antibody 5C4) and comprises the VH of SEQ ID NO: 24 and the VL of SEQ ID NO: 25. KEYTRUDA® (pembrolizumab) is described in for example U.S. Pat.No 8,354,509 and comprises the VH of SEQ ID NO: 22 and the VL of SEQ ID NO: 23. The amino acid sequences of nivolumab and pembrolizumab are also available through the CAS registry. Additional PD-1 antibodies that may be used are described in U.S. Pat. No. 7,332,582, U.S. Pat. Publ. No. 2014/0044738, Int. Pat. Publ. No. WO2014/17966 and U.S. Pat. Publ. No. 2014/0356363.

"Antagonist" refers to a molecule that, when bound to a cellular protein, suppresses at least one reaction or activity that is induced by a natural ligand of the protein. A molecule is an antagonist when the at least one reaction or activity is suppressed by at least about 30%, 40%, 45%, 50%, 55%, 60%, 65%, 70%, 75%, 80%, 85%, 90%, 95%, or 100% more than the at least one reaction or activity suppressed in the absence of the antagonist (e.g., negative control), or when the suppression is statistically significant when compared to the suppression in the absence of the antagonist. Antagonist may be an antibody, a soluble ligand, a small molecule, a DNA or RNA such as siRNA. A typical reaction or activity that is induced for example by PD-1 binding to its receptor PD-L1 or PD-L2 may be reduced antigen-specific CD4$^+$ or CD8$^+$ cell proliferation or reduced interferon-$\gamma$ (IFN-$\gamma$) production by T cells, resulting in suppression of immune responses against for example tumor. A typical reaction or activity that is induced by TIM-3 binding to its receptor, such as galectin-9, may be reduced antigen specific CD4$^+$ or CD8$^+$ cell proliferation, reduced IFN-$\gamma$ production by T cells, or reduced CD137 surface expression on CD4$^+$ or CD8$^+$ cells, resulting in suppression of immune responses against for example tumor. Hence, an antagonistic PD-1 antibody specifically binding PD-1, an antagonistic PD-L2, an antagonistic antibody specifically binding TIM-3 induces immune responses by inhibiting the inhibitory pathways.

SEQ ID NO: 22

QVQLVQSGVEVKKPGASVKVSCKASGYTFTNYYMYWVRQAPGQGLEWMGG
INPSNGGTNFNEKFKNRVTLTTDSSTTTAYMELKSLQFDDTAVYYCARRDYRFDM
GFDYWGQGTTVTVSS

SEQ ID NO: 23

EIVLTQSPATLSLSPGERATLSCRASKGVSTSGYSYLHWYQQKPGQAPRLLIYLAS
YLESGVPARFSGSGSGTDFTLTISSLEPEDFAVYYCQHSRDLPLTFGGGTKVEIK

SEQ ID NO: 24

QVQLVESGGGVVQPGRSLRLDCKASGITFSNSGMHWVRQAPGKGLEWVAVIWY
DGSKRYYADSVKGRFTISRDNSKNTLFLQMNSLRAEDTAVYYCATNDDYWGQG
TLVTVSS

SEQ ID NO: 25

EIVLTQSPATLSLSPGERATLSCRASQSVSSYLAWYQQKPGQAPRLLIYDASNRAT
GIPARFSGSGSGTDFTLTISSLEPEDFAVYYCQQSSNWPRTFGQGTKVEIK

Anti-PD-L1 antibodies that enhance immune response may be used (e.g. antagonistic anti-PD-L1 antibodies). Exemplary anti-PD-L1 antibodies that may be used are durvalumab, atezolizumab and avelumab, and those described in, for example, U.S. Pat. Publ. No. 2009/0055944, U.S. Pat. No. U.S. Pat. No. 8,552,154, U.S. Pat. No. 8,217,149 and U.S. Pat. No. 8,779,108.

Durvalumab comprises the VH of SEQ ID NO: 26 and the VL of SEQ ID NO: 27.

Atezolizumab comprises the VH of SEQ ID NO: 28 and the VL of SEQ ID NO: 29.

Avelumab comprises the VH of SEQ ID NO: 30 and the VL of SEQ ID NO: 31.

SEQ ID NO: 26

EVQLVESGGG LVQPGGSLRLSCAASGFTFSRYWMSWVRQAPGKGLEWVAN
IKQDGSEKYYVDSVKGRFTISRDNAKNSLYLQMNSLRAEDTAVYYCAREG
GWFGELAFDYWGQGTLVTVSS

SEQ ID NO: 27

EIVLTQSPGTLSLSPGERATLSCRASQRVSSSYLAWYQQK PGQAPRLLIY
DASSRATGIPDRFSGSGSGTDFTLTISRLEPEDFAVYYCQQYGSLPWTFG
QGTKVEIK

SEQ ID NO: 28

EVQLVESGGGLVQPGGSLRLSCAASGFTFSDSWIHWVRQAPGKGLEWVAW
ISPYGGSTYYADSVKGRFTISADTSKNTAYLQMNSLRAEDTAVYYCARRH
WPGGFDYWGQGTLVTVSS

SEQ ID NO: 29

DIQMTQSPSSLSASVGDRVTITCRASQDVSTAVAWYQQKPGKAPKLLIYS
ASFLYSGVPSRFSGSGSGTDFTLTISSLQPEDFATYYCQQYLYHPATFGQ
GTKVEIK

SEQ ID NO: 30

EVQLLESGGGLVQPGGSLRLSCAASGFTFSSYIMMWVRQAPGKGLEWVSS
IYPSGGITFYADTVKGRFTISRDNSKNTLYLQMNSLRAEDTAVYYCARIK
LGTVTTVDYWGQGTLVTVSS

SEQ ID NO: 31

QSALTQPASVSGSPGQSITISCTGTSSDVGGYNYVSWYQQHPGKAPKLMI
YDVSNRPSGVSNRFSGSKSGNTASLTISGLQAEDEADYYCSSYTSSSTRV
FGTGTKVTVL

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 24 and the VL of SEQ ID NO: 25 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 22 and the VL of SEQ ID NO: 23 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 26 and the VL of SEQ ID NO: 27 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 28 and the VL of SEQ ID NO: 29 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 30 and the VL of SEQ ID NO: 31 for a time sufficient to treat the solid tumor.

The disclosure also relates to a method of enhancing an immune response in a patient, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 24 and the VL of SEQ ID NO: 25 for a time sufficient to enhance the immune response.

The disclosure also relates to a method of enhancing an immune response in a patient, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 22 and the VL of SEQ ID NO: 23 for a time sufficient to enhance the immune response.

The disclosure also relates to a method of enhancing an immune response in a patient, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 26 and the VL of SEQ ID NO: 27 for a time sufficient to enhance the immune response.

The disclosure also relates to method of enhancing an immune response in a patient, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 28 and the VL of SEQ ID NO: 29 for a time sufficient to enhance the immune response.

The disclosure also relates to a method of enhancing an immune response in a patient, comprising administering to the patient in need thereof a therapeutically effective amount of an antibody that specifically binds CD38 comprising the VH of SEQ ID NO: 4 and the VL of SEQ ID NO: 5 in combination with an anti-PD-L1 antibody comprising the VH of SEQ ID NO: 30 and the VL of SEQ ID NO: 31 for a time sufficient to enhance the immune response.

The disclosure also relates to a method of treating a patient having a colorectal cancer, comprising administering to the patient in need thereof a therapeutically effective amount of an antibody that specifically binds CD38 in combination with an antagonistic anti-PD-1 antibody for a time sufficient to treat the colorectal cancer.

The disclosure also relates to a method of treating a patient having a colorectal cancer, comprising administering to the patient in need thereof a therapeutically effective amount of an antibody that specifically binds CD38 in combination with an antagonistic anti-PD-L1 antibody for a time sufficient to treat the colorectal cancer.

The disclosure also relates to a method of treating a patient having a colorectal cancer, comprising administering to the patient in need thereof a therapeutically effective amount of an antibody that specifically binds CD38 in combination with an antagonistic anti-PD-L2 antibody for a time sufficient to treat the colorectal cancer.

The disclosure also relates to a method of treating a patient having a lung cancer, comprising administering to the patient in need thereof a therapeutically effective amount of an antibody that specifically binds CD38 in combination with an antagonistic anti-PD-1 antibody for a time sufficient to treat the lung cancer.

The disclosure also relates to a method of treating a patient having a lung cancer, comprising administering to the patient in need thereof a therapeutically effective amount of an antibody that specifically binds CD38 in combination with an antagonistic anti-PD-L1 antibody for a time sufficient to treat the lung cancer.

The disclosure also relates to a method of treating a patient having a lung cancer, comprising administering to the patient in need thereof a therapeutically effective amount of an antibody that specifically binds CD38 in combination with an antagonistic anti-PD-L2 antibody for a time sufficient to treat the lung cancer.

The disclosure also relates to a method of treating a patient having a prostate cancer, comprising administering to the patient in need thereof a therapeutically effective amount of an antibody that specifically binds CD38 in combination with an antagonistic anti-PD-1 antibody for a time sufficient to treat the prostate cancer.

The disclosure also relates to a method of treating a patient having a prostate cancer, comprising administering to the patient in need thereof a therapeutically effective amount of an antibody that specifically binds CD38 in combination with an antagonistic anti-PD-L1 antibody for a time sufficient to treat the prostate cancer.

The disclosure also relates to a method of treating a patient having a prostate cancer, comprising administering to the patient in need thereof a therapeutically effective amount of an antibody that specifically binds CD38 in combination with an antagonistic anti-PD-L2 antibody for a time sufficient to treat the prostate cancer.

Anti-LAG-3 antibodies that enhance immune response may be used in the invention. Exemplary anti-LAG-3 antibodies that may be used are those described in, for example, Int. Pat. Publ. No. WO2010/019570.

Anti-CTLA-4 antibodies that enhance immune response may be used in the invention. An exemplary anti-CTLA-4 antibody that may be used is ipilimumab.

Anti-PD-1, anti-PD-L1, anti-PD-L2, anti-LAG3, anti-TIM3 and anti-CTLA-4 antibodies that may be used in the invention may also be generated *de novo* using methods described herein.

In some embodiments, anti-PD1 antibodies comprising the VH of SEQ ID NO: 32 and the VL of SEQ ID NO: 33 may be used.

In some embodiments, anti-PD1 antibodies comprising the VH of SEQ ID NO: 34 and the VL of SEQ ID NO: 35 may be used.

In some embodiments, anti-TIM-3 antibodies comprising the VH of SEQ ID NO: 36 and the VL of SEQ ID NO: 37 may be used.

In some embodiments, anti-TIM-3 antibodies comprising the VH of SEQ ID NO: 38 and the VL of SEQ ID NO: 39 may be used.

SEQ ID NO: 32

QVQLVQSGAEVKKPGSSVKVSCKASGGTFSSYAISWVRQAPGQGLEWMGGIIPIF
DTANYAQKFQGRVTITADESTSTAYMELSSLRSEDTAVYYCARPGLAAAYDTGSL
DYWGQGTLVTVSS

SEQ ID NO: 33

EIVLTQSPATLSLSPGERATLSCRASQSVRSYLAWYQQKPGQAPRLLIYDASNRAT
GIPARFSGSGSGTDFTLTISSLEPEDFAVYYCQQRNYWPLTFGQGTKVEIK

SEQ ID NO: 34

EVQLVESGGGLVQPGGSLRLSCAASGFAFSRYDMSWVRQAPGKGLESVAYISGG
GANTYYLDNVKGRFTISRDNAKNSLYLQMNSLRAEDTAVYYCASPYLSYFDVWG
QGTLVTVSS

SEQ ID NO: 35

EIVMTQSPATLSVSPGERATLSCRASQSLSDYLHWYQQKPGQAPRLLIKSASQSISG
IPARFSGSGSGTEFTLTISSLQSEDFAVYYCQNGHSFPYTFGQGTKLEIK

SEQ ID NO: 36

EVQLLESGGGLVQPGGSLRLSCAASGFTFSSYAMSWVRQAPGKGLEWVSAISGSG
GSTYYADSVKGRFTISRDNSKNTLYLQMNSLRAEDTAVYYCAKSPYAPLDYWGQ
GTLVTVSS

SEQ ID NO: 37

EIVLTQSPATLSLSPGERATLSCRASQSVNDYLAWYQQKPGQAPRLLIYDASNRAT
GIPARFSGSGSGTDFTLTISSLEPEDFAVYYCQQGGHAPITFGQGTKVEIK

SEQ ID NO: 38

EVQLVQSGAEVKKPGESLKISCKGSGYSFTSYWMQWVRQMPGKGLEWMGAIYP GDGDIRYTQNFKGQVTISADKSISTAYLQWSSLKASDTAMYYCARWEKSTTVVQ RNYFDYWGQGTTVTVSS

SEQ ID NO: 39

DIQMTQSPSSLSASVGDRVTITCKASENVGTFVSWYQQKPGKAPKLLIYGASNRY TGVPSRFSGSGSGTDFTLTISSLQPEDFATYYCGQSYSYPTFGQGTKLEIK

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 32 and the VL of SEQ ID NO: 33 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-PD-1 antibody comprising the VH of SEQ ID NO: 34 and the VL of SEQ ID NO: 35 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-TIM-3 antibody comprising the VH of SEQ ID NO: 36 and the VL of SEQ ID NO: 37 for a time sufficient to treat the solid tumor.

The disclosure also relates to a method of treating a patient having a solid tumor, comprising administering to the patient in need thereof a therapeutically effective amount of an antibody that specifically binds CD38 comprising the VH of SEQ ID NO: 4 and the VL of SEQ ID NO: 5 in combination with an anti-TIM-3 antibody comprising the VH of SEQ ID NO: 38 and the VL of SEQ ID NO: 39 for a time sufficient to treat the solid tumor.

The combination of the antibody that specifically binds CD38 and the second therapeutic agent may be administered over any convenient timeframe. For example, the antibody that specifically binds CD38 and the second therapeutic agent may be administered to a patient on the same day, and even in the same intravenous infusion. However, the antibody that specifically binds CD38 and the second therapeutic agent may also be administered on alternating days or alternating weeks or months, and so on. In some methods, the antibody that specifically binds CD38 and the second therapeutic agent may be administered with sufficient proximity in time that they are simultaneously present (e.g., in the serum) at detectable levels in the patient being treated. In some methods, an entire course of treatment with the antibody that specifically binds CD38 consisting of a number of doses over a time period is followed or preceded by a course of treatment with the second therapeutic agent, consisting of a number of doses. A recovery period of 1, 2 or several days or weeks may be used between administration of the antibody that specifically binds CD38 and the second therapeutic agent.

The antibody that specifically binds CD38 or a combination of the antibody that specifically binds CD38 and the second therapeutic agent may be administered together with any form of radiation therapy including external beam radiation, intensity modulated radiation therapy (IMRT), focused radiation, and any form of radiosurgery including Gamma Knife, Cyberknife, Linac, and interstitial radiation (e.g. implanted radioactive seeds, GliaSite balloon), and/or with surgery.

Focused radiation methods that may be used include stereotactic radiosurgery, fractionated stereotactic radiosurgery, and intensity-modulated radiation therapy (IMRT). It is apparent that stereotactic radiosurgery involves the precise delivery of radiation to a tumorous tissue, for example, a brain tumor, while avoiding the surrounding nontumorous, normal tissue. The dosage of radiation applied using stereotactic radiosurgery may vary, typically from 1 Gy to about 30 Gy, and may encompass intermediate ranges including, for example, from 1 to 5, 10, 15, 20, 25, up to 30 Gy in dose. Because of noninvasive fixation devices, stereotactic radiation need not be delivered in a single treatment. The treatment plan may be reliably duplicated day-to-day, thereby allowing multiple fractionated doses of radiation to be delivered. When used to treat a tumor over time, the radiosurgery is referred to as "fractionated stereotactic radiosurgery" or FSR. In contrast, stereotactic radiosurgery refers to a one-session treatment. Fractionated stereotactic radiosurgery may result in a high therapeutic ratio, i.e., a high rate of killing of tumor cells and a low effect on normal tissue. The tumor and the normal tissue respond differently to high single doses of radiation vs. multiple smaller doses of radiation. Single large doses of radiation may kill more normal tissue than several smaller doses of radiation may. Accordingly, multiple smaller doses of radiation can kill more tumor cells while sparing normal tissue. The dosage of radiation applied using fractionated stereotactic radiation may vary from range from 1 Gy to about 50 Gy, and may encompass intermediate ranges including, for example, from 1 to 5, 10, 15, 20, 25, 30, 40, up to 50 Gy in hypofractionated doses. Intensity-modulated radiation therapy (IMRT) may also be used. IMRT is an advanced mode of high-precision three-dimensional conformal radiation therapy (3DCRT), which uses computer-controlled linear accelerators to deliver precise radiation doses to a malignant tumor or specific areas within the tumor. In 3DCRT, the profile of each radiation beam is shaped to fit the profile of the target from a beam's eye view (BEV) using a multileaf collimator (MLC), thereby producing a number of beams. IMRT allows the radiation dose to conform more precisely to the three-dimensional (3-D) shape of the tumor by modulating the intensity of the radiation beam in multiple small volumes. Accordingly, IMRT allows higher radiation doses to be focused to regions within the tumor while minimizing the dose to surrounding normal critical structures. IMRT improves the ability to conform the treatment volume to concave tumor shapes, for example, when the tumor is wrapped around a vulnerable structure, such as the spinal cord or a major organ or blood vessel.

Subcutaneous administration of pharmaceutical compositions comprising an antibody that specifically binds CD38 and a hyaluronidase

The antibody that specifically binds CD38 may be administered as a pharmaceutical composition comprising the antibody that specifically binds CD38 and a hyaluronidase subcutaneously.

The concentration of the antibody that specifically binds CD38 in the pharmaceutical composition administered subcutaneously may be about 20 mg/ml.

The pharmaceutical composition administered subcutaneously may comprise between about 1,200 mg - 1,800 mg of the antibody that specifically binds CD38.

The pharmaceutical composition administered subcutaneously may comprise about 1,200 mg of the antibody that specifically binds CD38.

The pharmaceutical composition administered subcutaneously may comprise about 1,600 mg of the antibody that specifically binds CD38.

The pharmaceutical composition administered subcutaneously may comprise about 1,800 mg of the antibody that specifically binds CD38.

The pharmaceutical composition administered subcutaneously may comprise between about 30,000 U - 45,000 U of the hyaluronidase.

The pharmaceutical composition administered subcutaneously may comprise about 1,200 mg of the antibody that specifically binds CD38 and about 30,000 U of the hyaluronidase.

The pharmaceutical composition administered subcutaneously may comprise about 1,800 mg of the antibody that specifically binds CD38 and about 45,000 U of the hyaluronidase.

The pharmaceutical composition administered subcutaneously may comprise about 1,600 mg of the antibody that specifically binds CD38 and about 30,000 U of the hyaluronidase.

The pharmaceutical composition administered subcutaneously may comprise about 1,600 mg of the antibody that specifically binds CD38 and about 45,000 U of the hyaluronidase.

The pharmaceutical composition administered subcutaneously may comprise the hyaluronidase rHuPH20 having the amino acid sequence of SEQ ID NO: 40.

rHuPH20 is a recombinant hyaluronidase (HYLENEXOO recombinant) and is described in Int. Pat. Publ. No. WO2004/078140.

Hyaluronidase is an enzyme that degrades hyaluronic acid (EC 3.2.1.35) and lowers the viscosity of hyaluronan in the extracellular matrix, thereby increasing tissue permeability.
SEQ ID NO: 40

MGVLKFKHIFFRSFVKSSGVSQIVFTFLLIPCCLTLNFRAPPVIPNVPFLWAWNAPS
EFCLGKFDEPLDMSLFSFIGSPRINATGQGVTIFYVDRLGYYPYIDSITGVTVNGGIP
QKISLQDHLDKAKKDITFYMPVDNLGMAVIDWEEWRPTWARNWKPKDVYKNRS
IELVQQQNVQLSLTEATEKAKQEFEKAGKDFLVETIKLGKLLRPNHLWGYYLFPD
CYNHHYKKPGYNGSCFNVEIKRNDDLSWLWNESTALYPSIYLNTQQSPVAATLY
VRNRVREAIRVSKIPDAKSPLPVFAYTRIVFTDQVLKFLSQDELVYTFGETVALGA
SGIVIWGTLSIMRSMKSCLLLDNYMETILNPYIINVTLAAKMCSQVLCQEQGVCIR
KNWNSSDYLHLNPDNFAIQLEKGGKFTVRGKPTLEDLEQFSEKFYCSCYSTLSCK
EKADVKDTDAVDVCIADGVCIDAFLKPPMETEEPQIFYNASPSTLSATMFIVSILFLI
ISSVASL

The administration of the pharmaceutical composition comprising the antibody that specifically binds CD38 and the hyaluronidase may be repeated after one day, two days, three days, four days, five days, six days, one week, two weeks, three weeks, four weeks, five weeks, six weeks, seven weeks, two months, three months, four months, five months, six months or longer. Repeated courses of treatment are also possible, as is chronic administration. The repeated administration may be at the same dose or at a different dose. For example, the pharmaceutical composition comprising the antibody that specifically binds CD38 and the hyaluronidase may be administered once weekly for eight weeks, followed by once in two weeks for 16 weeks, followed by once in four weeks. The pharmaceutical compositions to be administered may comprise about 1,200 mg of the antibody that specifically binds CD38 and about 30,000 U of hyaluronidase, wherein the concentration of the antibody that specifically binds CD38 in the pharmaceutical composition is about 20 mg/ml. The pharmaceutical compositions to be administered may comprise about 1,800 mg of the antibody that specifically binds CD38 and about 45,000 U of hyaluronidase. The pharmaceutical compositions to be administered may comprise about 1,600 mg of the antibody that specifically binds CD38 and about 30,000 U of hyaluronidase. The pharmaceutical compositions to be administered may comprise about 1,600 mg of the antibody that specifically binds CD38 and about 45,000 U of hyaluronidase.

The pharmaceutical composition comprising the antibody that specifically binds CD38 and the hyaluronidase may be administered subcutaneously to the abdominal region.

The pharmaceutical composition comprising the antibody that specifically binds CD38 and the hyaluronidase may be administered in a total volume of about 80 ml, 90 ml, 100 ml, 110 ml or 120 ml.

For administration, 20 mg/ml of the antibody that specifically binds CD38 in 25 mM sodium acetate, 60 mM sodium chloride, 140 mM D-mannitol, 0.04% polysorbate 20, pH 5.5 may be mixed with rHuPH20, 1.0 mg/mL (75-150 kU/mL) in 10 mM L-Histidine, 130 mM NaCl, 10 mM L-Methionine, 0.02% Polysorbate 80, pH 6.5 prior to administration of the mixture to a subject.

While having described the invention in general terms, the embodiments of the invention will be further disclosed in the following examples that should not be construed as limiting the scope of the claims.

**Example 1. General materials and methods**

**Sample collection and processing**

Peripheral blood and bone marrow aspirates were collected in heparinized tubes at baseline immediately prior to the first infusion and at specified time points during treatment. The majority of samples were evaluated using real-time flow cytometry, as they arrived at a central laboratory, 24-48 hours after collection. Peripheral blood mononuclear cells (PBMCs) were obtained from whole blood, isolated by density-gradient centrifugation, and stored frozen until analysis. For the T-cell activation, clonality, and CD38$^+$ Treg suppression assays, pre- and post-treatment samples were analyzed at the same time, using frozen PBMC samples.

Flow cytometric analysis of these samples was performed at BARC global central laboratory for evaluation of NK, T, B, myeloma cells (CD138$^+$) and CD38 expression using a pre-validated immunophenotyping assay. Briefly, blood samples and bone marrow samples were stained with the following multifluorochrome antibody panels: cell lineage panel: PerCPCy5.5$\alpha$-CD19 (cloneHIB19; Becton Dickinson [BD]), APC$\alpha$-CD24 (SN3; eBioscience), PC7$\alpha$-CD3 (UCHT-1; Beckman Coulter), V500$\alpha$-CD16 (3G8; BD), and PE$\alpha$-CD56 (MY; BD); regulatory T cell (T$_{reg}$) panel: APC$\alpha$-CD25 (2A3; BD), PE$\alpha$-CD127 (HIL-7R-M21; BD), APC-H7$\alpha$-HLA-DR (G46-6; BD), and PerCP$\alpha$-CD4 (L200; BD); naive/memory T-cell panel: APC-H7α-CD4 (RPA-T4; BD), PerCP-Cy5.5α-CD8 (RPA-T4 BD), PEα-CD62L (SK11; BD), and APCα-CD45RA (HI100; BD). CD38 expression was evaluated using Alexa 647 labeled antibody mAb 003 described in U.S. Pat. No. 7,829,693 having the VH and the VL sequences of SEQ ID NO: 14 and SEQ ID NO: 15. The blood samples were prepared using different Lyse-wash methods. For bone marrow aspirate samples either membrane or intracellular staining was performed with various antibodies. Becton Dickinson FACSLysing solution was used for lysing red blood cells in peripheral blood samples and Fix and Perm cell permeabilization reagents from Invitrogen were used for intracellular staining of bone marrow aspirate samples. Stained samples were acquired on FACS Canto II flow cytometers and data was analyzed using FacsDiva software. Absolute counts of immune cell populations in the blood samples and as percent of lymphocytes in bone marrow samples were determined at all the time points tested.

## T-cell receptor (TCR) sequencing

T-cell diversity was analyzed by deep sequencing of TCR rearrangements to assess CD8[+] T-cell clonality using genomic DNA from PBMC samples. TCR sequencing was performed using Adaptive Biotechnologies commercial Immunoseq™ assay, and analysis was performed using prequalified multiplex polymerase chain reaction (PCR) assays (TR2015CRO-V-019), which were composed of forward and reverse primers that directly targeted the family of variable (V) genes (forward primers) and joining (J) genes (reverse primers). Each V and J gene primer acted as priming pairs to amplify somatically recombined TCRs, and each primer contained a specific universal DNA sequence. Following the initial PCR amplification, each amplicon was amplified a second time with forward and reverse primers containing the universal sequence and adaptor sequence needed for DNA sequencing by Illumina.

## T-cell responses to viral- and alloantigens

Patient PBMCs were seeded on 96 well plates ($2\times10^5$ cells/well) and stimulated for 5 days with a cocktail of 23 major histocompatibility complex (MHC) class I-restricted viral peptides from human cytomegalovirus (CMV), Epstein-Barr virus (EBV), and influenza virus (2 μg/ml; CEF peptide pool; PANATecs®) or an equivalent number of 25-Gy irradiated allogeneic PBMCs from healthy donors. Unstimulated PBMCs and PBMCs stimulated with anti-CD3/CD28-coated beads served as negative and positive controls, respectively. On day 5, interferon y (IFN-y) from cell-free supernatant was measured by sandwich enzyme-linked immunosorbent assay (ELISA; Human IFN gamma ELISA Ready-SET-Go; eBioscience) and served as a surrogate marker for T-cell activation.

## Regulatory T-cell (Treg) suppression of effector cell functions: Carboxyfluorescein succinimidyl (CFSE) dilution assay

PBMCs from healthy donors were labelled with PerCP-Cy5.5α-CD3 (SK7; BD), KOα-CD45, (J33; Beckman Coulter), V450α-CD4 (SK3; BD), PEα-CD25 (M-A251, BD), PE Cy7α-CD127 (HIL-7R-M21; BD), and APCα-CD38 (HB-7; BD) and sorted by FACS Aria (BD). Sorted effector cells were labelled with carboxyfluorescein succinimidyl ester (CFSE; eBioscience) and stimulated with anti-CD3/CD28-coated beads in the presence or absence of CD38[+]Tregs or CD38[-] Tregs (1:1 Treg to effector cell ratio) in RPMI plus 10% fetal calf serum. After 72 hours, flow cytometry was performed and the percent dilution of CFSE was used as a surrogate for T-cell proliferation.

## Myeloid derived suppressor cell (MDSC) phenotyping and DARZALEX™ (daratumumab)-mediated ADCC

PBMC from three normal healthy donors were co-cultured with myeloma tumor cell lines (RPMI8226, U266, H929) for six days, and evaluated for the production of granulocytic MDSC (G-MDSC) (CD11b[+]CD14[-]HLA[-]DR[-]CD15[+]CD33[+]) as described in Gorgun et al., Blood 121:2975-87, 2013. G-MDSC were not present in normal healthy PBMC, however following co-culture with all three myeloma cells lines G-MDSC were present as 5- 25% of total PBMC population (data not shown). Gating strategy for flow cytometric evaluation of G-MDSC included CD11b[+] as the first gate, followed by CD14[-]and HLA[-]DR[-] gating, and then followed by CD15[+] and CD33[+] gating. G-MDSCs were cell sorted and evaluated for CD38 expression levels and sensitivity to DARZALEX™ (daratumumab) mediated ADCC. To evaluate the effect of DARZALEX™ (daratumumab) on ADCC/CDC of MDSCs, serum containing complement or an isotype control was added to ADCC assays.

## Naïve and memory T-cell analysis

Heparinized peripheral-blood samples were obtained from patients prior to each infusion of DARZALEX™ (daratumumab). Peripheral-blood mononuclear cells (PBMCs) were isolated by Ficoll-Hypaque density-gradient centrifugation and stored in cryopreservation medium (RPMI supplemented with 10% human serum and 10% dimethyl sulfoxide) in liquid nitrogen. For FACS analysis, PBMCs were thawed and $2\times10^6$ cells/panel was resuspended in phosphate-buffered saline (PBS) with 0.05% azide and 0.1% HAS.

**Data analysis**

All data analysis and generation of relevant graphs were performed exclusively using R software (R: A Language and Environment for Statistical Computing, R Development Core Team, R Foundation for Statistical Computing, Vienna, Austria, 2011, ISBN 3-900051-07-0 http_//_www_R-project_org/). All treated subjects with an evaluable response were included in the data analysis. Consistently throughout, responders are defined as subjects with a Best Response per IRC of sCR, VGPR and PR, and non-responders are defined as subjects with a Best Response per IRC of MR, SD and PD.

Different statistical comparisons included (i) baseline levels between responders and non-responders, (ii) baseline versus on treatment for responders and for non-responders, (iii) percent changes between responders and non-responders, (iv) ratio changes of baseline versus on treatment. Each comparison included first a test for normality with a Shapiro-Wilk test (Royston (1995) Remark AS R94: A remark on Algorithm AS 181: The W test for normality. Applied Statistics, 44, 547-551). Almost exclusively, the data was found to not have a normal distribution. The differential level testing included conducting both a non-parametric Wilcox rank sum test (Hollander and Wolfe (1973), Nonparametric Statistical Methods. New York: John Wiley & Sons. Pages 27-33 (one-sample), 68-75 (two-sample), and at-test following a Box Cox transformation (Weisberg, S. (2014) Applied Linear Regression, Fourth Edition, Wiley Wiley, Chapter 7). For the Box Cox transformation, a small number (1e-07) was added to values equal to zero. In all cases, the two tests agreed. The Wilcox rank sum test p-values are shown in the tables throughout the specification unless otherwise indicated. When testing for differences of the baseline versus on treatment for responders and non-responders a two-group paired test per subject was ran, in all other cases a two group unpaired test was conducted.

As samples to analyze various lymphocyte populations were not taken at identical time points for the different dosing schedules, population modeling was conducted. Model fitting was done on the rank of the visits. Population modeling on total and activated NK cells involved fitting a broken stick model (Lutz et al., "Statistical model to estimate a threshold dose and its confidence limits for the analysis of sublinear dose-response relationships, exemplified for mutagenicity data." Mutation Research/Genetic Toxicology and Environmental Mutagenesis 678.2 (2009): 118-122.). Linear mixed effect models with random intercept and slope were fit on the B-cell, T-cell subpopulations, and leukocytes, monocytes, neutrophils and lymphocytes patient population data (Bates et al., (2014). "lme4: Linear mixed-effects models using Eigen and S4." ArXiv e-print; submitted to Journal of Statistical Software, http:_//_arxiv_org/abs/_1406.5823). This linear mixed modeling was done on the relative day since treatment start (ADY). The linear mixed model fitting were done on log transformed response variables. In case of response variable values equal to zero, 0.1 was added to all response variable values to allow for modeling on log scale.

**Example 2. Study 54767414MMY2002 design (SIRIUS)**

The target population for Study 54767414MMY2002 (SIRIUS) is patients with advanced multiple myeloma who received at least 3 prior lines of therapy including a proteasome inhibitor (PI) and an immunomodulatory drug (IMiD) or double refractory to a PI and an IMiD. Response evaluations for the primary endpoint/final analysis were based on assessments from an independent review committee (IRC) and computerized algorithm, using 2011 IMWG Guidelines (Clinical Study Report: An Open-label, Multicenter, Phase 2 Trial Investigating the Efficacy and Safety of DARZALEXTM (daratumumab) in Subjects With Multiple Myeloma Who Have Received at Least 3 Prior Lines of Therapy (Including a Proteasome Inhibitor and IMiD) or are Double Refractory to a Proteasome Inhibitor and an IMiD (EDMS-ERI-92399922; de Weers et al., (2011) J Immunol 186(3): 1840-1848).

These assessments included: overall response rate (ORR), duration of response, time to response and best response, clinical benefit rate, time to progression (TTP), progression free survival (PFS), and overall survival (OS).

A total of 124 subjects were treated with DARZALEX™ (daratumumab) in this study (de Weers et al., (2011) J Immunol 186(3): 1840-1848). 18 subjects were treated with 8 mg/kg and 106 subjects were treated with 16 mg/kg. The dosing schedule was as follows:

Group A: DARZALEX™ (daratumumab) 16 mg/kg: Cycles 1 and 2: Days 1, 8, 15, and 22 (weekly), Cycle 3 to 6: Days 1 and 15 (every other week), Cycles 7+: Day 1 (every 4 weeks). Each cycle was 4 weeks.
Group B: DARZALEX™ (daratumumab) 8 mg/kg: Cycle 1+: Day 1 (every 4 weeks).

The primary objective of the study was to determine the efficacy of 2 treatment regimens of DARZALEX™ (daratumumab), as measured by the ORR (CR + PR), in subjects with multiple myeloma who have received at least 3 prior lines of therapy including a PI and an IMiD or whose disease is double refractory to both a PI and an IMiD (Clinical Study Report: An Open-label, Multicenter, Phase 2 Trial Investigating the Efficacy and Safety of DARZALEX™ (daratumumab) in Subjects With Multiple Myeloma Who Have Received at Least 3 Prior Lines of Therapy (Including a Proteasome Inhibitor and IMiD) or are Double Refractory to a Proteasome Inhibitor and an IMiD. EDMS-ERI-92399922).

The secondary objectives of this study included evaluation of the safety and tolerability of DARZALEX™ (daratumumab) demonstration of additional measures of efficacy (e.g, clinical benefit, TTP, PFS, and OS) along with assessment of pharmacokinetics, immunogenicity, pharmacodynamics, and to explore biomarkers predictive of response to DARZALEX™ (daratumumab). Additional study related information is available from the clinical study protocol (Clinical Study Report: An Open-label, Multicenter, Phase 2 Trial Investigating the Efficacy and Safety of DARZALEX™ (daratumumab) in Subjects With Multiple Myeloma Who Have Received at Least 3 Prior Lines of Therapy (Including a Proteasome Inhibitor and IMiD) or are Double Refractory to a Proteasome Inhibitor and an IMiD. EDMS-ERI-92399922).

In Stage 1 of Part 1, 1 subject (6%) responded in the 8 mg/kg group, and 5 subjects (31%) responded in the 16 mg/kg group. Therefore, only the 16 mg/kg group was expanded in Stage 2 of Part 1 and in Part 2.

In the 16 mg/kg group, 31 subjects achieved response of PR or better based on IRC assessment; the ORR was 29% (95% CI: 21%, 39%). Three subjects (3%) achieved sCR, and 13 subjects (12%) achieved VGPR or better.

**Example 3. Effect of DARZALEX™ (daratumumab) on T-cell expansion and activity in patients enrolled in the 54767414MMY2002 study (SIRIUS)**

CD38 is expressed on a variety of immune and hematopoietic cells. Broad immune profiling by flow cytometry was performed to examine the effect of DARZALEX™ (daratumumab) on immune cell subsets and the association of baseline levels of these cells to clinical response. Various cell populations, including T-cells (CD3$^+$, CD4$^+$, CD8$^+$ and regulatory T-cells (Treg)), B-cells (CD19$^+$), NK cells, monocytes (CD14$^+$), leukocytes, and neutrophils were evaluated by flow cytometry in peripheral blood and bone marrow aspirates at baseline and following DARZALEX™ (daratumumab) treatment to monitor for changes in these cellular populations in responders and non-responders.

**Lymphocytes, Leukocytes, Monocytes and Neutrophils**

Leukocyte, lymphocyte, monocyte, and neutrophil counts were studied in peripheral blood in responders and non-responders. Total lymphocytes were found increased with DARZALEX™ (daratumumab) treatment in responders with both 8 mg/kg and 16 mg/kg dose **(Figure 1).** Linear mixed effect modeling revealed an increase of $0.8 \times 10^6$ cells/$\mu$L on log scale per 100 days (CI = 0.06, 0.11). Slight increases were found for monocytes and leukocytes with significant increase of $0.03 \times 10^6$ cells/$\mu$L (CI = 0.01, 0.04) and $0.03 \times 10^6$ cells/$\mu$L on log scale (CI = 0.01, 0.05) for each 100 days respectively. Median neutrophil counts were consistent with baseline and did not vary significantly, although there was neutropenia noted for some patients.

Baseline levels of each of these cellular populations were compared between response groups. No evidence was found for baseline levels to be different for any of these cell types across response groups using Wilcoxon signed-rank test (Table 1).

**Table 1.**

| Peripheral blood cell counts in responders vs non-responders at baseline | | | | | |
|---|---|---|---|---|---|
| | N | Median | Mean (SD) | Range | P-value* |
| Leukocytes: R | 33 | 4.3 | 4.32 (1.65) | (1.6; 8.8) | |
| Leukocytes: NR | 82 | 4.19 | 4.77 (2.26) | (2.13; 13.8) | 0.60987 |
| Lymphocytes: R | 33 | 0.9 | 1.09 (0.59) | (0.27; 2.67) | |
| Lymphocytes: NR | 82 | 1 | 1.05 (0.55) | (0.3; 2.8) | 0.85028 |
| Monocytes: R | 33 | 0.43 | 0.5 (0.25) | (0.2; 0.97) | |
| Monocytes: NR | 82 | 0.5 | 0.51 (0.25) | (0.04; 1.3) | 0.72803 |
| Neutrophils: R | 33 | 2.47 | 2.54 (1.23) | (1.06; 5.94) | |
| Neutrophils: NR | 82 | 2.44 | 3.05 (2.08) | (1; 11.7) | 0.40373 |
| N: number of samples per group | | | | | |
| R: responder | | | | | |
| NR: non-responder | | | | | |

(continued)

| Peripheral blood cell counts in responders vs non-responders at baseline | | | | | |
|---|---|---|---|---|---|
| | N | Median | Mean (SD) | Range | P-value* |
| *non-responder vs responder | | | | | |
| SD: standard deviation | | | | | |

**NK cells**

Total NK cells (CD16$^+$CD56$^+$) and activated NK cells (CD16$^+$CD56$^{dim}$) were reduced with DARZALEXTM (daratumumab) treatment over time (data not shown).

**B-cells**

Absolute counts of B-cells (CD45$^+$CD3$^-$CD19$^+$) were measured in peripheral blood or bone marrow aspirates during DARZALEX$^{™}$ (daratumumab) treatment over time in responders and non-responders. B-cells slightly increased in the whole blood and were maintained in the bone marrow aspirates. Linear mixed modeling of B-cells in peripheral blood revealed a minimal increase of $0.1 \times 10^6$ cells/$\mu$l [CI=0.04, 0.16 for each 100 days on log scale over the course of DARZALEX$^{™}$ (daratumumab) treatment. There were no changes to the percentages of B-cells (CD45$^+$CD3$^-$CD19$^+$/Lymphocytes) in the bone marrow aspirates during daratumumab treatment, in either responders or non-responders (p = 0.1 and 0.4, respectively). Further, no evidence was found for B-cell counts to be different at baseline between responders and non-responders (p = 0.5).

**T-cells**

Lymphocytes were noted to increase with DARZALEX$^{™}$ (daratumumab) treatment **(Figure 1)** even though B cells showed only a minimal increase (see above). To investigate further, various T-cell populations were studied (CD3$^+$, CD4$^+$, CD8$^+$ T cells, regulatory T cells) in both peripheral blood and bone marrow.

CD3$^+$, CD4$^+$ and CD8$^+$T-cells were increased in peripheral blood (both absolute counts/$\mu$l and percentage of lymphocytes) following DARZALEX$^{™}$ (daratumumab) treatment. **Figure 2** shows the percent change of absolute counts of CD3$^+$ T-cells (CD45$^+$CD3$^+$) from baseline in peripheral blood over time for every patient. The black line in the Figure shows the median absolute counts $\times 10^6$ cells/$\mu$L for all patients. Only visits with more than 2 observations were included into the Figure. **Figure 3** shows the % change of absolute counts of CD4$^+$ T-cells (CD45$^+$CD3$^+$CD4$^+$) from baseline in peripheral blood over time for every patient. The black line in the Figure shows the median for all patients. Only visits with more than 2 observations were included into the Figure. **Figure 4** shows the % change of absolute counts of CD8$^+$ T-cells (CD45$^+$CD3$^+$CD8$^+$) from baseline in peripheral blood over time for every patient. The black line in the Figure shows the median for all patients. Only visits with more than 2 observations were included into the Figure. Linear mixed modeling on absolute counts in peripheral blood revealed on average total T-cell (CD45$^+$CD3$^+$) increase of $0.13 \times 10^6$ cells/$\mu$l on log scale for each 100 days (CI = 0.1, 0.15 ) following DARZALEX$^{™}$ (daratumumab) treatment. CD8$^+$ T-cells were found to significantly increase by $0.16 \times 10^6$ cells/$\mu$l on log scale for each 100 days (CI =0.13, 0.19). CD4$^+$ cells were found to have a moderate increase of $0.11 \times 10^6$ cells/$\mu$l on log scale for each 100 days (CI = 0.09, 0.13).

For each of the T-cell subpopulations, responders showed a higher maximum percent change of absolute counts to baseline than non-responders (CD3$^+$ p=3.2993e-05; CD4$^+$ p=3.486e-05; CD8$^+$ p=2.7172e-05; regulatory T cell p=0.002). **Table 2** shows the Wilcoxon signed-rank test results for the comparison of each T-cell subpopulation in peripheral blood between responders and non-responders for percent change of absolute counts to baseline.

**Table 2.**

| Percent change of absolute cell counts; peripheral blood | | | | | |
|---|---|---|---|---|---|
| Sample | N | Median | Mean (SD) | Range | P-value* |
| CD45$^+$CD3$^+$: R | 33 | 86.76 | 118.91 (104.07) | (-16.1; 398.71) | |
| CD45$^+$CD3$^+$: NR | 80 | 28.08 | 43.02 (69.55) | (-67.11; 286.67) | 3.30E-05 |
| CD45$^+$CD3$^+$CD4$^+$: R | 33 | 72.08 | 77.74 (60.99) | (-21.05; 233.21) | |
| CD45$^+$ CD3$^+$CD4$^+$: NR | 80 | 19.48 | 29.36 (59.58) | (-68; 298.89) | 3.49E-05 |

(continued)

| Percent change of absolute cell counts; peripheral blood | | | | | |
|---|---|---|---|---|---|
| Sample | N | Median | Mean (SD) | Range | P-value* |
| CD45+ CD3+CD8+: R | 33 | 106.6 | 180.81 (192.37) | (-7.07; 760.51) | |
| CD45+ CD3+CD8+: NR | 80 | 32.24 | 63.96 (112.44) | (-66.22; 588.89) | 2.72E-05 |
| N: number of samples per group | | | | | |
| R: responder | | | | | |
| NR: non-responder | | | | | |
| *responder vs. non-responder | | | | | |
| SD: standard deviation | | | | | |

Similarly in bone marrow, total T-cells (CD45+CD3+ as a percentage of lymphocytes) and CD8+ T-cells (CD45+CD3+CD8+ as a percentage of lymphocytes) were found to significantly increase during DARZALEX™ (daratumumab) treatment, for both responders and non-responders (CD3+ responders p=3.8147e-06, non-responders p=9.8225e-05; CD8+ responders p=3.8147e-06, non-responders p=0.0003). There was no change in median CD4+ T-cells in either clinical response group in bone marrow. **Table 3** shows the Wilcoxon signed-rank test results for the various T cells as % lymphocytes in bone marrow. **Figure 5** shows the percentage (%) of CD45+CD3+ cells over time during DARZALEX™ (daratumumab) treatment (both responders and non-responders included in the graph). **Figure 6** shows the % CD45+CD3+CD8+ cells over time during DARZALEX™ (daratumumab) treatment (both responders and non-responders included in the graph).

**Table 3.**

| T cell populations (% lymphocytes) in bone marrow | | | | | |
|---|---|---|---|---|---|
| Sample | | NR: Baseline | NR: On treatment | R: Baseline | R: On treatment |
| CD45+CD3+/ Lymphocytes | N | 29 | 29 | 19 | 19 |
| | Median | 72.2 | 83.6 | 77.9 | 91.4 |
| | Mean (SD) | 68.57 (13.64) | 80.93 (11.57) | 71.82 (14.92) | 87.67 (9.49) |
| | Range | (36.3; 94.5) | (50.9; 97.4) | (42.2; 94.8) | (63.3; 97.2) |
| | P-value* | | 9.8225e-05 | | 3.8147e-06 |
| CD45+CD3+CD4+/ Lymphocytes | N | 29 | 29 | 19 | 19 |
| | Median | 33.7 | 29.2 | 22.7 | 22.8 |
| | Mean (SD) | 31.24 (12.14) | 32.96 (12.57) | 24.18 (7.37) | 24.29 (9.58) |
| | Range | (6.3; 54.2) | (9.6; 60.9) | (8.1; 36.6) | (12.5; 45.4) |
| | P-value* | | 0.18351 | | 0.98432 |
| CD45+CD3+CD8+/ Lymphocytes | N | 29 | 29 | 19 | 19 |
| | Median | 36.3 | 43.3 | 49.4 | 66.9 |
| | Mean (SD) | 37.39 (13.64) | 47.74 (18.14) | 46.91 (14.89) | 62.82 (12.79) |
| | Range | (15.9; 67.2) | (18.5; 81) | (24.5; 79.6) | (33.1; 83.3) |
| | P-value* | | 0.00026883 | | 3.8147e-06 |
| N: number of samples per group | | | | | |
| R: responder | | | | | |
| NR: non-responder | | | | | |
| *baseline vs. on treatment for responder or non-responder group | | | | | |

(continued)

| T cell populations (% lymphocytes) in bone marrow | | | | | |
|---|---|---|---|---|---|
| Sample | | NR: Baseline | NR: On treatment | R: Baseline | R: On treatment |
| SD: standard deviation | | | | | |

While both responders and non-responders demonstrated T-cell increases in the peripheral blood and bone marrow, responders had the largest percentage change from baseline. To distinguish whether responders or non-responders had different levels of CD3$^+$, CD4$^+$ and CD8$^+$ T-cells prior to DARZALEX™ (daratumumab) treatment, baseline measurements of each subgroup were compared in the peripheral blood.

There were no statistically significant differences between responders and non-responders in absolute T-cell counts at baseline in peripheral blood (Table 4) or in percentage of T cells from total lymphocytes in bone marrow (Table 5), Wilcoxon signed-rank test.

**Table 4.**

| Absolute cell counts in peripheral blood prior to treatment at baseline | | | | | |
|---|---|---|---|---|---|
| Sample | N | Median | Mean (SD) | Range | P-value* |
| CD45$^+$CD3$^+$: R | 33 | 574 | 715.91 (472.54) | (186; 2096) | |
| CD45$^+$CD3$^+$: NR | 80 | 638 | 672.5 (426.36) | (85; 2407) | 0.81527 |
| CD45$^+$CD3$^+$CD4$^+$: R | 33 | 190 | 276.91 (207.39) | (77; 1085) | |
| CD45$^+$CD3$^+$CD4$^+$: NR | 80 | 214 | 251.61 (146.13) | (21; 766) | 0.94965 |
| CD45$^+$CD3$^+$CD8$^+$: R | 33 | 332 | 424.55 (324.49) | (93; 1238) | |
| CD45$^+$CD3$^+$CD8$^+$: NR | 80 | 318 | 398.14 (354.52) | (43; 2221) | 0.56555 |
| N: number of samples per group | | | | | |
| R: responder | | | | | |
| NR: non-responder | | | | | |
| *responder vs non-responder per cell type | | | | | |
| SD: standard deviation | | | | | |

**Table 5.**

| T cells (% lymphocytes) in bone marrow prior to treatment | | | | | |
|---|---|---|---|---|---|
| Sample | N | Median | Mean (SD) | Range | P-value |
| CD45$^+$CD3$^+$: R | 23 | 78.4 | 73.66 (14.43) | (42.2; 94.8) | |
| CD45$^+$CD3$^+$: NR | 65 | 76.5 | 73.5 (13.93) | (36.3; 94.5) | 0.81232 |
| CD45$^+$CD3$^+$CD4$^+$: R | 23 | 25 | 25.57 (8.32) | (8.1; 41.4) | |
| CD45$^+$CD3$^+$CD4$^+$: NR | 65 | 25.3 | 27.53 (12.76) | (6.3; 55.2) | 0.76482 |
| CD45$^+$CD3$^+$CD8$^+$: R | 23 | 50 | 47.7 (13.73) | (24.5; 79.6) | |
| CD45$^+$CD3$^+$CD8$^+$: NR | 65 | 44.3 | 44.73 (15.49) | (15.9; 76. 1) | 0.41678 |
| N: number of samples per group | | | | | |
| R: responder | | | | | |
| NR: non-responder | | | | | |
| *responder vs non-responder per cell type | | | | | |

(continued)

| T cells (% lymphocytes) in bone marrow prior to treatment | | | | | |
|---|---|---|---|---|---|
| Sample | N | Median | Mean (SD) | Range | P-value |
| SD: standard deviation | | | | | |

**T regulatory cells**

Treg cells were identified as the $CD3^+CD4^+CD25^+CD127^{dim}$ cell population in a sample. The ratio of $CD8^+$ T cells to Tregs was assessed in the peripheral blood and bone marrow in patients treated with DARZALEX™ (daratumumab) over time. The ratio increased in both the periphery and bone marrow. **Figure 7A** shows the median values of the $CD8^+$/Treg and $CD8^+$/$CD4^+$ cell ratios of all patients per time point in peripheral blood. **Figure 7B** shows the median values of the $CD8^+$/Treg and $CD8^+$/$CD4^+$ T-cell ratios of all patients per time point in bone marrow. The changes in the ratios of absolute counts of $CD8^+$Tregs and $CD8^+$/$CD4^+$ were significant in peripheral blood over time of treatment **(Table 6)** and in bone marrow **(Table 7),** Wilcoxon signed-rank test.

In a combined data analyses of SIRIUS and GEN501 study (Example 6), median ratios of $CD8^+$/$CD4^+$ and $CD8^+$/Treg cells in peripheral blood were increased at week 8 (p = $5.1{\times}10^{-5}$ for $CD8^+$/$CD4^+$ and p = $1.8{\times}10^{-7}$ for $CD8^+$/Treg) and at week 16 (p=0.00017 for $CD8^+$/$CD4^+$ and p = $4.1{\times}10^{-7}$ for$CD8^+$/Treg ). Similarly, in bone marrow, median ratios of $CD8^+$/$CD4^+$ and $CD8^+$/Treg cells were increased on treatment (week 12 $\pm$ 1 cycle) compared to baseline (p = 0.00016 for $CD8^+$/$CD4^+$ and p = $2.8{\times}10^{-7}$ for $CD8^+$/Treg). No significant differences were observed between responders and nonresponders.

**Table 6.**

| T cell ratios in peripheral blood | | | | | |
|---|---|---|---|---|---|
| Sample | N | Median | Mean (SD) | Range | P-value* |
| $CD8^+$/$CD4^+$: Baseline | 66 | 119.75 | 191.78 (231.09) | (24.17; 1461.18) | |
| $CD8^+$/$CD4^+$: C3D1 | 66 | 204.86 | 222.96 (167.44) | (25.53; 867.58) | 0.00046409 |
| $CD8^+$/$CD4^+$: C4D1 | 66 | 210.05 | 215.15 (151.31) | (25.86; 798.83) | 0.00042154 |
| $CD8^+$/Tregs: Baseline | 66 | 1258.33 | 2338.46 (3465.12) | (206.82; 18550) | |
| $CD8^+$/Tregs: C3D1 | 66 | 2326.74 | 3361.87 (3661.61) | (155; 23066.67) | 5.25E-06 |
| $CD8^+$/Tregs: C4D1 | 66 | 2763.16 | 3382.86 (3629.69) | (316.67; 22087.5) | 9.95E-08 |
| *comparison to baseline; N: number of samples per group; SD: standard deviation | | | | | |

**Table 7.**

| T cell ratios in bone marrow | | | | | |
|---|---|---|---|---|---|
| Sample | N | Median | Mean (SD) | Range | P-value* |
| $CD8^+$/$CD4^+$(/Lymphocytes): Baseline | 31 | 163.18 | 184.4 (129.5) | (32.58; 674.58) | |
| $CD8^+$/$CD4^+$(/Lymphocytes): On treatment | 31 | 221.89 | 240.85 (155.57) | (30.38; 666.4) | 0.0038599 |
| $CD8^+$/Tregs(/Lymphocytes): Baseline | 30 | 1219.58 | 1802.73 (1582.7) | (306.41; 7960) | |
| $CD8^+$/Tregs(/Lymphocytes): On treatment | 30 | 2273.56 | 3905.72 (4232.73) | (451.22; 20825) | 3.15E-07 |
| *comparison to baseline; N: number of samples per group; SD: standard deviation | | | | | |

**Example 4. Study design (GEN501)**

Study GEN501 (NCT00572488) evaluated DARZALEX™ (daratumumab) as monotherapy in double-refractory MM patients. Sample isolation, processing and statistical analyses was as described in Example 1 and Example 2. The study has been described in Lokhorst et al., N Eng J Med 373:1207-19, 2005.

Briefly, Study GEN501 was the first-in-human clinical study of DARZALEXTM (daratumumab) in subjects with MM. It is a Phase 1/2, dose-escalation, safety study divided into 2 parts. Part 1 is an open-label, dose-escalation study; Part 2 is an open-label, single-arm study with multiple cohorts, based on the dose levels established in Part 1

In Part 1, 10 dose levels of DARZALEX™ (daratumumab) were evaluated: 0.005, 0.05, 0.10, 0.50, 1, 2, 4, 8, 16, and 24 mg/kg. The 2 lowest dose cohorts were allocated 1 (+3) subject(s) each, and a standard 3 (+3) subject allocation was applied to the remaining 8 dose cohorts. Part 2 was an open-label, single study including two dose levels, 8 mg/kg and 16 mg/kg. Part 1 included 32 subjects and Part 2 included 72 subjects.

**Example 5. DARZALEX™ (daratumumab) treatment induces T cell clonality in patients**

Given the expansion of $CD8^+$ T-cells noted in both the periphery and the bone marrow in the MY2002 study, high throughput next-generation sequencing of the T-cell receptor (TCR) was performed using the Immunoseq™ assay to determine whether expanding $CD8^+$ T-cells were clonal in nature, indicative of an adaptive immune response. Total of 17 patient samples of subjects who were enrolled in the GEN501 study were evaluated (n=6 responders *i.e.,* $\geq$ PR; n=11 non-responders *i.e.,* MR, SD, PD).

TCR sequencing revealed that DARZALEX™ (daratumumab) treatment significantly increased clonality across patients. **Figure 8A** shows the correlation between T cell clonality pre- vs. post- DARZALEX™ (daratumumab) treatment (p = 0.0056). **Figure 8B** shows the fold change in clonality in individual patients. Responders are marked with the star. This data suggests that the T cell expansion noted with DARZALEX™ (daratumumab) treatment may be clonal in nature.

Responders had a greater total expansion in the TCR repertoire (as measured by change in abundance; CIA) when compared to non-responders. **Figure 8C** shows the % CIA for individual patients. Group A: responders, Group B: non-responders. Statistically significant difference was observed between responders and non-responders (p=0.037). **Figure 8D** shows the sum of absolute change in abundance (CIA) in responders and non-responders for each expanded T cell clone. **Figure 8E** shows the maximum % CIA for each individual patient. Group A: responders, Group B: non-responders. Statistically significant difference was observed between responders and non-responders (p=0.048). **Figure 8F** shows the maximum CIA of a single T-cell clone in responders (Group A) and non-responders (Group B).

CIA was obtained by identifying significant differences in clonal abundance between two samples using Fisher's exact test (DeWitt et al. J. Virol. 2015) and summing the absolute change in abundance for each expanded clone.

**Example 6. Immunomodulatory effects of DARZALEX™ (daratumumab) in patients enrolled in the GEN501 study**

Various T and B cell populations were evaluated in responders and non-responders enrolled in the GEN501.

**Lymphocytes**

Similar to SIRIUS (MMY2002) study, lymphocytes were increased in both peripheral blood and bone marrow during DARZALEX™ (daratumumab) treatment. This increment was attributed to increased numbers of both $CD4^+$ and $CD8^+$ cells.

**$CD8^+$ central memory cells**

$CD8^+$ T-cell phenotype was studied in patients treated with DARZALEX™ (daratumumab) over time in a subset of 17 patients enrolled in the GEN 501 study. $CD8^+$ cells from patients were identified as naïve (CD45RO-/$CD62L^+$) ($T_N$) or central memory ($T_{CM}$) (CD45RO$^+$/$CD62L^{+high}$) cells using standard protocols.

**Figure 9A** shows the % of $CD8^+$ naive cells (% of $CD8^+$ cells) and **Figure 9B** shows the % of $CD8^+$ central memory cells. DARZALEX™ (daratumumab) treatment significantly decreased the quantity of naive $CD8^+$ T cells (*p* = $1.82 \times 10^{-4}$ at Week 8) and increased the quantity of $CD8^+$ memory T cells (*p* = $4.88 \times 10^{-2}$ at Week 8). This would suggest a transition from naive cytotoxic T cells to memory T cells which may be activated against a specific antigen. White squares indicate patients that achieved at least a minimal response ($\geq$ MR) and black squares indicate patients that had stable disease or progressive disease. A significantly greater decrease in $CD8^+$ naive T cells was apparent in patients who responded to treatment (data not shown). **Figure 9C** shows that DARZALEX™ (daratumumab) treatment increased the percentage in HLA Class I-restricted T cells, which partially drive the virus-specific and alloreactive T cell responses.

**Figure 9D** shows that the expanding effector memory T cells expressed low levels of CD38. It is important to note that these T cells display normal and even increased functional activity against viral peptides and alloantigens (see **Example 8**). From these functional results we concluded that there is an expansion of, or improved activity of, antigen-experienced T cells against viral and alloantigens during DARZALEX™ (daratumumab) treatment. These data suggest that, unlike regulatory cell subsets, effector T cells do not need CD38 expression to properly function and expand.

**CD38-positive Regulatory T-cells**

The observation of the robust expansion and increased activity of cytotoxic T-cells together with recent literature indicating that several immune-suppressive cell subsets express CD38 prompted examination of the effects of DARZALEX™ (daratumumab) on regulatory cell populations regulatory T-cells (Tregs), myeloid derived suppressor cells (MDSCs) and regulatory B-cells (Bregs).

Regulatory T-cells (Tregs) ($CD3^+CD4^+CD25^+CD127^{dim}$) were isolated using standard protocols. The frequency of the Tregs was analyzed using flow cytometry.

A subpopulation of peripheral Tregs (10% $\pm$ 10%) expressed high levels of CD38 prior to Treg activation. **Figure 10A,** top panel shows the frequency of the Tregs in the $CD3^+CD4^+$ cell population (P4 cell population) at baseline. **Figure 10A,** bottom panel shows the subset of Tregs expressing high CD38 (P5 cell population). These $CD38^+$ Tregs were highly sensitive to DARZALEX™ (daratumumab) treatment and exhibited a significant and almost immediate decline following the first dose of DARZALEX™ (daratumumab) (n = 17 patients; $P = 8.88 \times 10^{-16}$ at Week 1 versus baseline). The frequency of Tregs after DARZALEX™ (daratumumab) treatment is shown in **Figure 10B,** top panel (P4 cell population). **Figure 10B,** bottom panel shows that the $CD38^{high}$ Tregs (P5 cells) was the most significantly depleted Treg population after $1^{st}$ DARZALEX™ (daratumumab) infusion. These $CD38^+$ Tregs remained depleted throughout DARZALEX™ (daratumumab) treatment ($p = 8.88 \times 10^{-16}$, $1.11 \times 10^{-15}$, and $1.50 \times 10^{-11}$ at Weeks 1, 4, and 8, respectively, versus baseline. **Figure 10C** shows the % of $CD38^{high}$ Tregs from total $CD3^+$ cells at baseline, week 1, week 4, week 8, relapse, and 6 months after the end of treatment (EOT). The $CD38^{high}$ Tregs were recovered to the baseline at that time point. Changes in $CD38^+$ Tregs were similar between patients who did and did not respond to treatment however, the $CD8^+$ T-cell:Treg ratio was significantly higher at Week 8 in patients who showed a response to DARZALEX™ (daratumumab) ($P = 0.00955$; **Figure 10D**).

To assess the possible biological relevance of depletion of $CD38^+$ Tregs with DARZALEX™ (daratumumab) treatment, the suppressive capacity of $CD38^+$ Tregs versus $CD38^-$ Tregs on autologous $CD3^+$ T cells was assessed. In a series of experiments performed with sample from multiple heathy donors, $CD38^+$ Tregs suppressed T-cell proliferation more robustly (9.9% cell proliferation observed) than $CD38^-$ Tregs (53.2% cell proliferation observed) or the negative control (74.9% cell proliferation observed) **(Figure 10E).**

Since MDSCs were not readily detectable in frozen PBMC samples, $CD38^+$ granulocytic MDSCs ($CD11b^+CD14^-HLA-DR-CD15^+CD33^+$) were generated *in vitro* from PBMCs isolated from patients at baseline and from patients who had received one infusion of DARZALEX™ (daratumumab). **Figure 11** shows the flow cytometry histogram of identified MDSCs (**Figure 11,** top histogram, boxed cell population). Approximately half of the MDSCs expressed CD38 (**Figure 11**, middle graph; circled P7 cell population). The $CD38^{high}$ MDSCs were nearly depleted in patients treated with DARZALEX™ (daratumumab) (**Figure 11**, bottom graph; circled P7 cell population).

The $CD38^{high}$ lineage nonspecific MDSCs were depleted with DARZALEX™ (daratumumab) treatment over time in both non-responders and patients who have at least Minimal Repose to treatment. **Figure 12** shows that the percentage of the $CD38^{high}$ MDSCs was reduced to nearly 0% in patients at 1 week, 4 weeks or 8 weeks of treatment. The $CD38^{high}$ lineage nonspecific MDSCs returned to baseline after the end of treatment.

Patients with the largest $CD38^+$ populations within lineage nonspecific MDSC's demonstrated the best and most durable responces to DARZALEX™ (daratumumab) treatment. **Figure 13** shows that the patients 2, 4, 15, 16 and 17 having the highest percentage of $CD38^{high}$ MDSC (as shown in Figure 11) and classified as patients with PR or MR, had a Progression-Free Survival (PFS) of at least 8 months.

The $CD38^{high}$ lineage nonspecific MDSCs were also sensitive to DARZALEX™ (daratumumab)-induced ADCC *in vitro.* ADCC assays were performed using $CD38^{high}$ MDSCs from two donors and Daudi cells as control target cells with effector:target cell ratio of 50:1. **Figure 14** shows the results of the experiment from one donor. DARZALEX™ (daratumumab) induced lysis of MDSC cells.

$CD38^+$ Bregs were measured in DARZALEXTM (daratumumab)-treated patients (n = 16) and, similar to $CD38^+$ Tregs, were depleted following the first dose of DARZALEXTM (daratumumab) ($p = 0.0018$ at Week 1 compared with baseline; paired Wilcoxon rank test) and remained low while patients were on treatment **(Figure 15A).** The FACS sorted Bregs, when stimulated, produced IL-10 **(Figure 15B).**

Collectively, these observations suggest that the depletion of immunosuppressive $CD38^+$ MDSCs, Bregs, and Tregs is a significant contributory mechanism to DARZALEX™ (daratumumab)-induced changes in T-cell populations and clonality.

**Example 7. CD38⁺ MDSC cells are present in cancer patients**

Percentage of MDSC (Lin⁻CD14⁻HLADR^low/-)) and their CD38 expression was studied in in the peripheral blood of patients with NSCLC or prostate cancer using flow cytometry.

The percentage of MDSCs was between about 10%-37% and between about 10%-27% of PBMCs in the analyzed samples from the NSCLC and prostate cancer patients, respectively. CD38 expression was identified in 80-100% of Lin⁻CD14⁺HLADR^-/low MDSCs from PBMCs from NSCLC patients and in 70-100% of MDSCs from PBMCs from prostate cancer patients.

**Example 8. DARZALEX™ (daratumumab) enhances antiviral T-cell responses**

To further assess the effect of DARZALEX™ (daratumumab) on T-cell activation and functionality, IFN-γ production from peripheral T cells in response to viral and alloantigens was measured in DARZALEX™ (daratumumab)-treated patients (n = 7) with a range of clinical outcomes. Patients with a PR or better demonstrated significant increases in IFN-γ secretion in response to viral and alloantigens following DARZALEX™ (daratumumab) treatment, compared with baseline, for at least one time point during treatment, suggesting that T cell function is not impaired by low CD38 expression (see Example 6, Figure9C). Similar to the TCR clonality data, this increase was more marked in patients who responded to DARZALEX™ (daratumumab) than those who did not. **Figure 16A** shows the anti-viral response of one representative patient with VGPR. **Figure 16B** shows the anti-viral response of one representative patient with CR. **Figure 16C** shows the anti-viral response of one representative patient with PD. **Figure 16D** shows the anti-viral response of one representative patient with MR. In the Figures, error bars represent standard error of the mean of duplicate cultures. Asterisk denotes statistically significant changes between the indicated comparisons. Best response per Independent Review Committee is shown. Consistent with these results, virus-reactive T-cells in patients with VGPR (Figure 16E) or CR (Figure 16F) demonstrated an increase in proliferative capacity during DARZALEX™ (daratumumab) treatment.

**Example 9. Mechanism of sensitivity of CD38 expressing immune cell subtypes to DARZALEX™ (daratumumab)**

Data from both GEN501 and SIRIUS studies indicated that some immune cells that express CD38 are depleted (NK cells, regulatory T-cells (Tregs), regulatory B-cells (Bregs), and myeloid derived suppressor cells (MDSCs)), while others that express CD38 increase in number (cytotoxic and helper T cells) with DARZALEX™ (daratumumab) therapy.

To address the mechanism of sensitivity, expression levels of CD38 were assessed in various subpopulations of immune cells in healthy donors and in multiple myeloma patients enrolled in either GEN501 or SIRIUS study. **Figure 17A** shows a histogram of expression of CD38 in immune cells from a healthy donor, and **Figure 17B** shows a histogram of expression of CD38 in immune cells from a multiple myeloma patient. In a healthy donor, CD38 expression was highest on NK cells, followed by monocytes, Band T cells. In a multiple myeloma patient, CD38 expression was highest on plasma cells, followed by a subset of B cells, NK cells, monocytes, B-cells and T-cells. **Figure 17C** shows a comparison of the mean fluorescent intensity (MFI) of CD38 across NK cells, Tregs, Bregs, B- and T-cells cells from relapsed and refractory myeloma patients, demonstrating that after plasma cells, NK cells expressed the highest levels of CD38, followed by regulatory T-cells (Tregs) and regulatory B-cells (Bregs).

In addition to CD38 expression, other cell surface proteins such as complement inhibitory proteins (CIPs; CD46, CD55, CD59) may contribute to sensitivity or resistance to DARZALEX™ (daratumumab). *In vitro* evaluation of CIPs across immune cell subpopulations found that NK cells express very low levels of CD59 and CD55, while other T and B cell populations express much higher levels. This could also contribute to the variability of DARZALEX™ (daratumumab) sensitivity across immune cell subtypes (data not shown).

**Discussion**

This study describes previously unknown immunomodulatory effects of DARZALEX™ (daratumumab) through reduction of CD38⁺ immune suppressive cellular populations and concomitant induction of helper and cytotoxic T-cell expansion, production of IFN-γ in response to viral peptides, and increased TCR clonality, indicating an improved adaptive immune response.

This study demonstrates that MDSCs and Bregs express CD38 and were susceptible to DARZALEX™ (daratumumab) treatment. These cells are known to be present in the tumor microenvironment and contribute to tumor growth, immune evasion, angiogenesis, metastasis, and production of suppressive cytokines. In addition to these CD38⁺ suppressive cellular subsets, a novel subpopulation of regulatory T cells (CD4⁺CD25⁺CD127^dim) was identified that also expressed high levels of CD38 and demonstrated superior autologous T-cell suppressive capacities. These cells were also sensitive to DARZALEX™ (daratumumab) and were significantly reduced in patients receiving treatment. DARZALEX™ (daratumumab)-mediated elimination of these CD38+ immune-regulatory cells may reduce local immune suppression within the myeloma microenvironment and allow positive immune effector cells to expand and contribute to antitumor response.

Indeed, significant increases in broad T-cells populations, including both CD4+ and CD8+, were observed in both peripheral blood and within bone marrow (i.e., the tumor). Specific CD8+ subpopulations were altered with DARZALEX™ (daratumumab) therapy, including significant decreases in naive T-cells and concomitant significant increases in effector memory CD8+ T-cells, indicating a shift in effector T-cells towards an antigenic experienced phenotype that retained immunological memory and may be reactive against tumor antigens. Ratios of CD8+:CD4+ and CD8+:Tregs also increased significantly with treatment, demonstrating a shift in positive versus negative immune regulators.

To evaluate whether expanded CD4+ and CD8+ T-cells were clonal in nature, the T-cell repertoire was examined in a subset of patients. T-cell clonality significantly increased with DARZALEX™ (daratumumab) treatment, even in patients who had a best response of SD or who progressed. Therefore, increased T-cell clonality cannot be due simply to reduction in tumor burden. However, the skew in T-cell clonality was greater in patients with a good clinical response, and was correlated with the increase in CD8+ T-cells, suggesting the observed T-cell expansion with DARZALEX™ (daratumumab) treatment was antigen-driven. This is remarkable in this patient population, which was heavily pretreated (median of 5 prior lines of therapy) and not expected to be able to mount a strong antitumor immune response. In addition to increased TCR clonality, patients with a response to DARZALEX™ (daratumumab) demonstrated increased T-cell responses to preexisting viral- and alloantigens, suggesting the rescue of the immune system from an immunosuppressive state.

Treatment with DARZALEX™ (daratumumab) caused a reduction in immune suppressive MDSC and regulatory T- and B-cells. These reductions were concomitant with an expansion of CD4+ T-helper cells and CD8+ cytotoxic T-cells. T-cell clonality and functional anti-viral responses as measured by IFN-γ production also increased with DARZALEX™ (daratumumab) treatment. These observations indicate that T-cells continued to function properly, despite low CD38 expression, and suggest that increased T-cell response may be due to depletion of regulatory cells. Further, these changes in T-cell expansion, activity, and clonality were more pronounced in patients who responded to DARZALEX™ (daratumumab) compared with those who did not. Relapse from DARZALEX™ (daratumumab) therapy was associated with reversal of many of these changes. This suggests an additional, previously-uncharacterized mechanism of action of DARZALEX™ (daratumumab) through immunomodulation that may contribute to clinical responses and its efficacy.

Recently, antibodies that promote antitumor immune responses, rather than targeting the cancer directly, have demonstrated efficacy in a range of settings. Antibodies inhibiting CTLA-4 and PD-1 promote T-cell expansion and enhance T-cell activation, resulting in prolonged survival and delayed disease recurrence in patients with advanced solid tumors and hematologic malignancies such as Hodgkin lymphoma. By enhancing anticancer immunity, these immunomodulatory antibodies may not only induce clinical responses, but also prevent disease recurrence.

**Example 10. Serum proteomic analysis of multiple myeloma subjects traded with single-agent DARZALEX™ (daratumumab) in 54767414MMY2002 (SIRIUS) Part 2 clinical study**

**Biomarker sample collection and processing**

Peripheral blood samples were collected in standard serum separator tubes (2.5 mL to 5 mL) and serum aliquots were shipped frozen SomaLogic, Inc (Boulder, CO) for multianalyte serum protein profiling.

The serum protein profiling was performed at SomaLogic using a pre-validated SOMAscan assay that measures 1129 protein analytes by use of SOMAmer affinity based molecules. SOMAmer reagents are single stranded DNA-based protein affinity reagents. The assay uses small amounts of input sample (150 μL plasma) and converts the protein signal to a SOMAmer signal that is quantified by custom DNA microarray.

Each SOMAmer contains 4 functional moieties:

1. A unique protein recognition sequence
2. Biotin for capture
3. Photocleavable linker
4. Fluorescent molecule for detection

The unique protein recognition sequence uses DNA and incorporates chemically modified nucleotides that mimic amino acid side chains, expanding the diversity of standard aptamers and enhancing the specificity and affinity of protein-nucleic acid interactions (Gold et al., PLoS One 5:e15004, 2010) The aptamers are selected for by SELEX. SOMAmer reagents are selected using proteins in their native conformations. As such SOMAmer reagents require an intact, tertiary protein structures for binding. Unfolded or denatured presumably inactive proteins are not detected by SOMAmer reagents.

Master mixes of SOMAmer reagents are grouped for sample type and dilution. The reagents are pre-bound to streptavidin beads prior to sample incubation. Proteins in the samples are bound to the cognate SOMAmers during equilibrium, washed, incubated with NHS-biotin, washed and then the beads are exposed to UV light to cleave the photocleavable linker. The elution contains the SOMAmer reagents bound to their biotin labeled proteins. A streptavidin capture and subsequent washes removes the unbound SOMAmer reagents. In the final elution the SOMAmer molecules are released from their cognate proteins through denaturing conditions. The final eluate is hybridized to custom Agilent DNA microarrays and the fluorophore from the SOMAmer molecules it quantified by relative fluorescent units (RFU). The RFU is proportional to the amount of protein in the sample.

Samples from the MMY2002 study were tested in two primary batches. A first batch of 180 samples contained paired Cycle 1 Day 1 (C1D1, baseline) and C3D1 (Cycle 3 Day1) serum samples from 90 subjects. The 180 samples were analyzed together on 3 separate SomaScan plates. The second batch of samples includes 50 C1D1 samples, including 35 repeated samples from batch 1.

### Data analyses

### Input datasets and definitions

Treated subjects with an evaluable response were included in the data analysis. Consistently throughout the report, responders are defined as subjects with an overall best response (per IRC, for MMY2002) of sCR, VGPR, and PR, stable disease (SD) subjects as a subject with minimal response (MR) or SD, and non-responders are defined as subjects with an overall best response (per IRC, for MMY2002) of progressive disease (PD).

### Somalogic data pre-processing

### Batch alignment

Batches 1 and 2 of MMY2002 samples were tested on two different versions of the SOMAscan platform. Differences between the two versions were minor, and included three SOMAmer sequences that changed between the versions (CTSE: 3594-6_1 -> 3594-6_5, FCN1: 3613-62_1 -> 3613-62_5, BMPER: 3654-27_1 -> 3654-27_4). These were removed from the analysis.

The measurements of the three batch 1 plates were aligned according to SomaLogic's standard inter-plate calibration workflow, by defining plate-wide calibration scaling factors for each SOMAmer by calculating the ratio of a Master-mix specific global reference value to the median of 7 in-plate control calibrator measurements. The plate-specific scaling factor for each SOMAmer reagent was applied to each sample on the plate equivalently.

Given the different SOMAscan platform versions of batch 1 and 2, systematic inter-batch variability correction was done with a modified implementation of SomaLogic's standard inter-plate calibration workflow, by leveraging the repeated measurement of 35 samples across batches. For each SOMAmer the ratio of the batch 1 post-calibration measurement divided by the batch 2 pre-calibration measurement was calculated for each of the 35 repeated samples ($r_{i,j}$). The median of these 35 ratios was used to define the revised SOMAmer-specific calibration scaling factor for the batch 2 samples ($\tilde{r}_i$). These calibration scaling factors were then implemented identically to the standard SOMAscan procedure.

$$r_{i,j} = \left( \frac{\text{Post-Calib. Conc}_{\text{Batch 1},i,j}}{\text{Pre-Calib. Conc}_{\text{Batch 2},i,j}} \right), \quad \vec{r_i} = \left( r_{i,1}, r_{i,2}, \ldots, r_{i,j} \right) \quad \text{for all repeated samples } j$$

$$\text{Calibration Scaling Factor}_i = median(\vec{r_i}) = \tilde{r}_i$$

Once the revised calibration scaling factors were calculated, the distributions of all the scaling factors for each batch of the analysis were plotted to assess the presence of outliers. 9 SOMAmers with extremely large or small calibrators (> 0.25 and < 3) were removed from the analyses due to poor reproducibility.

After batch alignment and SOMAmer filtering was complete for MMY2002, a log2 transformation was applied to all protein concentration values of MMY2002 to bring the data more in line with a normal distribution and to improve the performance of parametric statistical tests.

**Confounding Variable Correction**

Estimation of the portion of dataset variance explained by meta-variables (like demographics, response class, and sample time point) and identification of potential confounding factors was performed by principal component analysis on the centered and scaled dataset. Simple linear models were fit to identify the highest ranked PC that was significantly associated with each of the variables of interest. The significance of these associations was determined using a Wald test and the fraction of the PC variability explained by the model was estimated by the R2 of the fit. For the MMY2002 data, site ID was found to be correlated with PC1 and explained the largest portion of dataset variability ($\geq$ 7.37%, p-value = $3.71 \times 10$-9). In order reduce the impact of sample acquisition site related effects within the data, ComBat28 was utilized to correct for site ID effects.

**Repeated Sample Merging**

The data of the 35 samples repeated between MMY2002 batches 1 and 2 was merged by calculating the mean for each protein.

**Differential Protein Concentration Analysis**

**Responders versus non-responders**

Statistical comparison of protein concentration distributions in DARZALEXTM (daratumumab) responders versus non-responders was performed at both baseline and on treatment using two complementary methods (i) Wilcoxon rank-sum test (Hollander and Wolfe, Ninparametirc Statistical Methods. New York: John Wiley & Sons. 1973. 27-33 (one-sample), 68-75 (two-sample) on each individual SOMAmer and (ii) Limma analysis (Ritchie, M.E., et al., Nucleic Acids Res. 2015; 20:43(7):e47) on all SOMAmers simultaneously. All p-values were adjusted using the Benjamini-Hochberg (BH) method for multiple hypothesis correction (Benjamini and Hochberg, (1995) J. R. Statist. Soc. B.57: 289-300; R: A Language and Environment for Statistical Computing, R Development Core Team, R Foundation for Statistical Computing, Vienna, Austria. 2011; ISBN 3-900051-07-0). The null hypothesis of no differential expression was rejected when the adjusted p-value < 0.05.

**On treatment versus baseline**

Baseline versus on treatment protein levels were compared using three alternative statistical methods: (i) two-way repeated-measures ANOVA6 (ii) the Wilcoxon signed-rank test and (iii) the Friedman test (Johnson et al., (2007) Biostatistics 8(1):118-127). All p-values were adjusted to control FDR using the BH method for multiple hypothesis correction (Benjamini and Hochberg, J.R. Statist. Soc. B.57:289-300, 1995). In addition to the treatment significance, the two-way repeated-measures ANOVA (Chambers et al., Analysis of variance; designed experiments: Chapter 5. Statistical Models in S, Editors J.M Chambers and T.J Hastie. Wadsworth & Brookes/Cole. 1992) was also applied to determine if significant time-point response-class interaction occurred for each SOMAmer. A modified Wilcoxon rank-sum test was applied as a *post-hoc* test to specifically determine if responders and non-responders showed different treatment effects, by calculating the difference between every subject's on treatment and baseline protein concentration values and performing a Wilcoxon rank-sum test. Significance values were adjusted using the BH method and the null hypothesis was rejected when the adjusted p-value < 0.05.

**Classifier training**

Baseline protein level MMY2002 data was used to build a response prediction classifier. A nested-loop stratified 10-fold cross-validation approach repeated 30 times, using 4 different machine learners: Support Vector Machines (SVM), Random Forests (RF), Naive Bayes (NB), and j48 decision trees. For each learner, the training procedure began with creating 10 balanced folds of the dataset (outer loop). One of these folds was held out as a test cohort while the remaining 9 were passed to an inner loop as the training cohort. Within the inner loop, the training cohort was once again split into 10 balanced folds, creating inner-training and inner-test sets. Learners were trained on each of these inner-training sets and this process was repeated 30 times for each cohort within the outer loop. The accuracy of each inner loop learner at predicting the inner-test sets was used to select features and optimize model parameters. Once the 30x inner looping was complete for each training cohort, the performance of the outer loop (using the optimized parameters and features) was assessed on each corresponding test cohort. The entire outer looping procedure was then repeated 30 times, producing 30 response predictions for every sample within the dataset. The AUC, Sensitivity, and Specificity statistics obtained from this looping approach were an approximation of how well the final model, trained on the full original dataset, will perform on new test cases.

**Results from MMY2002 study**

Various comparisons were conducted including treatment induced response dependent changes in protein expression. One of the proteins that showed decreased expression in responders over time was PD-L1, whereas PD-L1 protein expression increased in non-responders over time. The engagement of PD-L1 on T cells leads to reduced T cell function and increased Treg development. **Figure 18** shows protein expression profile of PD-L1 in responders, non-responders and in patients with stable disease at cycle 1 and cycle 3.

PD-L1 engagement with its receptor PD-1 suppresses anti-tumor responses and drives T cell anergy and exhaustion. While not wishing to be bound by any particular theory, downregulation of PD-L1 upon CD38 treatment may also result in improved potentiation of anti-tumor immune responses in solid tumors.

**Claims**

1. An antibody that specifically binds CD38 for use in treating a patient having a solid tumor, comprising the VH of SEQ ID NO: 4 and the VL of SEQ ID NO: 5, wherein the antibody is of IgG1 isotype.

2. The antibody that specifically binds CD38 for use according to claim 1, wherein the antibody comprises the heavy chain amino acid of SEQ ID NO: 12, and the light chain amino acid of SEQ ID NO: 13.

3. The antibody that specifically binds CD38 for use according to claim 1 wherein the antibody is of IgG1κ isotype

4. The antibody that specifically binds CD38 for use according to any of claims 1-3, wherein the solid tumor is a melanoma, a lung cancer, a squamous non-small cell lung cancer (NSCLC), a non-squamous NSCLC, a colorectal cancer, a prostate cancer, a castration-resistant prostate cancer, a stomach cancer, an ovarian cancer, a gastric cancer, a liver cancer, a pancreatic cancer, a thyroid cancer, a squamous cell carcinoma of the head and neck, a carcinoma of the esophagus or gastrointestinal tract, a breast cancer, a fallopian tube cancer, a brain cancer, an urethral cancer, a genitourinary cancer, an endometriosis, a cervical cancer or a metastatic lesion of the cancer.

5. The antibody that specifically binds CD38 for use according to any of claims 1-4, wherein the solid tumor lacks detectable CD38 expression.

6. The antibody that specifically binds CD38 for use according to any of claims 1-5, wherein the antibody that specifically binds CD38 elicits an immune response in the patient, wherein the immune response is an effector T cell (Teff) response, wherein the Teff response is mediated by the CD8$^+$ T cells, and wherein the Teff response is an increase in the number of the CD8$^+$ T cells.

7. The antibody that specifically binds CD38 for use according to any of claims 1-5, wherein the antibody that specifically binds CD38 inhibits function of an immune suppressor cell, wherein:

   a. the immune suppressor cell is a regulatory T cell (Treg), wherein the Treg is a CD3$^+$CD4$^+$CD25$^+$CD127$^{dim}$ T cell, wherein the Treg expresses CD38, wherein the Treg function is inhibited by killing the Treg, and wherein killing the Treg is mediated by antibody-dependent cell cytotoxicity (ADCC); or
   b. the immune suppressor cell is a myeloid-derived suppressor cell (MDSC), wherein the MDSC is a CD11b$^+$HLADR$^-$CD14$^-$CD33$^+$CD15$^+$cell, wherein the CD11b$^+$HLADR$^-$CD14$^-$CD33$^+$CD15$^+$ cell expresses CD38, wherein the MDSC function is inhibited by killing the MDSC, and wherein killing of MDSC is mediated by ADCC; or
   c. the immune suppressor cell is a regulatory B cell (Breg), wherein the Breg is a CD19$^+$CD24$^+$CD38$^+$ cell, wherein the Breg function is inhibited by killing the Breg, and wherein killing the Breg is mediated by ADCC; or
   d. the immune suppressor cell resides in peripheral blood.

8. The antibody that specifically binds CD38 for use according to any of claims 1-7, wherein the antibody that specifically binds CD38 is administered in combination with a second therapeutic agent, such as wherein the second therapeutic agent is a chemotherapeutic agent, a targeted anti-cancer therapy, a standard of care drug for treatment of solid tumor, or an immune checkpoint inhibitor.

9. The antibody that specifically binds CD38 for use according to claim 8, wherein the immune checkpoint inhibitor is an anti-PD-1 antibody, an anti-PD-L1 antibody, an anti-PD-L2 antibody, an anti-LAG3 antibody, an anti-TIM3 antibody, or an anti-CTLA-4 antibody.

10. The antibody that specifically binds CD38 for use according to claim 9, wherein:

   a. the immune checkpoint inhibitor is an anti-PD-1 antibody, optionally wherein the anti-PD-1 antibody comprises:

      i. the VH of SEQ ID NO: 22 and the VL of SEQ ID NO: 23;
      ii. the VH of SEQ ID NO: 24 and the VL of SEQ ID NO: 25;
      iii. the VH of SEQ ID NO: 32 and the VL of SEQ ID NO: 33; or
      iv. the VH of SEQ ID NO: 34 and the VL of SEQ ID NO:35; or

   b. the immune checkpoint inhibitor is an anti-PD-L1 antibody, optionally wherein the anti-PD-L1 antibody comprises:

      i. the VH of SEQ ID NO: 26 and the VL of SEQ ID NO: 27;
      ii. the VH of SEQ ID NO: 28 and the VL of SEQ ID NO: 29; or
      iii. the VH of SEQ ID NO: 30 and the VL of SEQ ID NO: 31; or

   c. the immune checkpoint inhibitor is an anti-PD-L2 antibody; or
   d. the immune checkpoint inhibitor is an anti-LAG3 antibody; or
   e. the immune checkpoint inhibitor is an anti-TIM-3 antibody; optionally wherein the anti-TIM-3 antibody comprises:

      i. the VH of SEQ ID NO: 36 and the VL of SEQ ID NO: 37; or
      ii. the VH of SEQ ID NO: 38 and the VL of SEQ ID NO: 39.

11. The antibody that specifically binds CD38 for use according to any of claims 8-10, wherein the second therapeutic agent is administered simultaneously, sequentially or separately.

12. The antibody that specifically binds CD38 for use according to any of the claims 1-11,
wherein the antibody that specifically binds CD38 is administered parentally by intravenous infusion or bolus injection, intramuscularly, subcutaneously or intraperitoneally.

13. The antibody that specifically binds CD38 for use according to any of the claims 1-12,
wherein the antibody the antibody that specifically binds CD38 is administered intravenously.

14. The antibody that specifically binds CD38 for use according to any of the claims 1-12,
wherein the antibody the antibody that specifically binds CD38 is administered subcutaneously.

15. The antibody that specifically binds CD38 for use according to any of the claims 1-14, wherein:

   a. the patient is treated or has been treated with radiation therapy; and/or
   b. the patient has had or will undergo surgery.


**Patentansprüche**

1. Antikörper, der CD38 spezifisch bindet, zur Verwendung bei der Behandlung eines an einem soliden Tumor leidenden Patienten, umfassend die VH unter SEQ ID NO: 4 und die VL unter SEQ ID NO: 5, wobei es sich um einen Antikörper des IgG1-Isotyps handelt.

2. Antikörper, der CD38 spezifisch bindet, zur Verwendung nach Anspruch 1, wobei der Antikörper die Schwere-Kette-Aminosäure unter SEQ ID NO: 12 und die Leichte-Kette-Aminosäure unter SEQ ID NO: 13 umfasst.

3. Antikörper, der CD38 spezifisch bindet, zur Verwendung nach Anspruch 1, wobei wobei es sich um einen Antikörper des IgG1K-Isotyps handelt.

4. Antikörper, der CD38 spezifisch bindet, zur Verwendung nach einem der Ansprüche 1-3, wobei es sich bei dem soliden Tumor um ein Melanom, einen Lungenkrebs, ein Plattenepithelkarzinom der Lunge (squamöses NSCLC), ein nichtsquamöses NSCLC, ein Kolorektalkarzinom, einen Prostatakrebs, einen kastrationsresistenten Prostatakrebs, einen Magenkrebs, ein Ovarialkarzinom, ein Magenkarzinom, einen Leberkrebs, einen Bauchspeicheldrüsenkrebs, einen Schilddrüsenkrebs, ein Plattenepithelkarzinom im Kopf- und Halsbereich, ein Karzinom der Speiseröhre oder des Magen-Darm-Trakts, einen Brustkrebs, einen Eileiterkrebs, einen Hirntumor, einen Harnröhrenkrebs, einen Urogenitalkrebs, eine Endometriose, einen Gebärmutterhalskrebs oder eine metastatische Läsion des Krebses handelt.

5. Antikörper, der CD38 spezifisch bindet, zur Verwendung nach einem der Ansprüche 1-4, wobei der solide Tumor keine nachweisbare CD38-Expression aufweist.

6. Antikörper, der CD38 spezifisch bindet, zur Verwendung nach einem der Ansprüche 1-5, wobei der Antikörper, der CD38 spezifisch bindet, eine Immunantwort bei dem Patienten hervorruft, wobei es sich bei der Immunantwort um eine T-Effektorzell(Teff)-Antwort handelt, wobei die Teff-Antwort durch die CD8$^+$-T-Zellen vermittelt wird und wobei es sich bei der Teff-Antwort um eine Erhöhung der Anzahl der CD8$^+$-T-Zellen handelt.

7. Antikörper, der CD38 spezifisch bindet, zur Verwendung nach einem der Ansprüche 1-5, wobei der Antikörper, der CD38 spezifisch bindet, die Funktion einer Immunsuppressorzelle hemmt, wobei:

   a. die Immunsuppressorzelle eine T-Regulatorzelle (Treg) ist, wobei es sich bei der Treg um eine CD3$^+$CD4$^+$CD25$^+$CD127$^{dim}$-T-Zelle handelt, wobei die Treg CD38 exprimiert, wobei die Treg-Funktion durch Abtöten der Treg gehemmt wird und wobei Abtöten der Treg durch ADCC (Antibody-Dependent Cell Cytotoxicity) vermittelt wird; oder
   b. die Immunsuppressorzelle eine myeloide Suppressorzelle (MDSC) ist, wobei es sich bei der MDSC um eine CD11b$^+$HLADR$^-$CD14$^-$CD33$^+$CD15$^+$-Zelle handelt, wobei die CD11b$^+$HLADR$^-$CD14$^-$CD33$^+$CD15$^+$-Zelle CD38, wobei die MDSC-Funktion durch Abtöten der MDSC gehemmt wird und wobei Abtöten der MDSC durch ADCC vermittelt wird; oder
   c. die Immunsuppressorzelle eine B-Regulatorzelle (Breg) ist, wobei es sich bei der Breg um eine CD19$^+$CD24$^+$CD38$^+$-Zelle handelt, wobei die Breg-Funktion durch Abtöten der Breg gehemmt wird und wobei Abtöten der Breg durch ADCC vermittelt wird; oder
   d. sich die Immunsuppressorzelle in peripherem Blut befindet.

8. Antikörper, der CD38 spezifisch bindet, zur Verwendung nach einem der Ansprüche 1-7, wobei der Antikörper, der CD38 spezifisch bindet, in Kombination mit einem zweiten Therapeutikum verabreicht wird, wie etwa wobei es sich bei dem zweiten Therapeutikum um ein Chemotherapeutikum, eine gezielte Krebstherapie, ein Standardmedikament für die Behandlung solider Tumoren oder einen Immuncheckpoint-Inhibitor handelt.

9. Antikörper, der CD38 spezifisch bindet, zur Verwendung nach Anspruch 8, wobei es sich bei dem Immuncheckpoint-Inhibitor um einen Anti-PD-1-Antikörper, einen Anti-PD-L1-Antikörper, einen Anti-PD-L2-Antikörper, einen Anti-LAG3-Antikörper, einen Anti-TIM3-Antikörper oder einen einen Anti-CTLA-4-Antikörper handelt.

10. Antikörper, der CD38 spezifisch bindet, zur Verwendung nach Anspruch 9, wobei:

   a. es sich bei dem Immuncheckpoint-Inhibitor um einen Anti-PD-1-Antikörper handelt, gegebenenfalls wobei der Anti-PD-1-Antikörper Folgendes umfasst:

      i. die VH unter SEQ ID NO: 22 und die VL unter SEQ ID NO: 23;
      ii. die VH unter SEQ ID NO: 24 und die VL unter SEQ ID NO: 25
      iii. die VH unter SEQ ID NO: 32 und die VL unter SEQ ID NO: 33; oder
      iv. die VH unter SEQ ID NO: 34 und die VL unter SEQ ID NO: 35; oder

   b. es sich bei dem Immuncheckpoint-Inhibitor um einen Anti-PD-L1-Antikörper handelt, gegebenenfalls wobei der Anti-PD-L1-Antikörper Folgendes umfasst:

      i. die VH unter SEQ ID NO: 26 und die VL unter SEQ ID NO: 27;
      ii. die VH unter SEQ ID NO: 28 und die VL unter SEQ ID NO: 29; oder
      iii. die VH unter SEQ ID NO: 30 und die VL unter SEQ ID NO: 31; oder

c. es sich bei dem Immuncheckpoint-Inhibitor um einen Anti-PD-L2-Antikörper handelt; oder

d. es sich bei dem Immuncheckpoint-Inhibitor um einen Anti-LAG3-Antikörper handelt; oder

e. es sich bei dem Immuncheckpoint-Inhibitor um einen Anti-TIM-3-Antikörper handelt; gegebenenfalls wobei der Anti-TIM-3-Antikörper Folgendes umfasst:

    i. die VH unter SEQ ID NO: 36 und die VL unter SEQ ID NO: 37; oder
    ii. die VH unter SEQ ID NO: 38 und die VL unter SEQ ID NO: 39.

**11.** Antikörper, der CD38 spezifisch bindet, zur Verwendung nach einem der Ansprüche 8-10, wobei das zweite Therapeutikum gleichzeitig, aufeinander folgend oder getrennt verabreicht wird.

**12.** Antikörper, der CD38 spezifisch bindet, zur Verwendung nach einem der Ansprüche 1-11, wobei der Antikörper, der CD38 spezifisch bindet, parental durch intravenöse Infusion oder Bolusinjektion, intramuskulär, subkutan oder intraperitoneal verabreicht wird.

**13.** Antikörper, der CD38 spezifisch bindet, zur Verwendung nach einem der Ansprüche 1-12, wobei der Antikörper, der CD38 spezifisch bindet, intravenös verabreicht wird.

**14.** Antikörper, der CD38 spezifisch bindet, zur Verwendung nach einem der Ansprüche 1-12, wobei der Antikörper, der CD38 spezifisch bindet, subkutan verabreicht wird.

**15.** Antikörper, der CD38 spezifisch bindet, zur Verwendung nach einem der Ansprüche 1-14, wobei:

    a. der Patient mit einer Strahlentherapie behandelt wird oder wurde; und/oder
    b. der Patient operiert wurde oder wird.

**Revendications**

**1.** Anticorps qui se lie spécifiquement à CD38 pour une utilisation dans le traitement d'un patient présentant une tumeur solide, comprenant la VH de la SEQ ID NO: 4 et la VL de la SEQ ID NO: 5, l'anticorps étant de l'isotype IgG1.

**2.** Anticorps qui se lie spécifiquement à CD38 pour une utilisation selon la revendication 1, l'anticorps comprenant l'acide aminé à chaîne lourde de la SEQ ID NO: 12 et l'acide aminé de la chaîne légère de la SEQ ID NO: 13.

**3.** Anticorps qui se lie spécifiquement à CD38 pour une utilisation selon la revendication 1, l'anticorps étant de l'isotype IgG1K.

**4.** Anticorps qui se lie spécifiquement à CD38 pour une utilisation selon l'une quelconque des revendications 1-3, dans lequel la tumeur solide est un mélanome, un cancer du poumon, un cancer du poumon épidermoïde non à petites cellules (NSCLC), un NSCLC non squameux, un cancer colorectal, un cancer de la prostate, un cancer de la prostate résistant à la castration, un cancer de l'estomac, un cancer de l'ovaire, un cancer gastrique, un cancer du foie, un cancer du pancréas, un cancer de la thyroïde, un carcinome à cellules squameuses de la tête et du cou, un carcinome de l'œsophage ou du tube digestif, un cancer du sein, un cancer de la trompe de Fallope, un cancer du cerveau, un cancer urétral, un cancer génito-urinaire, une endométriose, un cancer du col de l'utérus ou une lésion métastatique du cancer.

**5.** Anticorps qui se lie spécifiquement à CD38 pour une utilisation selon l'une quelconque des revendications 1-4, dans lequel la tumeur solide ne présente pas d'expression détectable de la CD38.

**6.** Anticorps qui se lie spécifiquement à CD38 pour une utilisation selon l'une quelconque des revendications 1-5, l'anticorps qui se lie spécifiquement à CD38 produisant une réponse immunitaire chez le patient, dans lequel la réponse immunitaire est une réponse à des cellules T effectrices (Teff), dans lequel la réponse à Teff est médiée par les cellules T CD8[+] et dans lequel la réponse à Teff est une augmentation du nombre de cellules T CD8[+].

**7.** Anticorps qui se lie spécifiquement à CD38 pour une utilisation selon l'une quelconque des revendications 1-5, l'anticorps qui se lie spécifiquement à CD38 inhibant la fonction d'une cellule immunosuppressive, dans lequel

a. la cellule de suppression immunitaire est une cellule T régulatrice (Treg), la Treg étant une cellule T CD3$^+$CD4$^+$CD25$^+$CD127$^{dim}$, la Treg exprimant CD38, la fonction Treg étant inhibée en tuant la Treg, et la destruction de la Treg étant médiée par la cytotoxicité cellulaire dépendante des anticorps (ADCC) ; ou

b. la cellule de suppression immunitaire est une cellule suppressive d'origine myéloïde (MDSC), la MDSC étant une cellule CD11b$^+$HLADR$^-$CD14$^-$CD33$^+$CD15$^+$, la cellule CD11b$^+$HLADR$^-$CD14$^-$CD33$^+$CD15$^+$ exprimant CD38, la fonction MDSC étant inhibée en tuant la MDSC, et la destruction de la MDSC étant médiée par l'ADCC ; ou

c. la cellule de suppression immunitaire est une cellule B régulatrice (Breg), la Breg étant une cellule CD19$^+$CD24$^+$CD38$^+$, la fonction Breg étant inhibée en tuant la Breg, et la destruction de la Breg étant médiée par l'ADCC ; ou

d. la cellule immunosuppressive se trouve dans le sang périphérique.

8. Anticorps qui se lie spécifiquement à CD38 pour une utilisation selon l'une quelconque des revendications 1-7, l'anticorps qui se lie spécifiquement à CD38 étant administré en combinaison avec un deuxième agent thérapeutique, tel que dans lequel le deuxième agent thérapeutique étant un agent chimiothérapeutique, un traitement anticancéreux ciblé, un médicament de référence pour le traitement des tumeurs solides ou un inhibiteur de point de contrôle immunitaire.

9. Anticorps qui se lie spécifiquement à CD38 pour une utilisation selon la revendication 8, dans lequel l'inhibiteur de point de contrôle immunitaire est un anticorps anti-PD-1, un anticorps anti-PD-L1, un anticorps anti-PD-L2, un anticorps anti-LAG3, un anticorps anti-TIM3, ou un anticorps anti-CTLA-4.

10. Anticorps qui se lie spécifiquement à CD38 pour une utilisation selon la revendication 9, dans lequel :

a. l'inhibiteur de point de contrôle immunitaire est un anticorps anti-PD-1, dans lequel l'anticorps anti-PD-1 comprend :

i. la VH de la SEQ ID NO: 22 et la VL de la SEQ ID NO: 23 ;
ii. la VH de la SEQ ID NO: 24 et la VL de la SEQ ID NO: 25 ;
iii. la VH de la SEQ ID NO: 32 et la VL de la SEQ ID NO: 33 ; ou
iv. la VH de la SEQ ID NO: 34 et la VL de la SEQ ID NO: 35 ; ou

b. l'inhibiteur de point de contrôle immunitaire est un anticorps anti-PD-L1, éventuellement dans lequel l'anticorps anti-PD-L1 comprend :

i. la VH de la SEQ ID NO: 26 et la VL de la SEQ ID NO: 27 ;
ii. la VH de la SEQ ID NO: 28 et la VL de la SEQ ID NO: 29 ; ou
iii. la VH de la SEQ ID NO: 30 et la VL de la SEQ ID NO: 31 ; ou

c. l'inhibiteur de point de contrôle immunitaire est un anticorps anti-PD-L2 ; ou
d. l'inhibiteur de point de contrôle immunitaire est un anticorps anti-LAG3 ; ou
e. l'inhibiteur de point de contrôle immunitaire est un anticorps anti-TIM-3 ; éventuellement dans lequel l'anticorps anti-TIM-3 comprend :

i. la VH de la SEQ ID NO: 36 et la VL de la SEQ ID NO: 37 ; ou
ii. la VH de la SEQ ID NO: 38 et la VL de la SEQ ID NO: 39.

11. Anticorps qui se lie spécifiquement à CD38 pour une utilisation selon l'une quelconque des revendications 8-10, dans lequel le deuxième agent thérapeutique est administré simultanément, séquentiellement ou séparément.

12. Anticorps qui se lie spécifiquement à CD38 pour une utilisation selon l'une quelconque des revendications 1-11, l'anticorps qui se lie spécifiquement à CD38 étant administré de manière parentérale par perfusion intraveineuse ou injection de bolus, de manière intramusculaire, de manière sous-cutanée ou de manière intrapéritonéale.

13. Anticorps qui se lie spécifiquement à CD38 pour une utilisation selon l'une quelconque des revendications 1-12, l'anticorps qui se lie spécifiquement à CD38 étant administré par voie intraveineuse.

14. Anticorps qui se lie spécifiquement à CD38 pour une utilisation selon l'une des revendications 1-12, l'anticorps qui se lie spécifiquement à CD38 étant administré par voie sous-cutanée.

15. Anticorps qui se lie spécifiquement à CD38 pour une utilisation selon l'une des revendications 1-14, dans lequel :

a. le patient est traité ou a été traité par radiothérapie ; et/ou
b. le patient a subi ou va subir une intervention chirurgicale.

Figure 1.

Figure 2.

Figure 3.

Figure 4.

Figure 5.

Figure 6.

Figure 7A.

Figure 7B.

Figure 8A.

Clonality change upon treatment

Figure 8B.

Figure 8C.

Figure 8D.

Sum of change in clone abundance

Figure 8E.

Figure 8F.

Maximum change in a single clone abundance

Figure 9A.

CD8 naive all

Figure 9B.

Figure 9C.

Figure 9D.

Figure 10A.

Figure 10B.

Figure 10C.

Figure 10D.

Figure 10E.

Figure 11.

Figure 12.

Figure 13.

Figure 14.

Figure 15A.

Figure 15B.

Figure 16A.

Figure 16B.

Figure 16C.

Figure 16D.

Figue 16E.

**pt 1**

**VGPR**

Figure 16F.

Figure 17A.

Figure 17B.

Figure 17C.

Figure 18.

Programmed cell death 1 ligand 1 »
CD274..5060.62_3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015009726 A2
- WO 2012092612 A1
- WO 199844001 A
- WO 198801649 A
- WO 199413804 A
- WO 199201047 A
- WO 2009085462 A
- US 8779105 B
- US 6737056 B
- WO 2008077546 A
- US 7829693 B
- US 8088896 B
- US 8153765 B
- WO 05103083 A
- WO 06125640 A
- WO 07042309 A
- WO 08047242 A
- WO 14178820 A
- WO 09085462 A
- US 5223409 A
- US 5403484 A
- US 5571698 A
- US 5427908 A
- US 5580717 A
- US 5969108 A
- US 6172197 B
- US 5885793 A
- US 6521404 B
- US 6544731 B
- US 6555313 B
- US 6582915 B
- US 6593081 B
- US 4683195 A
- US 7695936 B
- WO 04111233 A
- US 20100015133 A
- US 20070287170 A
- WO 2008119353 A
- US 20090182127 A
- US 20100286374 A
- US 20110123532 A
- WO 2011131746 A
- WO 2011143545 A
- US 20120149876 A
- WO 2009134776 A
- WO 2012022811 A
- US 5932448 A
- US 6833441 B
- US 20040197332 A
- US 8008449 B
- US 8354509 B
- US 7332582 B
- US 20140044738 A
- WO 201417966 A
- US 20140356363 A
- US 20090055944 A
- US 8552154 B
- US 8217149 B
- US 8779108 B
- WO 2010019570 A
- WO 2004078140 A

### Non-patent literature cited in the description

- **WANG et al.** *J Exp Med,* 07 March 2011, vol. 208 (3), 577-92
- **LEPENIES et al.** *Endocr Metab Immune Disord Drug Targets,* 2008, vol. 8, 279-288
- **SWAIKA et al.** *Mol Immunol,* 2015
- **M DE WEERS et al.** *Journal of Immunology,* 27 December 2010, vol. 186 (3), 1840-1848
- **CHILLEMI et al.** *Molecular Medicine,* 2013, vol. 19 (1), 99-108
- **WARD et al.** *Nature,* 1989, vol. 341, 544-6
- **WU ; KABAT.** *J Exp Med,* 1970, vol. 132, 211-50
- **KABAT et al.** Sequences of Proteins of Immunological Interest. Public Health Service, National Institutes of Health, 1991
- **CHOTHIA ; LESK.** *Mol Biol,* 1987, vol. 196, 901-17
- **LEFRANC et al.** *Dev Comparat Immunol,* 2003, vol. 27, 55-77
- **ALMAGRO.** *Mol Recognit,* 2004, vol. 17, 132-43
- **LEFRANC et al.** *Dev Comparat -Immunol,* 2003, vol. 27, 55-77
- **AL-LAZIKANI et al.** *J Mol Biol,* 1997, vol. 273, 927-48

- **KNAPPIK et al.** *J Mol Biol,* 2000, vol. 296, 57-86
- **SHI et al.** *J Mol Biol,* 2010, vol. 397, 385-96
- **DING et al.** *Human Immunology,* 2015, vol. 76, 615-621
- **MANDRUZZATO et al.** *J Immunol,* 2009, vol. 182, 6562-6568
- **LIU et al.** *J Cancer Res Clin Oncol,* 2009, vol. 136, 35-45
- **KO et al.** *Clin Cancer Res,* 2009, vol. 15, 2148-2157
- **MORSE et al.** *Expert Opin Biol Ther,* 2009, vol. 9, 331-339
- **DIAZ-MONTERO et al.** *Cancer Immunol Immunother,* 2009, vol. 58, 49-59
- **CORZO et al.** *J Immunol,* 2009, vol. 182, 5693-5701
- **DING et al.** *Human Immunology,* 2015, vol. 76, 615-62
- **KONNO et al.** *Cytotechnology,* 2012, vol. 64, 249-65
- **SHIELDS et al.** *J Biol Chem,* 2002, vol. 277, 26733-26740
- **OLIVIER et al.** *MAbs,* 2010, vol. 2 (4
- **SHINKAWA et al.** *J Biol Chem,* 2003, vol. 278, 3466-3473
- **MORI et al.** *Biotechnol Bioeng,* 2004, vol. 88, 901-908
- **FERRARA et al.** *J Biol Chem,* 2006, vol. 281, 5032-5036
- **FERRARA et al.** *Biotechnol Bioeng,* 2006, vol. 93, 851-861
- **XHOU et al.** *Biotechnol Bioeng,* 2008, vol. 99, 652-65
- **KREBS et al.** *J Immunol Meth,* 2001, vol. 254, 67-84
- **VAUGHAN et al.** *Nature Biotechnology,* 1996, vol. 14, 309-314
- **SHEETS et al.** *PITAS (USA),* 1998, vol. 95, 6157-6162
- **HOOGENBOOM ; WINTER.** *J Mol Biol,* 1991, vol. 227, 381
- **MARKS et al.** *J Mol Biol,* 1991, vol. 222, 581
- **MACLENNAN et al.** *Acta Physiol Scand Suppl,* 1998, vol. 643, 55-67
- **SASAKI et al.** *Adv Biophys,* 1998, vol. 35, 1-24
- Pharmaceutical Manufacturing. Remington: The Science and Practice of Pharmacy. Lipincott Williams and Wilkins, 2006, 691-1092, 958-989
- **WIEMANN et al.** Medical Oncology. McMillan Publishing, 1985
- **MAATTA et al.** *Mol Biol Cell,* 2006, vol. 17, 67-79
- **GORGUN et al.** *Blood,* 2013, vol. 121, 2975-87
- A Language and Environment for Statistical Computing, R Development Core Team. *R Foundation for Statistical Computing,* 2011, ISBN 3-900051-07-0, http_//_www_R-project_org
- **ROYSTON.** Remark AS R94: A remark on Algorithm AS 181: The W test for normality. *Applied Statistics,* 1995, vol. 44, 547-551
- **HOLLANDER ; WOLFE.** Nonparametric Statistical Methods. John Wiley & Sons, 1973, 27-33, 68-75
- **WEISBERG, S.** Applied Linear Regression. Wiley Wiley, 2014
- **LUTZ et al.** Statistical model to estimate a threshold dose and its confidence limits for the analysis of sublinear dose-response relationships, exemplified for mutagenicity data. *Mutation Research/Genetic Toxicology and Environmental Mutagenesis,* 2009, vol. 678 (2), 118-122
- **BATES et al.** lme4: Linear mixed-effects models using Eigen and S4. *Journal of Statistical Software,* 2014, http:_//_arxiv_org/abs/_1406.5823
- **DE WEERS et al.** *J Immunol,* 2011, vol. 186 (3), 1840-1848
- **LOKHORST et al.** *N Eng J Med,* 2005, vol. 373, 1207-19
- **DEWITT et al.** *J. Virol.,* 2015
- **GOLD et al.** *PLoS One,* 2010, vol. 5, e15004
- **HOLLANDER ; WOLFE.** Ninparametirc Statistical Methods. John Wiley & Sons, 1973, 27-33, 68-75
- **RITCHIE, M.E. et al.** *Nucleic Acids Res.,* 2015, vol. 43 (7), e47
- **BENJAMINI ; HOCHBERG.** *J. R. Statist. Soc. B.,* 1995, vol. 57, 289-300
- **JOHNSON et al.** *Biostatistics,* 2007, vol. 8 (1), 118-127
- **BENJAMINI ; HOCHBERG.** *J.R. Statist. Soc. B.,* 1995, vol. 57, 289-300
- Analysis of variance; designed experiments. **CHAMBERS et al.** Statistical Models in S. Wadsworth & Brookes/Cole, 1992